# EUROPEAN PATENT APPLICATION

(11) **EP 4 011 419 A1**
(43) Date of publication of application: **15.06.2022**
(21) Application number: 20852390.2
(22) Date of filing: 07.08.2020
(51) Int. Cl.: A61M 5/158, A61M 5/32, B29C 33/42

(54) **NEEDLE TIP PROTECTOR AND NEEDLE ASSEMBLED BODY USING SAME**

(30) Priority: 09.08.2019 JP 2019147724; 03.09.2019 JP 2019160609; 28.10.2019 JP 2019195623; 20.01.2020 JP 2020007050; 11.03.2020 JP 2020041999
(71) Applicant: Nipro Corporation, Osaka-shi, Osaka 531-8510 (JP)
(72) Inventor: NAKAGAMI, Hiroyuki, Osaka-shi, Osaka 531-8510 (JP); ISHIKURA, Kohzo, Osaka-shi, Osaka 531-8510 (JP)
(74) Representative: Bertsch, Florian Oliver
(86) International application number: PCT/JP2020/030480
(87) International publication number: WO 2021/029393

(57) **Abstract**

Provided are a novel needle tip protector that can be stably produced by resin molding such as injection molding, and a novel needle assembly using the same. The invention provides a resin needle tip protector A-10 including a peripheral wall configured to move in a needle axis direction relative to a puncture needle A-16 to cover a needle tip A-20 of the puncture needle A-16, wherein the peripheral wall A-54 includes a tubular part on a distal end side, the peripheral wall A-54 includes a thick-walled part A-70 on a proximal end side of the tubular part A-58, the thick-walled part A-70 including a portion having a wall thickness dimension larger than that of the tubular part A-58, and an injection gate trace A-98 of a resin material during molding is provided to the thick-walled part A-70.

## Description

### TECHNICAL FIELD

The present invention (Invention A) relates to a needle tip protector that covers the needle tip of the removed puncture needle and a needle assembly including the needle tip protector.

### BACKGROUND ART

Conventionally, for the purpose of preventing inadvertent puncture and frequent use of a puncture needle body such an indwelling needle used for infusion, blood collection, hemodialysis, etc., or facilitating disposal after use, a needle tip protector that protects the needle tip of the puncture needle removed from the somatic lumen is adopted. A needle tip protector and a needle assembly including the needle tip protector are disclosed in, for example, Japanese Unexamined Patent Publication No. JP-A-2017-196060 (Patent Document 1). That is, according to the medical needle with a protector of Patent Document 1, when the needle tube is pulled out from a blood vessel or the like, the protector moves toward the needle tip side with respect to the needle tube, and the needle tube removed from the patient is accommodated in the radial inside of the protector. Therefore, the needle tip of the needle tube is prevented from being exposed to the outside.

Meanwhile, the needle tip protector may be formed of resin, and in this case, it is manufactured by molding such as injection molding, for example. That is, the needle tip protector having a predetermined shape is manufactured by filling the cavity of the mold corresponding to the shape of the needle tip protector with the resin material and curing the resin material.

### BACKGROUND ART DOCUMENT

### PATENT DOCUMENT

Patent Document 1: JP-A-2017-196060

### SUMMARY OF THE INVENTION

### PROBLEM THE INVENTION ATTEMPTS TO SOLVE

However, the needle tip protector has a relatively thin peripheral wall that surrounds and covers the puncture needle, and when the needle tip protector is to be formed by molding, in some cases, there may be a problem such as poor filling of the resin material due to pressure loss. In addition, there was room for improvement from the viewpoint of the usage method for the health care worker and the patient side, who is the target for use of the needle tip protector.

It is an object of Invention A to provide a novel needle tip protector which is highly safe and can be stably manufactured by molding of a resin such as injection molding.

It is another object of Invention A to provide a needle assembly provided with the above-mentioned needle tip protector as a needle tip protector for protecting the needle tip of the puncture needle constituting the puncture needle body.

### MEANS FOR SOLVING THE PROBLEM

Hereinafter, preferred embodiments for grasping Invention A will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in Invention A, various other preferred embodiments can be realized without being limited to those described below.

A first preferred embodiment of Invention A provides a needle tip protector that is made of resin and includes a peripheral wall configured to move in a needle axis direction with respect to a puncture needle to cover a needle tip of the puncture needle, wherein the peripheral wall includes a tubular part on a distal end side of the peripheral wall, the peripheral wall includes a thick-walled part on a proximal end side of the tubular part, the thick-walled part including a portion having a wall thickness dimension larger than that of the tubular part, and at least one injection gate trace of a resin material during molding is provided to the thick-walled part.

In the needle tip protector of the present preferred embodiment, an injection gate of the resin material is set at a portion where the thick-walled part of the peripheral wall is molded during molding, and the resin material is charged from a portion having a large cross-sectional area toward a portion having a small cross-sectional area in the cavity of the mold. Therefore, when the cavity is filled with the resin material, pressure loss due to a change in the cross-sectional area of the cavity is unlikely to occur, so that poor filling of the resin material can be avoided, thereby stably obtaining a needle tip protector having a predetermined shape.

A second preferred embodiment of Invention A provides the needle tip protector according to the first preferred embodiment, wherein a recess is provided on an outer surface of the thick-walled part.

According to the needle tip protector of the present preferred embodiment, it is possible to prevent the thick-walled part from being excessively thick. As a result, it is possible to avoid troubles such as air bubbles being mixed, and sink marks or voids being generated in the thick-walled part. For example, in the case where the thickness of the thick-walled part is not constant because the shapes of the inner surface and the outer surface of the thick-walled part are different, the recess can be provided on the outer surface of the portion of the thick-walled part that is particularly thick. As a result, it is possible to prevent the thick-walled part from being partially remarkably thick.

A third preferred embodiment of Invention A provides the needle tip protector according to the second preferred embodiment of Invention A, wherein the injection gate trace is provided on an inner surface of the recess.

According to the needle tip protector of the present preferred embodiment, for example, in the case where the injection gate trace is in the shape of a protrusion, by providing the injection gate trace on the inner surface of the recess, the injection gate trace is less likely to project outward from the outer surface of the thick-walled part. With this configuration, when the thick-walled part comes into contact with the patient's skin or the like, problems such as the injection gate trace sticking and causing pain are unlikely to occur.

The thickness of the peripheral wall decreases in the portion where the recess is formed, but if the recess is formed in the thick-walled part including the portion having a large thickness dimension, it is possible to prevent the peripheral wall from being excessively thin in the portion where the recess is formed. Therefore, even in the case where the injection gate trace is provided on the inner surface of the recess to obtain the effect of avoiding contact of the injection gate trace with a patient or the like, poor filling of the resin material is effectively prevented during molding, thereby stably manufacturing the needle tip protector having a predetermined shape.

A fourth preferred embodiment of Invention A provides the needle tip protector according to the second or third preferred embodiment of Invention A, wherein a depth dimension of the recess gradually decreases toward the distal end side of the peripheral wall.

According to the needle tip protector of the present preferred embodiment, for example, in the case where the position of the injection gate is set so that the injection gate trace is provided on the inner surface of the recess, sudden changes in the filling pressure of the resin material during molding and hence in the resin density during molding can be avoided, thereby securing the strength of the needle tip protector and preventing molding defects such as sink marks.

A fifth preferred embodiment of Invention A provides the needle tip protector according to any one of the second to fourth preferred embodiments of Invention A, wherein an opening end edge of the recess has a curved cross-sectional shape with no corners.

According to the needle tip protector of the present preferred embodiment, when the opening end edge of the recess touches the patient's skin, the opening end edge is unlikely to cause pain or sense of discomfort for the patient. Further, since the opening end edge of the recess has a shape with no corners, troubles such as the opening end edge of the recess being chipped are unlikely to occur.

A sixth preferred embodiment of Invention A provides the needle tip protector according to any one of the first to fifth preferred embodiments of Invention A, wherein the peripheral wall includes an expansion part on the proximal end side of the tubular part, the expansion part including a portion whose wall thickness is larger than that of the tubular part and having an outer dimension larger than that of the tubular part, and the injection gate trace is provided on an outer surface of the expansion part.

According to the needle tip protector of the present preferred embodiment, the thick-walled part, to which the injection gate trace is set, is provided in the expansion part having an increased outer diameter. This makes it possible to prevent the inner diameter of the peripheral wall including the expansion part from being remarkably smaller in the thick-walled part.

A seventh preferred embodiment of Invention A provides a needle tip protector that is made of resin and includes a peripheral wall configured to move in a needle axis direction with respect to a puncture needle to cover a needle tip of the puncture needle, wherein the peripheral wall includes a tubular part and an expansion part having an outer dimension larger than that of the tubular part, and at least one injection gate trace of a resin material during molding is provided to the expansion part.

According to the needle tip protector of the present preferred embodiment, during molding, the injection gate of the resin material is set at the portion molding the expansion part, which has an outer dimension larger than that of the tubular part and has a circumferential length of the outer circumferential surface longer than that of the tubular part. With this configuration, it is possible to fill the portion having a long circumferential length in the cavity of the molding die with the resin material with sufficient pressure, thereby avoiding poor filling of the resin material.

An eighth preferred embodiment of Invention A provides the needle tip protector according to any one of the first to seventh preferred embodiments of Invention A, wherein the needle tip protector further includes an engaging part engaged with a needle hub provided on a proximal end side of the puncture needle, and the injection gate trace is provided at a position away from the engaging part.

According to the needle tip protector of the present preferred embodiment, the injection gate is set at a position away from the portion where the shape is complicated or the cross-sectional area is decreased due to the formation of the engaging part. This makes it possible to more advantageously avoid molding defects.

A ninth preferred embodiment of Invention A provides the needle tip protector according to any one of the first to eighth preferred embodiments of Invention A, wherein the needle tip protector further includes a locking piece configured to, with the needle tip of the puncture needle covered with the peripheral wall, be locked to a needle hub provided on a proximal end side of the puncture needle to prevent re-exposure of the needle tip, and the injection gate trace is provided at a position away from the locking piece.

According to the needle tip protector of the present preferred embodiment, if the portion of the peripheral wall away from the locking piece comprises the thick-walled part, the portion of the peripheral wall where the locking piece is provided can be made relatively thin.
As a result, it is possible to suppress mixing of air bubbles in the locking piece and generation of sink marks and voids. Further, the injection gate is set at a position away from the portion where the shape is complicated or the cross-sectional area is decreased due to the formation of the locking piece. This makes it possible to more advantageously avoid molding defects.

A tenth preferred embodiment of Invention A provides the needle tip protector according to any one of the first to ninth preferred embodiments of Invention A, wherein an outer surface of the peripheral wall is molded by split molds combined in an axis-perpendicular direction and has a parting line extending in an axial direction.

According to the needle tip protector of the present preferred embodiment, a large degree of freedom in shape can be realized in the outer surface of the peripheral wall. If the injection gate position of the resin material is set such that the injection gate trace is formed on the outer surface of the peripheral wall, it is possible to adopt a pin gate in which the runner is pulled and cut when removing the split molds in the axis-perpendicular direction. Thus, a plurality of needle tip protectors can be easily molded at the same time in a small space, thereby improving mass-productivity of the needle tip protector.

An eleventh preferred embodiment of Invention A provides the needle tip protector according to any one of the first to ninth preferred embodiments of Invention A, wherein an outer surface of the peripheral wall is molded by a die to be released in an axial direction and has no parting line extending in the axial direction.

With the needle tip protector of the present preferred embodiment, if the injection gate position of the resin material is set such that the injection gate trace is formed on the outer surface of the peripheral wall, it is possible to adopt a tunnel gate for which the runner is cut by the die when removing the die in the axial direction. Therefore, the needle tip protector can be manufactured with a relatively small amount of forming material.

A twelfth preferred embodiment of Invention A provides the needle tip protector according to any one of the first to eleventh preferred embodiments of Invention A, wherein the at least one injection gate trace comprises a plurality of injection gate traces provided in a circumferential direction of the peripheral wall.

According to the needle tip protector of the present preferred embodiment, during molding, by filling the cavity of the mold with the resin material from a plurality of locations in the circumferential direction, poor filling of the resin material is more unlikely to occur.

A thirteenth preferred embodiment of Invention A provides the needle tip protector according to any one of the first to twelfth preferred embodiments of Invention A, wherein at least a proximal end part of the peripheral wall has outer dimensions in an axis-perpendicular direction that are different in a circumferential direction, in a wide part in which the outer dimension is larger, at least one of an engaging part engaged with a needle hub fixed to a proximal end part of the puncture needle and a locking piece configured to be locked to the needle hub to prevent re-exposure of the needle tip, and in a narrow part in which the outer dimension is smaller, the injection gate trace is provided.

According to the needle tip protector of the present preferred embodiment, in the wide part having a large outer dimension in the axis-perpendicular direction, it is easy to secure a space for arranging at least one of the engaging part and the locking part. The narrow part having a small outer dimension in the axis-perpendicular direction is easy to be thicker than the wide part in which at least one of the engaging part and the locking part is arranged, and filling of the resin material can be efficiently realized during molding.

A fourteenth preferred embodiment of Invention A provides the needle tip protector according to any one of the first to thirteenth preferred embodiments of Invention A, wherein the peripheral wall is transparent or translucent to allow transmission of light, and light transparency of the peripheral wall at the injection gate trace is lower than that of a rest of the peripheral wall.

According to the needle tip protector of the present preferred embodiment, the injection gate trace can be easily visually confirmed due to the difference in light transparency. Thus, the orientation of the needle tip protector or the like can be easily grasped by using the injection gate trace as a mark.

A fifteenth preferred embodiment of Invention A provides a needle assembly including: a puncture needle body having a puncture needle; and a needle tip protector configured to move in a needle axis direction with respect to the puncture needle body to cover a needle tip of the puncture needle, wherein the needle tip protector comprises the needle tip protector according to any one of the first to the fourteenth preferred embodiments, and at least a part of an outer surface of the thick-walled part of the needle tip protector comprises a touch part configured to touch a skin of a patient, and the recess according to the second preferred embodiment opens onto the touch part.

According to the needle assembly of the present preferred embodiment, by providing the recess in the touch part of the needle tip protector, the area of the touch part configured to touch the body surface of the patient is made small, thereby reducing pain and sense of discomfort due to the touch by the touch part.

A sixteenth preferred embodiment of Invention A provides the needle assembly according to the fifteenth preferred embodiment of Invention A, wherein the touch part is configured to touch the skin of the patient when the puncture needle body is indwelled.

According to the needle assembly of the present preferred embodiment, the recess is provided in the touch part that is configured to touch the patient when the indwelling needle serving as the puncture needle body is indwelled, and the contact area of the touch part with the patient is made small. This configuration reduces the pain and sense of discomfort caused for the patient due to the contact by the touch part during indwelling.

A seventeenth preferred embodiment of Invention A provides the needle assembly according to the fifteenth or sixteenth preferred embodiment of Invention A, wherein the injection gate trace is provided at a position away from the touch part.

According to the needle assembly of the present preferred embodiment, the contact of the injection gate trace with the patient is avoided, so that even if the needle tip protector touches the patient, pain or sense of discomfort is less likely to be caused for the patient.

An eighteenth preferred embodiment of Invention A provides the needle assembly according to any one of the fifteenth to seventeenth preferred embodiments of Invention A, wherein on the outer surface of the thick-walled part of the needle tip protector, a protruding pin trace is provided at one of the touch part and a portion located on an opposite side of the touch part in a diametrical direction.

According to the needle assembly of the present preferred embodiment, the protruding pin trace formed by the protruding pin of the mold used for removing the molded article from the mold is located and set in one of the touch part and the portion on the diametrically opposite side of the touch part on the outer surface of the thick-walled part of the needle tip protector. With this configuration, the orientation of the touch part in the needle tip protector can be grasped by using the protruding pin trace as a mark.

A nineteenth preferred embodiment of Invention A provides a needle assembly including: a puncture needle body having a puncture needle; and a needle tip protector configured to move in a needle axis direction with respect to the puncture needle body to cover a needle tip of the puncture needle, wherein the needle tip protector comprises the needle tip protector according to the sixth or seventh preferred embodiment, the expansion part includes a narrow part and a wide part in which an outer dimension is larger than that of the narrow part, and the injection gate trace is provided to a wide part side, and the wide part of the needle tip protector includes an engaging part detachably engaged with an engaging arm provided to a needle hub fixed to a proximal end part of the puncture needle.

According to the needle assembly of the present preferred embodiment, by providing the injection gate (the injection gate trace) in the wide part, a weld line is less likely to be generated in the engaging part. As a result, the strength of the engaging part of the needle tip protector is increased, and the engaging arm of the needle hub can be more firmly engaged with the needle tip protector. Therefore, unintended separation of the needle hub and the needle tip protector or the like can be avoided, thereby preventing malfunction of the needle tip protection mechanism. Further, for example, it is possible to prevent troubles such as the needle tip protection mechanism failing to normally function due to, for example, the engaging part being destroyed by an external force.

In the nineteenth preferred embodiment, it is preferable that the injection gate trace is provided on the proximal end side of the engaging part. The engaging part is often provided at the proximal end part of the needle tip protector, and the strength of the proximal end part of the needle tip protector tends to decrease, so that higher strength is required. Therefore, with such a configuration, the weld line is less likely to be generated on the proximal end side of the engaging part. As a result, the strength of the engaging part is increased, and the safety is further improved.

### EFFECT OF THE INVENTION

According to Invention A, it is possible to stably manufacture the needle tip protector with high safety by molding of a resin such as injection molding.

### BRIEF DESCRIPTION OF THE DRAWINGS

FIG. 1 is a top plan view showing a needle assembly according to a first practical embodiment of Invention A.
FIG. 2 is a bottom plan view of the needle assembly shown in FIG. 1.
FIG. 3 is a front view of the needle assembly shown in FIG. 1.
FIG. 4 is a cross sectional view taken along line 4-4 of FIG. 3.
FIG. 5 is a cross sectional view taken along line 5-5 of FIG. 1.
FIG. 6 is a perspective view of a needle tip protector constituting the needle assembly shown in FIG. 1.
FIG. 7 is a right side view of the needle tip protector shown in FIG. 6.
FIG. 8 is a vertical cross sectional view for explaining a manufacturing process of the needle tip protector shown in FIG. 6.
FIG. 9 is a vertical cross sectional view showing a protected state of a needle tip by the needle tip protector in the needle assembly shown in FIG. 1.
FIG. 10 is a vertical cross sectional view showing a principal part of a needle assembly according to a second practical embodiment of Invention A.
FIG. 11 is a vertical cross sectional view for explaining a manufacturing process of a needle tip protector according to another practical embodiment of Invention A.
FIG. 12 is a perspective view of a needle tip protector according to yet another practical embodiment of Invention A.
FIG. 13 is a perspective view of a needle tip protector according to still yet another practical embodiment of Invention A.
FIG. 14 is a perspective view of a needle tip protector according to a further practical embodiment of Invention A.
FIG. 15 is a top plan view of a needle assembly including a puncture needle body according to a first practical embodiment of Invention B.
FIG. 16 is a front view of the needle assembly shown in FIG. 15.
FIG. 17 is a cross sectional view taken along line 17-17 of FIG. 16.
FIG. 18 is a cross sectional view taken along line 18-18 of FIG. 15.
FIG. 19 is a vertical cross sectional view showing a protected state of a needle tip by a needle tip protector in the needle assembly shown in FIG. 15.
FIG. 20 is a vertical cross sectional view of a puncture needle body according to a second practical embodiment of Invention B.
FIG. 21 is a top plan view of a needle assembly according to a first practical embodiment of Invention C.
FIG. 22 is a front view of the needle assembly shown in FIG. 21.
FIG. 23 is a cross sectional view taken along line 23-23 of FIG. 22.
FIG. 24 is an enlarged view of a principal part of FIG. 23.
FIG. 25 is a cross sectional view taken along line 25-25 of FIG. 21.
FIG. 26 is a perspective view of a needle tip protector constituting the needle assembly shown in FIG. 21.
FIG. 27 is a right side view of the needle tip protector shown in FIG. 26.
FIG. 28 is a cross sectional view showing a state in which a needle hub is being pulled out to a proximal end side with respect to the needle tip protector in the needle assembly shown in FIG. 25, and showing a state in which tilt of an indwelling needle is limited by a contact part.
FIG. 29 is a cross sectional view showing a state in which the needle hub is pulled out to a proximal end side to reach a position where a needle tip of a puncture needle coincides with a distal end of the needle tip protector in the needle assembly shown in FIG. 25, and showing a state in which the tilt of the indwelling needle is limited by a contact part.
FIG. 30 is a cross sectional view showing the needle assembly shown in FIG. 21 with the needle tip protected, corresponding to FIG. 34.
FIG. 31 is an enlarged cross sectional view of a principal part of FIG. 30.
FIG. 32 is a cross sectional view of a principal part of a needle assembly including a needle tip protector according to another practical embodiment of Invention C with a needle tip protected, corresponding to FIG. 31.
FIG. 33 is a top plan view of a needle tip protector-attached needle assembly according to a first practical embodiment of Invention D.
FIG. 34 is a front view of the needle tip protector-attached needle assembly shown in FIG. 33.
FIG. 35 is a cross sectional view taken along line 35-35 of FIG. 33.
FIG. 36 is a cross sectional view taken along line 36-36 of FIG. 35.
FIG. 37 is a perspective view of a second component of a needle hub constituting the needle tip protector-attached needle assembly shown in FIG. 33.

### EMBODIMENTS FOR CARRYING OUT THE INVENTION

Hereinafter, practical embodiments of Invention A will be described with reference to the drawings.

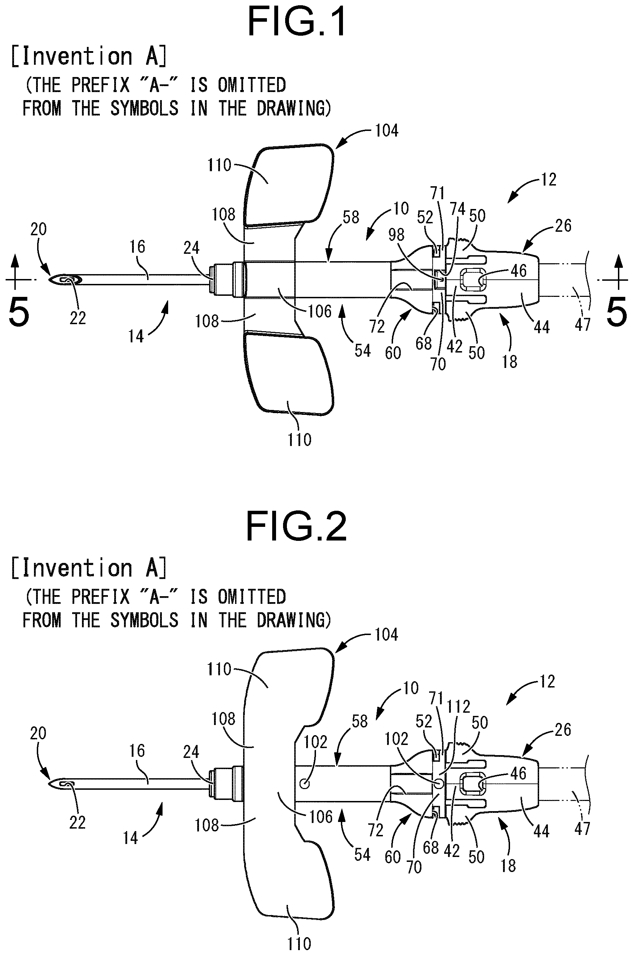

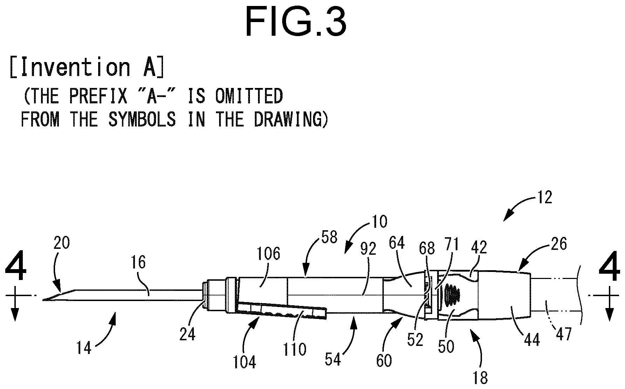

FIGS. 1 to 5 show a needle assembly A-12 including a needle tip protector A-10 according to a first practical embodiment of Invention A. The needle assembly A-12 has a structure in which a puncture needle body A-14 is movably inserted through the needle tip protector A-10 in the needle axis direction. In the following description, unless otherwise specified, the distal end side refers to the left side in FIG. 1 which is a needle tip A-20 side of the puncture needle body A-14, which will be described later, and the proximal end side refers to the right side in FIG. 1, which is the rear side of the puncture direction of the puncture needle body A-14. Further, as a general rule, the vertical direction refers to the vertical direction in FIG. 3, and the left-right direction refers to the vertical direction in FIG. 1, which is the horizontal direction. In FIGS. 1 to 14 according to Invention A, the description of "A-" is omitted in each reference numeral "A-XX" (where XX is a number corresponding to the part) for the sake of legibility. Besides, in order to make it easier to understand that FIGS. 1 to 14 are drawings according to Invention A, FIGS. 1 to 14 are labelled with [Invention A].

More specifically, the puncture needle body A-14 includes a puncture needle A-16 and a needle hub A-18. The puncture needle A-16 is made of a metal such as stainless steel, and has a sharp, tapered needle tip A-20 at one end. A through hole A-22 is formed at the distal end portion of the puncture needle A-16, so that blood can easily flow into the lumen of the puncture needle A-16.

The needle hub A-18 has an approximately cylindrical shape overall, and the proximal end portion of the puncture needle A-16 is fixed to the distal end portion of the needle hub A-18 in an inserted state. With this configuration, the lumen of the needle hub A-18 and the lumen of the puncture needle A-16 communicate with each other in the needle axis direction. The needle hub A-18 includes a needle support part A-24 forming its distal end portion and a protector coupling part A-26 forming its proximal end portion.

The needle support part A-24 has an approximately cylindrical shape, and is formed of, for example, a rigid resin. The inner diameter dimension at the distal end portion of the needle support part A-24 is approximately the same as the outer diameter dimension of the puncture needle A-16. An annular positioning protrusion A-28 that protrudes radially inward is provided on the inner circumferential surface of the distal end portion of the needle support part A-24. The proximal end portion of the puncture needle A-16 is inserted from the opening on the distal end side of the needle support part A-24, and the proximal end of the puncture needle A-16 and the positioning protrusion A-28 come into contact with each other, so that the puncture needle A-16 is positioned with respect to the needle hub A-18 in the needle axis direction (the left-right direction in FIG. 1). The puncture needle A-16 and the needle support part A-24 may be adhered or the like to each other, if necessary.

The outer circumferential surface of the middle portion in the needle axis direction of the needle support part A-24 comprises a tapered surface A-30 that gradually increases in diameter toward the distal end side. With this configuration, in the needle support part A-24, the middle portion in the needle axis direction having the tapered surface A-30 comprises a small-diameter tube part A-32 having a diameter smaller than that of the distal end portion and the proximal end portion.

A locking concave part A-34 that opens onto the outer circumferential surface is formed at the further distal end portion of the needle support part A-24 than the tapered surface A-30. The locking concave part A-34 has an annular shape that is continuous with an approximately constant cross-sectional shape over the entire circumference. The minimum outer diameter dimension of the needle support part A-24 at the location where the locking concave part A-34 is formed is approximately the same as the outer diameter dimension of the proximal end of the small-diameter tube part A-32, which is the minimum outer diameter dimension thereof.

An annular large-diameter fulcrum part A-36 projects from the outer circumferential surface of the needle support part A-24 on the distal end side with respect to the locking concave part A-34. The large-diameter fulcrum part A-36 is provided at the distal end or a position close to the distal end of the needle support part A-24, and constitutes the distal end portion of the needle support part A-24 in the present practical embodiment. The outer diameter dimension of the needle support part A-24 in the portion forming the large-diameter fulcrum part A-36 is larger than the outer diameter dimension of the distal end of the small-diameter tube part A-32, which is the maximum outer diameter dimension thereof.

The proximal end side surface forming the inner surface of the locking concave part A-34 comprises an annular proximal-end-side restricting surface A-38 extending in the axis-perpendicular direction, and the bottom surface of the locking concave part A-34 and the tapered surface A-30 are continuous in a stepped manner via the proximal-end-side restricting surface A-38. The distal end side surface forming the inner surface of the locking concave part A-34 comprises an annular distal-end-side restricting surface A-40 extending in the axis-perpendicular direction, and the bottom surface of the locking concave part A-34 and the outer circumferential surface of the large-diameter fulcrum part A-36 are continuous in a stepped manner via the distal-end-side restricting surface A-40. The dimension in the axis-perpendicular direction of the proximal-end-side restricting surface A-38 formed by the proximal end side inner surface of the locking concave part A-34 is slightly larger than the dimension in the axis-perpendicular direction of the distal-end-side restricting surface A-40.

The protector coupling part A-26 is made of a rigid resin, like the needle support part A-24, for example. The protector coupling part A-26 has a stepped, approximately cylindrical shape overall, and has a coupling tube A-42 having a small diameter on the distal end side and a connecting tube A-44 having a large diameter on the proximal end side. The proximal end portion of the needle support part A-24 is inserted into the coupling tube A-42, and adhesion or welding is performed as necessary, so that the protector coupling part A-26 is fixed to the needle support part A-24. An approximately rectangular concave part A-46 is formed on the surface of the protector coupling part A-26 in the vertical direction (the vertical direction in FIG. 3). As shown in FIG. 1, the concave part A-46 of the present practical embodiment is provided on the surface of the coupling tube A-42, but may be provided on the surface of the connecting tube A-44, or may be provided so as to straddle the coupling tube A-42 and the connecting tube A-44.

The protector coupling part A-26 is fixed to an external conduit A-47 by the distal end of the external conduit A-47 being inserted into the connecting tube A-44 and adhered or welded as necessary. By the proximal end portion (the needle support part A-24) of the needle hub A-18 and the external conduit A-47 being connected to the opposite sides of the protector coupling part A-26, the lumen of the puncture needle A-16 and the lumen of the needle hub A-18 communicate with the external conduit A-47, and a fluid flow path A-48 from the distal end opening of the puncture needle A-16 to the external conduit A-47 is provided to the puncture needle body A-14.

The connecting tube A-44 is provided with a pair of engaging arms A-50, 50 extending toward the distal end side. The engaging arms A-50, 50 are arranged in opposition to each other in the diametrical direction, and extend further to the distal end side than the coupling tube A-42 in the outer periphery of the coupling tube A-42. The engaging arms A-50, 50 are arranged apart from the coupling tube A-42 to the radially outer side, and the connecting portion with the connecting tube A-44 is thin and narrow, so as to be elastically deformable in the direction of approaching each other by the action of an external force.

A hook A-52 is formed at the distal end portion of each of the engaging arms A-50, 50. The hook A-52 projects outward in the direction of opposition of the engaging arms A-50, 50 in each engaging arm A-50. The pair of hooks A-52, 52 are configured to move in the direction of approaching each other by the engaging arms A-50, 50 elastically deforming in the direction of approach.

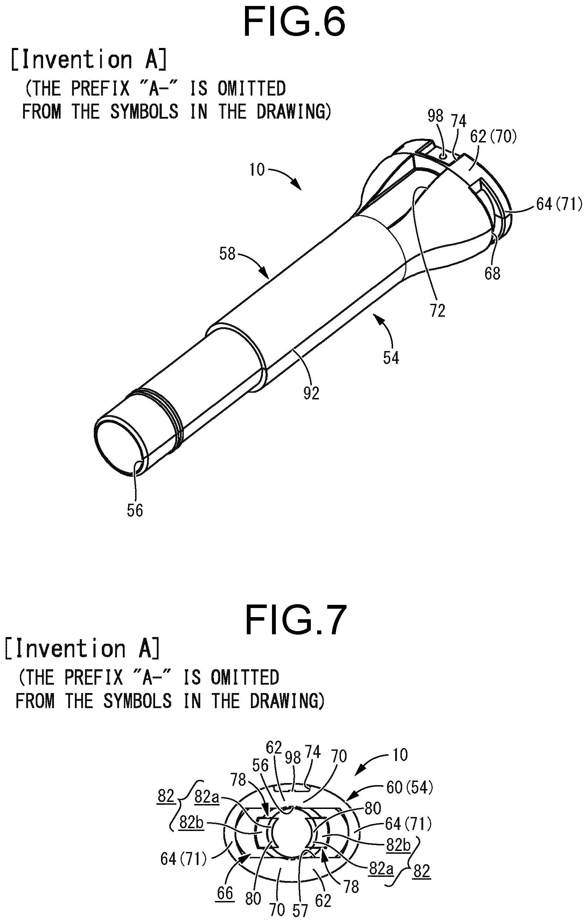

As shown in FIG. 6, the needle tip protector A-10 has an approximately tubular shape extending in the needle axis direction overall, and is integrally molded by using a rigid synthetic resin such as polypropylene, polycarbonate, polyethylene terephthalate glycol, and ABS resin. The needle tip protector A-10 is preferably transparent or translucent to allow transmission of visible light. The needle tip protector A-10 is provided with a tubular peripheral wall A-54, and an inner hole A-56 penetrating in the needle axis direction is formed in the radial inside of the peripheral wall A-54. The needle tip protector A-10 has no air bubbles, sink marks, voids, etc. in the peripheral wall A-54. The needle tip protector A-10 has a proximal end side opening A-57 that opens at the proximal end in the needle axis direction.

The peripheral wall A-54 has a cylindrical part A-58 serving as a tubular part having an approximately perfect circular ring-shaped cross section on the distal end side, and has an expansion part A-60 having an outer dimension larger than that of the cylindrical part A-58 and extending radially outward on the proximal end side. Thus, the shape of the peripheral wall A-54 is varied in the needle axis direction. Since the expansion part A-60 has a larger outer dimension than the cylindrical part A-58, the length in the circumferential direction is longer than that of the cylindrical part A-58.

As shown in FIG. 7, the expansion part A-60 has an approximately elliptical tubular shape, and the width dimension (the outer dimension) of the outer circumferential surface in the left-right direction (the horizontal direction) in FIG. 7, which is the large-diameter direction, is larger than the width dimension (the outer dimension) of the outer circumferential surface in the vertical direction in FIG. 7, which is the small-diameter direction. Of the wall parts that make up the expansion part A-60, the parts that make up the wall part in the vertical direction comprise narrow parts A-62, 62 with small outer dimensions, while the parts that make up the wall part in the horizontal direction comprise wide parts A-64, 64 with large outer dimensions. The direction of opposition of the narrow parts A-62, 62 and the direction of opposition of the wide parts A-64, 64 are approximately orthogonal to each other. In this practical embodiment, a mode in which the expansion part A-60 has different dimensions in the vertical direction and the horizontal direction will be described, but the present invention is not limited to this mode. For example, the dimensions of the expansion part in the vertical direction and in the horizontal direction may be approximately the same. Further, it is possible to adopt various types of shape as the cross-sectional shapes of the expansion part A-60 on the distal end side and the proximal end side of the needle tip protector, such as a circular shape (including an ellipse, an oval, etc.) and a polygonal shape. However, the expansion part provided on the proximal end side of the needle tip protector is not essential, and the needle tip protector may have a simple, straight tubular shape.

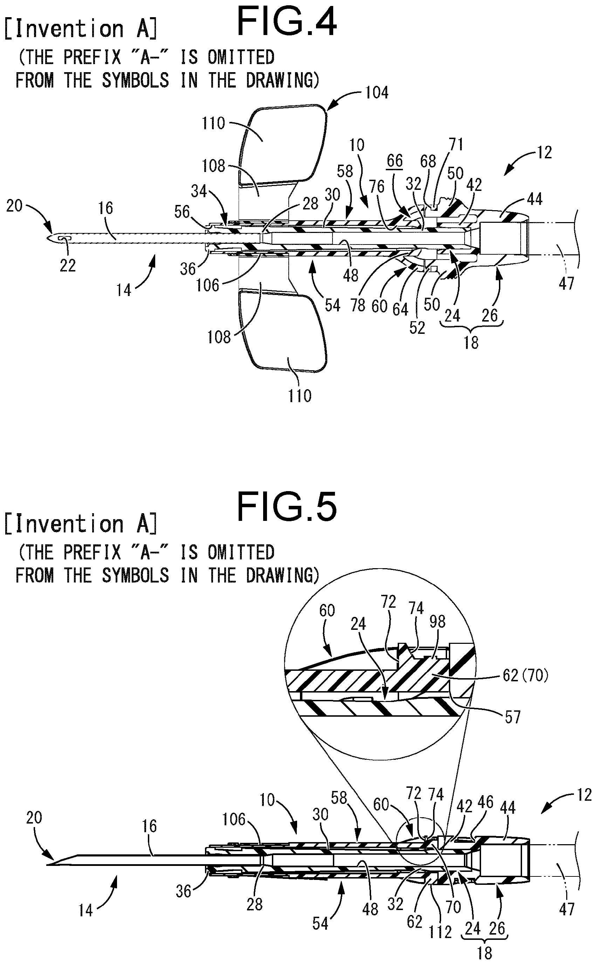

As shown in FIGS. 4 to 6, the outer circumferential surfaces of the wide parts A-64, 64 gradually increase in outer diameter dimension from the distal end side to the proximal end side. The outer circumferential surface of the cylindrical part A-58 and the outer circumferential surface of the wide parts A-64, 64 are smoothly connected. The wall thickness dimension from the cylindrical part A-58 to the wide parts A-64, 64 is approximately constant in the needle axis direction. The wide parts A-64, 64 have an approximately elliptical arc cross section in which the outer circumferential surface and the inner circumferential surface correspond to each other, and the thickness dimension is approximately constant in the circumferential direction. In the radial inside of the expansion part A-60, an internal space A-66 having a flat cross section with its horizontal dimension larger than its vertical dimension is formed at the proximal end portion of the inner hole A-56 penetrating the needle tip protector A-10. The inside dimension in the horizontal direction of the internal space A-66 gradually increases toward the proximal end side.

The wide parts A-64, 64 are provided with through windows A-68, 68 serving as engaging parts that penetrate in the plate thickness direction. The circumferential dimension of the through windows A-68, 68 is larger than the circumferential dimension of the hooks A-52, 52. In the present practical embodiment, when the needle tip protector A-10 is molded, the mold for molding the outer circumferential surface is vertically split. Thus, the through windows A-68, 68 have a shape that will not cause any undercut at the time of mold release in the vertical direction.

The narrow parts A-62, 62 have an outer circumferential surface of approximately elliptical cross section, and an inner circumferential surface of approximately flat surface orthogonal to the direction of opposition of the narrow parts A-62, 62. With this configuration, the narrow parts A-62, 62 are gradually thickened toward the center in the circumferential direction, and the central portions in the circumferential direction comprise thick-walled parts A-70, 70. In other words, the needle tip protector A-10 includes the thick-walled parts A-70, 70 including a portion whose thickness dimension is larger than the thickness dimension in the distal end portion.

As shown in FIG. 7, the thick-walled parts A-70, 70 of the narrow parts A-62, 62 include the portion whose thickness dimension is larger than the thickness dimensions of the cylindrical part A-58 and the wide parts A-64, 64. The narrow parts A-62, 62 having a diameter smaller than that of the wide parts A-64, 64 are thicker than the wide parts A-64, 64 at least in the thick-walled parts A-70, 70. With this configuration, the internal space A-66 of the expansion part A-60 has a horizontally elongated flat shape having an aspect ratio larger than that of the outer circumferential surface of the expansion part A-60 viewed in the axial direction of the peripheral wall A-54. Further, the peripheral wall portion of the proximal end side opening A-57 includes the thick-walled parts A-70, 70 on the narrow parts A-62, 62 side and bridge parts A-71, 71 on the wide parts A-64, 64 side. The bridge part A-71 is a portion of the wide part A-64 that extends in the circumferential direction on the proximal end side with respect to the through window A-68, and the inner shape has an approximately arc shape. The peripheral wall A-54 at the proximal end of the needle tip protector A-10 and the inner shapes of the bridge parts A-71, 71 have corresponding shapes. With such a configuration, it is possible to prevent the connection location between the bridge parts A-71, 71 and the thick-walled parts A-70, 70 from becoming thin, thereby easily securing the strength.

As shown in FIGS. 1 and 5, a recess A-72 opens onto the outer surface of each of the thick-walled parts A-70, 70 on the upper and lower opposite sides. The recesses A-72, 72 have an approximately rectangular opening shape. The recesses A-72, 72 are provided at the distal end portions of the thick-walled parts A-70, 70, and are notch-shaped recesses that open onto both the outer circumferential surface and the distal end surface that form the outer surface of the thick-walled parts A-70, 70. It is desirable that the opening end edges of the recesses A-72, 72 have a curved cross-sectional shape with no corners. The bottom surfaces of the recesses A-72, 72 are flat surfaces extending approximately orthogonal to the vertical direction, and the depth dimension of the recesses A-72, 72 gradually decreases toward the distal end side.

A concavity A-74 serving as a recess opens onto the outer surface of the upper thick-walled part A-70. The concavity A-74 has an approximately rectangular opening shape. The concavity A-74 is a notch-shaped recess that opens onto both the outer circumferential surface and the proximal end surface that form the outer surface of the thick-walled part A-70. It is desirable that the opening end edge of the concavity A-74 has a curved cross-sectional shape with no corners. The concavity A-74 is arranged in the central portion circumferentially between the through windows A-68, 68, and is remote from the through windows A-68, 68 in the circumferential direction. In the portion forming the concavity A-74 in the thick-walled part A-70, the bottom wall part of the concavity A-74 is preferably thicker than the cylindrical part A-58, but may be thinner than the cylindrical part A-58. The depth dimension of the concavity A-74 is smaller than the maximum depth dimension of the recess A-72. With this configuration, the expansion part A-60 is unlikely to be excessively thin due to the formation of the concavity A-74, and the strength required for the needle tip protector A-10 can be secured in the expansion part A-60. The expansion part A-60 of the needle tip protector A-10 is a place where locking pieces A-78, 78 (flexible pieces) described later are arranged. Therefore, it is important that the expansion part A-60 has the necessary strength for enhancing the safety of the needle tip protector A-10. Besides, the depth dimension of the concavity A-74 may be larger than the maximum depth dimension of the recess A-72. With this configuration, in the peripheral wall A-54, the strength is increased in the portion forming the recess A-72 rather than in the portion forming the concavity A-74, thereby making it possible to secure the required strength further on the distal end side than the concavity A-74.

As shown in FIG. 4, an annular stepped surface A-76 extending in the axis-perpendicular direction is formed on the inner circumferential surface of the peripheral wall A-54 at the front end portion of the expansion part A-60. The inner circumferential surface of the peripheral wall A-54 has a diameter larger on the distal end side of the stepped surface A-76 than on the proximal end side thereof. The dimension in the axis-perpendicular direction of the stepped surface A-76 is smaller than the dimension in the axis-perpendicular direction of the distal-end-side restricting surface A-40 of the locking concave part A-34.

Further, the peripheral wall A-54 is provided with a pair of locking pieces A-78, 78 (flexible pieces) protruding from the inner circumferential surface of the expansion part A-60 to the internal space A-66. As shown in FIG. 4, the locking pieces A-78, 78 are integrally formed with the wide parts A-64, 64 of the expansion part A-60 of the peripheral wall A-54. The locking pieces A-78, 78 are arranged at positions corresponding to the wide parts A-64, 64 on the radially inner side of the wide parts A-64, 64. The locking pieces A-78, 78 are provided in opposition to each other in the horizontal direction and are remote from each other in the circumferential direction. The locking pieces A-78, 78 project toward the proximal end side of the peripheral wall A-54 on the proximal end side of the stepped surface A-76. The locking pieces A-78, 78 extend from the stepped surface A-76 toward the proximal end side approximately parallel to the needle axis direction, and at least the protruding tip portion is arranged away from the wide parts A-64, 64 of the peripheral wall A-54 to the radially inner side. The locking pieces A-78, 78 are each curved in the circumferential direction. The locking pieces A-78, 78 are flexible plate-shaped members. The locking pieces A-78, 78 are not limited to a pair, and it is sufficient that at least one locking piece A-78 is provided.

A pair of locking claws A-80, 80 that bend and project radially inward are formed at the respective protruding tips (the proximal ends in the needle axis direction) of the locking pieces A-78, 78. As shown in FIG. 7, the inner circumferential surfaces of the locking claws A-80, 80 are each curved in the circumferential direction, and the radius of curvature of the inner circumferential surface at the protruding tip of the locking claws A-80, 80 is approximately equal to the radius of curvature of the outer circumferential surface of the small-diameter tube part A-32 of the needle support part A-24. Further, the distance between the opposed faces in the diametrical direction at the protruding tips of the locking claws A-80, 80 is approximately equal to the outer diameter dimension of the small-diameter tube part A-32, while being smaller than the maximum outer diameter dimension of the distal end portion of the tapered surface A-30. However, the distance between the opposed faces in the diametrical direction at the protruding tips of the locking claws A-80, 80 may be slightly smaller or slightly larger than the outer diameter dimension of the small-diameter tube part A-32.

As shown in FIG. 4, regarding proximal end side end faces A-82, 82 of the respective locking claws A-80, 80, the radially inner portions constitute small wall parts A-82a, 82a extending in the axis-perpendicular direction, while the radially outer portions constitute inclined surfaces A-82b, 82b inclining toward the distal end side in the needle axis direction toward the radially outer side. In the present practical embodiment, the dimension in the axis-perpendicular direction of the small wall parts A-82a, 82a is approximately the same as or slightly larger than the dimension in the axis-perpendicular direction of the proximal-end-side restricting surface A-38 of the locking concave part A-34. With this configuration, when the locking claws A-80, 80 come into contact with the proximal-end-side restricting surface A-38 as described later, the entire surface of the proximal-end-side restricting surface A-38 comes into contact with the small wall parts A-82a, 82a, thereby obtaining a sufficiently large contact area. Furthermore, since the inclined surfaces A-82b, 82b located on the radially outer side of the small wall parts A-82a, 82a incline toward the distal end side in the needle axis direction toward the radially outer side, the small wall parts A-82a, 82a and the proximal-end-side restricting surface A-38 come into contact with each other without any interference by the inclined surfaces A-82b, 82b.

Besides, the protruding tips (the proximal ends in the needle axis direction) of the locking claws A-80, 80 are located on the distal end side in the needle axis direction with respect to the proximal end of the expansion part A-60. Therefore, the entire locking pieces A-78, 78 are housed in the internal space A-66 of the expansion part A-60.

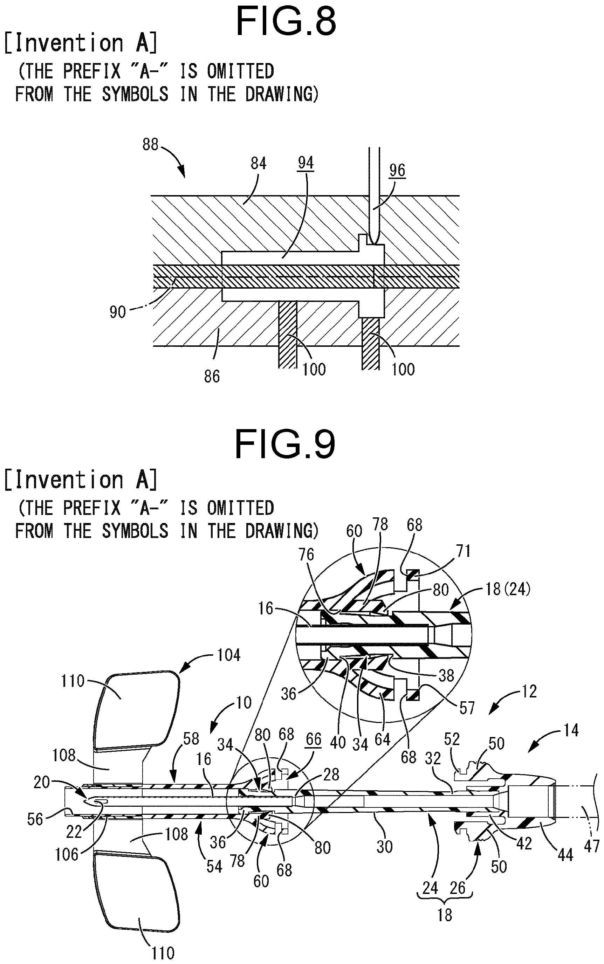

As shown in FIG. 8, the needle tip protector A-10 has an outer peripheral shape molded by a mold A-88 configured including split molds A-84, 86 combined in the axis-perpendicular direction. By the outer circumferential surface of the needle tip protector A-10 being molded by the split molds A-84, 86 combined in the vertical direction, as shown in FIG. 3, a parting line A-92 that protrudes from a parting surface A-90 of the split molds A-84, 86 is formed so as to extend in the axial direction on the horizontally outer surface of the needle tip protector A-10. With this configuration, the parting line A-92 is less likely to touch the patient, thereby avoiding a pain or the like caused by the touch of the parting line A-92. By forming the needle tip protector A-10 so as not to have the parting line A-92, it is possible to avoid a pain due to touch of the parting line A-92.

As shown in FIGS. 1 and 5, an injection gate trace A-98, which is a trace of an injection gate A-96 that fills the cavity A-94 of the mold A-88 with the resin material when molding the needle tip protector A-10, is provided on the outer surface of the thick-walled part A-70, which is the central portion in the circumferential direction of one of the narrow parts A-62. In the present practical embodiment, the injection gate A-96 is provided at one position in the circumferential direction, and the injection gate trace A-98 is provided on the inner surface of the concavity A-74 that opens onto the outer surface of the upper thick-walled part A-70. By providing one injection gate A-96 in the narrow part A-62, it is possible to prevent occurrence of weld lines at the bridge parts A-71, 71 that tends to be thin, while preventing a weld line from being generated in the narrow part A-62 on the injection gate A-96 side. As a result, the strength of the bridge parts A-71, 71 can be improved. Since the injection gate trace A-98 is provided in the narrow part A-62, the injection gate trace A-98 is provided at a position circumferentially away from the through windows A-68, 68 and the locking pieces A-78, 78 provided on the wide parts A-64, 64 side.

As can be seen from FIG. 8, the resin material injected from the injection gate A-96 is poured (injected) into the cavity A-94 from the portion where the thick-walled part A-70 is molded. By so doing, the resin material is charged from the wide portion having a large cross-sectional area to the narrow portion having a small cross-sectional area in the cavity A-94. Therefore, when the resin material is charged into the cavity, poor charge of the resin material due to pressure loss is unlikely to occur, and the needle tip protector A-10 can be manufactured with stable quality.

The cross-sectional area of the needle tip protector A-10 in the axial direction or the cross-axis direction is preferably not greater than the cross-sectional area of the injection gate trace A-98 on the proximal end side of the injection gate trace A-98, while being preferably not greater than the cross-sectional area of the injection gate trace A-98 on the distal end side of the injection gate trace A-98. However, the cross-sectional area is not limited to such an embodiment, and between the injection gate trace A-98 and the distal end or the proximal end of the needle tip protector A-10, there may be provided a portion whose cross-sectional area is larger than the cross-sectional area in the injection gate trace A-98. Further, it is preferable that the amount of change in the said cross-sectional area (the volume) gradually changes. However, it is not limited to such an embodiment, and there may be some parts whose cross-sectional area (the volume) does not gradually change, for example, the cross-sectional area (the volume) changes in a stepwise manner, as long as voids, sink marks, etc. do not occur.

Moreover, since the injection gate trace A-98 is housed in the concavity A-74, while preventing the patient or the like from touching the injection gate trace A-98, it is possible to set the position of the injection gate A-96 to the thick-walled, large-diameter proximal end portion of the needle tip protector A-10 to efficiently manufacture the needle tip protector A-10.

The injection gate A-96 is a pin gate that is pulled and cut off when the split molds A-84, 86 are demolded. By so doing, the injection gate trace A-98 is likely to be formed so as to protrude from the bottom surface of the concavity A-74, and the sense of discomfort and pain are likely to occur when the needle assembly A-12 is placed on the skin. The protruding height of the injection gate trace A-98 is smaller than the depth of the concavity A-74, and the injection gate trace A-98 protruding from the bottom surface of the concavity A-74 is located on the bottom side from the open end surface of the concavity A-74, and is housed in the concavity A-74.

The injection gate trace A-98 has lower light transparency than the rest of the peripheral wall A-54 when pulled and cut off. That is, the injection gate trace A-98 absorbs more visible light passing therethrough than the rest of the peripheral wall A-54 does, and the transparency is lowered. This makes it possible to easily visually confirm the injection gate trace A-98. Therefore, for example, the position of the injection gate trace A-98 can be used as a mark for visually grasping the orientation of the needle tip protector A-10 with respect to the puncture needle body A-14 in the circumferential direction.

When the injection gate trace A-98 is provided on the outer circumferential surface of the expansion part A-60, it is desirable that the injection gate trace A-98 is provided in a direction approximately orthogonal to the tangential direction of the outer circumferential surface in the transverse cross section of the expansion part A-60. More preferably, when the injection gate trace A-98 is provided on the outer circumferential surface of the expansion part A-60, the injection gate trace A-98 is provided on at least one of the minor axis and the major axis of the expansion part A-60 having an elliptical tubular shape. With this configuration, the resin injected from the injection gate A-96 into the cavity A-94 of the mold A-88 easily spreads uniformly in the cavity A-94, and molding defects such as shrinkage cavities are less likely to occur.

Further, the portion of the inner circumferential surface of the expansion part A-60 corresponding to the injection gate trace A-98 may have a concave shape so as to be convex toward the injection gate trace A-98. With this configuration, the resin injected from the injection gate A-96 into the cavity A-94 is guided to the opposite sides in the circumferential direction by the convex part of the mold A-88 that forms the concave shape, and the resin material is readily distributed to the opposite sides in the circumferential direction of the cavity A-94, so that the molding defects are less likely to occur.

The mold for molding the needle tip protector A-10 is provided with protruding pins A-100, 100 for demolding in addition to the split molds A-84, 86. The protruding pin A-100 extends in the vertical direction, which is the direction of demolding the split molds A-84, 86, and the protruding tip surface forms a part of the wall inner surface of the cavity A-94. Then, after the molding of the resin molded article is completed, the split molds A-84, 86 are opened, and the resin molded article is pushed out by the protruding pins A-100, 100, so that the resin molded article (the needle tip protector A-10) is removed from the split mold A-84. In this way, the wall inner surface of the cavity A-94 is partially formed by the tip surfaces of the protruding pins A-100, 100, so that the outer circumferential surface of the needle tip protector A-10 includes two protruding pin traces A-102, 102 as shown in FIG. 2. The protruding pin traces A-102, 102 have a shallow circular concave shape. The protruding pin traces A-102, 102 are formed on the lower surface of the needle tip protector A-10, one of which is provided on the lower surface of the cylindrical part A-58 and the other of which is provided on the outer circumferential surface of the lower thick-walled part A-70 of the expansion part A-60. By providing such protruding pin traces A-102, 102 in a part in the circumferential direction, the protruding pin traces A-102, 102 can be utilized as a mark for specifying the orientation of the needle tip protector A-10 in the circumferential direction. The formation position of the protruding pin traces A-102 is not particularly limited, and one or three or more protruding pin traces A-102 may be provided.

As shown in FIG. 1, a wing-shaped part A-104 is provided at the distal end portion of the needle tip protector A-10. The wing-shaped part A-104 is formed of, for example, a soft resin. The wing-shaped part A-104 is integrally formed with a tubular fitting tube part A-106 with plate-shaped coupling parts A-108, 108 protruding in the tangential direction of the fitting tube part A-106. Besides, a wing main body A-110 is integrally formed on the protruding tip side of each of the coupling parts A-108, 108 from the fitting tube part A-106. The wing-shaped part A-104 is attached to the peripheral wall A-54 of the needle tip protector A-10 by the fitting tube part A-106 being fitted externally onto the distal end portion of the cylindrical part A-58. Note that FIG. 7 shows a resin molded article of the needle tip protector A-10 before the wing-shaped part A-104 is attached. The wing-shaped part A-104, which is a separate component from the needle tip protector A-10 of FIG. 7, is attached in an externally fitted state.

The puncture needle body A-14 is inserted through the inner hole A-56 of the needle tip protector A-10 from the opening on the proximal end side, so that the needle tip protector A-10 is externally mounted onto the puncture needle body A-14, thereby constituting the needle assembly A-12. In the initial state before use of the needle assembly A-12 shown in FIG. 1 and the like, the needle tip A-20 of the puncture needle body A-14 is located on the distal end side with respect to the needle tip protector A-10, and the needle tip A-20 is exposed. In this initial state, as shown in FIG. 4, the hooks A-52, 52 provided to the needle hub A-18 of the puncture needle body A-14 are inserted and locked in the through windows A-68, 68 provided to the expansion part A-60 of the needle tip protector A-10. With this configuration, the needle tip protector A-10 and the needle hub A-18 are coupled to each other, and the protruding state of the needle tip A-20 is maintained. Besides, for example, when puncture is performed by holding the wing-shaped part A-104, the movement of the puncture needle A-16 toward the proximal end side in the needle axis direction due to resistance during the puncture or the like is restricted.

In the initial state of the needle assembly A-12, the locking claws A-80, 80 of the needle tip protector A-10 are in contact with the outer circumferential surface of the small-diameter tube part A-32 of the needle support part A-24. These locking claws A-80, 80 may be slightly pushed radially outward by being in contact with the outer circumferential surface of the small-diameter tube part A-32, or may be slightly remote from the outer circumferential surface of the small-diameter tube part A-32.

The needle assembly A-12 is used for infusion, blood collection, and hemodialysis through the fluid flow path A-48 by the puncture needle A-16 being stuck into the patient's blood vessel and the puncture needle body (the indwelling needle) 14 being indwelled therein. Since the needle assembly A-12 of the present practical embodiment is provided with the wing-shaped parts A-104, it is possible to stick the puncture needle A-16 of the puncture needle body A-14 while, for example, pinching the wing-shaped parts A-104. When indwelling the puncture needle body A-14 in the punctured state, by fixing with a tape fastener at the positions of the wing-shaped parts A-104, it is possible to fix to the skin with a wide touch area.

With the puncture needle body A-14 indwelled, the central portion in the circumferential direction of the thick-walled part A-70 on the lower side of the needle tip protector A-10 (the lower side in FIG. 7) touches the patient's skin. Therefore, the central portion in the circumferential direction on the lower surface of the lower thick-walled part A-70 comprises a touch part A-112 of the present practical embodiment. The recess A-72 opens in the touch part A-112. At least a part of the inner surface of the recess A-72 is configured not to touch the patient, and the touch area of the touch part A-112 on the patient is reduced. By so doing, pain and sense of discomfort when the touch part A-112 touches the patient can be reduced. In particular, if the opening end edge of the recess A-72 has a curved cross-sectional shape with no corners, the opening end edge of the recess A-72 is less likely to get caught in the patient's skin, thereby reducing pain and sense of discomfort due to touch by the opening end edge of the recess A-72. Further, the injection gate trace A-98 is provided on the thick-walled part A-70 on the upper side (the upper side in FIG. 7) opposite to the touch part A-112 on the patient. Since the injection gate trace A-98 is placed in a position away from the touch part A-112 the patient's skin, the injection gate trace A-98 does not touch the patient's skin. In FIG. 5, the body surface of the patient in a state where the puncture needle body A-14 is stuck and indwelled in the patient is hypothetically illustrated by the chain double-dashed line.

Further, when a health care worker touches the thick-walled parts A-70, 70 of the needle tip protector A-10 during the puncture of the puncture needle body A-14 and the removal thereof described later, the opening area of the concavity A-74 that opens onto the outer surface of the thick-walled part A-70 is sufficiently small, and the fingertip is less likely to be inserted further to the deep side of the concavity A-74. With this configuration, the health care worker is less likely to touch the injection gate trace A-98 protruding from the bottom surface of the concavity A-74, and the health care worker is less likely to feel pain or sense of discomfort due to the touch by the injection gate trace A-98. In particular, if the opening end edge of the concavity A-74 has a curved cross-sectional shape with no corners, the opening end edge of the concavity A-74 is less likely to get caught by the health care worker's fingertip or the like, thereby reducing pain and sense of discomfort due to touch by the opening end edge of the concavity A-74.

When the puncture needle body A-14 is removed, the engaging arms A-50, 50 of the needle hub A-18 are pressed inward by fingers while the needle tip protector A-10 is kept fixed with a tape fastener at the wing-shaped parts A-104. By so doing, the hooks A-52, 52 and the through windows A-68, 68 are detached from each other, making it possible to move the puncture needle body A-14 toward the proximal end side with respect to the needle tip protector A-10. By moving the puncture needle body A-14 toward the proximal end side with respect to the needle tip protector A-10 and removing the puncture needle A-16 from the skin, the needle tip protector A-10 moves to the needle tip A-20 side with respect to the puncture needle body A-14.

With the hooks A-52, 52 and the through windows A-68, 68 detached from each other, unless an external force is applied to the puncture needle body A-14 to move toward the proximal end side with respect to the needle tip protector A-10, the puncture needle body A-14 is configured not to move toward the proximal end side with respect to the needle tip protector A-10. That is, since the locking claws A-80, 80 are in contact with the tapered surface A-30 that increases in diameter toward the distal end side, the puncture needle body A-14 is prevented from moving toward the proximal end side with respect to the needle tip protector A-10. With this configuration, it is possible to prevent a trouble that the needle tip A-20 of the puncture needle body A-14 is accidentally protected by the needle tip protector A-10.

By moving the puncture needle body A-14 toward the proximal end side with respect to the needle tip protector A-10, the locking claws A-80, 80 come into sliding contact with the tapered surface A-30, and elastic recovery force of the locking pieces A-78, 78 acts as an urging force for pressing the locking claws A-80, 80 against the tapered surface A-30. Therefore, the user can move the puncture needle body A-14 while confirming the feeling that the puncture needle body A-14 is being pulled out from the needle tip protector A-10 by the resistance force due to the contact between the locking claws A-80, 80 and the tapered surface A-30.

As shown in FIG. 9, by the puncture needle body A-14 being moved backward to a predetermined position with respect to the needle tip protector A-10 (the needle tip protector is moved forward to the needle tip A-20 side of the puncture needle body A-14), the needle tip A-20 of the puncture needle A-16 is covered with the needle tip protector A-10. Moreover, the locking claws A-80, 80 provided on the free end side of the locking pieces A-78, 78 move over the opening of the locking concave part A-34, so that the locking claws A-80, 80, which elastically recover their shapes, enter the locking concave part A-34. By so doing, the small wall parts A-82a, 82a of the locking claws A-80, 80 and the proximal-end-side restricting surface A-38 of the locking concave part A-34 are brought into contact and locked to each other. Then, the movement of the puncture needle body A-14 toward the distal end side with respect to the needle tip protector A-10 is restricted, thereby preventing re-exposure of the needle tip A-20 of the puncture needle body A-14.

The stepped surface A-76 provided at the front end portion of the locking pieces A-78, 78 and the distal-end-side restricting surface A-40 of the locking concave part A-34 are brought into contact and locked to each other, so that movement of the puncture needle body A-14 to the proximal end side is restricted. By so doing, the puncture needle body A-14 is prevented from becoming dislodged to the proximal end side with respect to the needle tip protector A-10. Therefore, the puncture needle body A-14 is prevented from moving to the opposite sides in the axial direction with respect to the needle tip protector A-10, thereby maintaining the protected state of the needle tip A-20 by the needle tip protector A-10.

In this practical embodiment, the pair of locking pieces A-78, 78 are provided, and in combination with the fact that the locking pieces A-78, 78 are integrally molded with the peripheral wall A-54 of the needle tip protector A-10, when the locking claws A-80, 80 and the proximal-end-side restricting surface A-38 are locked, troubles such as the needle tip protector A-10 rattling with respect to the puncture needle body A-14 can be effectively prevented. By providing two, or three or more locking pieces A-78 in this way, the needle tip protector A-10 is more reliably prevented from moving backward to the proximal end side with respect to the puncture needle body A-14, thereby more stably exhibiting the effect of preventing re-exposure of the needle tip A-20. In this case, it is preferable that the plurality of locking pieces A-78 are arranged approximately symmetrically with respect to the central axis of the needle tip protector A-10.

With the needle tip A-20 of the puncture needle body A-14 protected by the needle tip protector A-10, the tape fixing of the wing-shaped parts A-104 is released, and the needle assembly A-12 is removed from the patient. However, the procedure for protecting the needle tip A-20 is not limited to the aforementioned one. Specifically, for example, it would also be acceptable to release the tape fixing at the wing-shaped part A-104, and move the entire needle assembly A-12 backward to remove the puncture needle A-16 from the blood vessel, and then move the puncture needle body A-14 backward with respect to the needle tip protector A-10, thereby protecting the needle tip A-20 of the puncture needle A-16.

When the locking claws A-80, 80 enter the locking concave part A-34, the tips of the locking claws A-80, 80 come into contact with the bottom surface of the locking concave part A-34, so that the user can confirm the impact and sound of the contact. Therefore, for example, the risk that the user may stop the pulling-out operation of the puncture needle body A-14 midway or the like is avoided, thereby more reliably protecting the needle tip A-20 with the needle tip protector A-10.

In the needle tip protector A-10 and the needle assembly A-12 having the above-mentioned structure, the locking pieces A-78, 78 that prevent the re-exposure of the needle tip A-20 of the puncture needle body A-14 are provided so as to be entirely housed inside the tubular peripheral wall A-54. This makes it almost impossible to unintentionally touch the locking pieces A-78, 78 from the outside. Therefore, in the protected state of the needle tip A-20 of the puncture needle body A-14 by the needle tip protector A-10, it is possible to effectively prevent the locking pieces A-78, 78 and the locking concave part A-34 from being accidentally disengaged from each other to re-expose the needle tip A-20 from the needle tip protector A-10.

With the locking pieces A-78, 78 locked to the needle hub A-18, for example, when an external force such as a force in the bending direction is applied to the needle tip protector A-10 and/or the needle hub A-18, the locking pieces A-78, 78 may come into contact with the wide parts A-64, 64 located apart to the radially outer side. By so doing, the deformation of the locking pieces A-78, 78 to the radially outer side is restricted, and the risk of the locking pieces A-78, 78 being disengaged from the needle hub A-18 is reduced, thereby stably maintaining the protected state of the needle tip A-20 of the puncture needle body A-14.

The locking pieces A-78, 78 are provided in the expansion part A-60 expanded on the proximal end side of the needle tip protector A-10. With this configuration, while obtaining a sufficient size of the locking pieces A-78, 78, the outer diameter dimension of the needle support part A-24 of the needle hub A-18 inserted between the locking pieces A-78, 78 can also be made large enough. Furthermore, in the present practical embodiment, the expansion part A-60 has a flat and approximately elliptical shape, and the outer circumferential surface thereof is smoothly continuous from the outer circumferential surface of the cylindrical part A-58. This reduces the risk that the expansion part A-60 or the like may come into contact with the patient so that the patient feels pain.

Moreover, regarding the expansion part A-60, in cross sectional view in the cross-axis direction, a curvature of the curve in the direction corresponding to the recess A-72 or the concavity A-74 is gentler than a curvature of the curve in the direction corresponding to the locking pieces A-78, 78. In other words, the recess A-72 or the concavity A-74 is provided in the portion of the expansion part A-60 where the curvature is gentler than the curvature of the curve in the direction corresponding to the locking pieces A-78, 78. The said portion is a portion that can touch the patient at the time of indwelling, and is likely to have an influence on the patient's pain, discomfort feeling or the like due to the touch. However, with the said configuration, the touch area on the patient is reduced, thereby further reducing pain, discomfort feeling or the like during indwelling.

Further, the injection gate trace A-98 is provided on the surface of the needle tip protector A-10 on the side opposite to the surface on the side where the wing main body A-110 of the wing-shaped part A-104 is located. With this configuration, the injection gate trace A-98, which is likely to cause pain to the patient when touching the patient, is provided at a position which is less likely to touch the patient, thereby reducing pain, discomfort feeling or the like during indwelling. Moreover, the injection gate trace A-98 is provided in the recess A-72 or the concavity A-74 provided on the surface opposite to the surface on which the wing main body A-110 is located. With this configuration, pain, discomfort feeling or the like during indwelling can be further reduced.

The locking pieces A-78, 78 are provided inside the wide parts A-64, 64 constituting the expansion part A-60. This prevents the wide parts A-64, 64 from being thick, thereby suppressing mixing of air bubbles in a component during molding, occurrence of dimensional errors and deterioration in quality due to generation of voids and sink marks, and the like. Further, it is preferable that the locking part (the locking pieces A-78, 78) is provided so as to extend in the same direction as the wide parts A-64, 64 extend (to the proximal end side in the axial direction). With this configuration, the expansion part A-60 is unlikely to become larger than necessary, so that the space inside the wide parts A-64, 64 can be used skillfully, and the wide parts A-64, 64 are prevented from being thick.

Since the locking pieces A-78, 78 extend toward the proximal end side, they do not get caught when the puncture needle body A-14 is pulled out with respect to the needle tip protector A-10, thereby realizing smooth pulling out.

The small wall parts A-82a, 82a (the first locking part) of the locking claws A-80, 80 and the proximal-end-side restricting surface A-38 (the third locking part) of the locking concave part A-34, which are mutually locked, all extend in the axis-perpendicular direction. Moreover, the stepped surface A-76 (the second locking part) and the distal-end-side restricting surface A-40 (the fourth locking part) of the locking concave part A-34, which are mutually locked, both extend in the axis-perpendicular direction. Therefore, the needle tip protector A-10 having the small wall parts A-82a, 82a and the stepped surface A-76, as well as the needle hub A-18 having the proximal-end-side restricting surface A-38 and the distal-end-side restricting surface A-40 do not have undercut shape and are easy to mold. Moreover, the contact force and the contact reaction force due to contact between the small wall parts A-82a, 82a and the proximal-end-side restricting surface A-38 act without being inclined with respect to the axial direction. Similarly, the contact force and the contact reaction force due to contact between the stepped surface A-76 and the distal-end-side restricting surface A-40 act without being inclined with respect to the axial direction. Besides, each contact area can be sufficiently obtained, and the movement of the needle tip protector A-10 with respect to the puncture needle body A-14 to the distal end side and to the proximal end side can be more reliably prevented.

An expansion part A-60 is provided at the proximal end part of the peripheral wall A-54 of the needle tip protector A-10. The through windows A-68, 68 and locking pieces A-78, 78 are provided in the wide parts A-64, 64 having a large outer diameter in the expansion part A-60, while the injection gate trace A-98 is provided in the narrow part A-62 having a small outer diameter in the expansion part A-60. This configuration makes it easy to obtain a sufficient space for providing the through windows A-68, 68 and the locking pieces A-78, 78 in the wide parts A-64, 64. The narrow parts A-62, 62 have a larger degree of freedom in shape than the wide parts A-64, 64, which has a limitation in shape in order to provide the through windows A-68, 68 and the locking pieces A-78, 78, so that the thick-walled parts A-70 can be easily provided in the narrow parts A-62, 62.

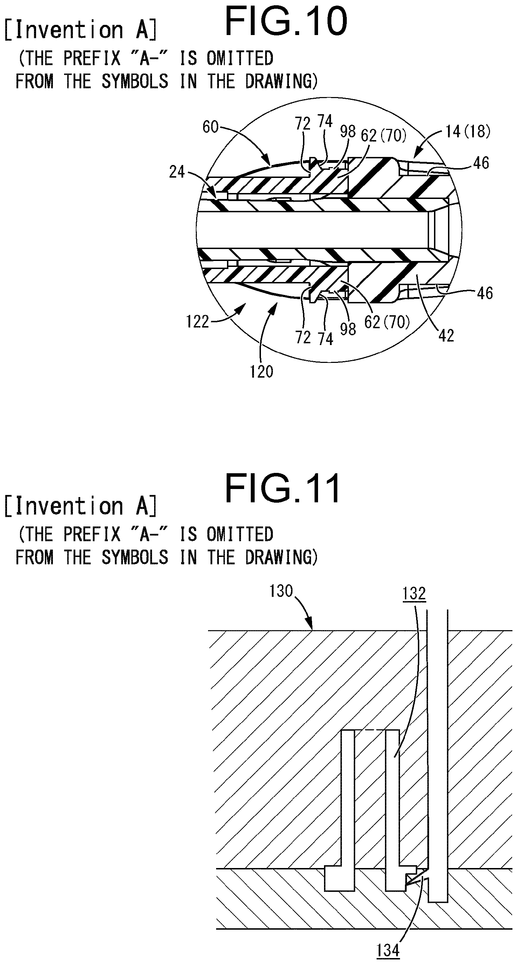

FIG. 10 depicts a needle assembly A-122 including a needle tip protector A-120 according to a second practical embodiment of Invention A. In the following description, components and parts that are substantially identical with those in the first practical embodiment of Invention A will be assigned like symbols and not described in any detail.

The needle tip protector A-120 includes the concavities A-74, 74 formed in the thick-walled parts A-70, 70 on the upper and lower opposite sides, and the injection gate traces A-98 is formed on the bottom surface of each concavity A-74. That is, the needle tip protector A-120 is provided with a plurality of injection gate traces A-98, and the plurality of injection gate traces A-98, 98 are provided in the respective concavities A-74, 74.

Therefore, regarding the needle tip protector A-120, at the time of molding, the cavity (not shown) is filled with the resin material from two locations in the circumferential direction. By so doing, poor filling of the resin material is less likely to occur during molding of the needle tip protector A-120, thereby stably obtaining the needle tip protector A-120 having a predetermined shape.

In the touch part A-112, which is provided on the lower surface of the lower thick-walled part A-70 and may touch the patient's skin, in addition to the recess A-72, the concavity A-74 opens. Regarding the inner surface of the concavity A-74, at least a bottom part thereof does not touch the patient's skin, and the bottom surface of the concavity A-74 is a portion that is away from the touch part A-112. Therefore, the injection gate trace A-98 protruding from the bottom surface of the concavity A-74 that opens onto the touch part A-112 is provided apart from the touch part A-112. With this configuration, even if the injection gate trace A-98 is formed on the touch part A-112 side, the injection gate trace A-98 is prevented from touching the patient, thereby avoiding pain, discomfort feeling or the like caused by the touch by the injection gate trace A-98.

The number of injection gate traces A-98 may be three or more. Further, in the case where a plurality of injection gate traces A-98 are formed, at least one injection gate trace A-98 may be formed on the outer surface of the thick-walled part A-70 at a position away from the concavity A-74.

While Invention A has been described in detail hereinabove in terms of the practical embodiments, Invention A is not limited by the specific disclosures thereof. For example, in the preceding practical embodiment, the recesses A-72, 72 and the concavity A-74 are provided as recesses so as to open onto the outer circumferential surface of the thick-walled parts A-70, 70, but the recess may be provided so as to open onto the axial end face of the thick-walled part A-70. Specifically, a recess may be provided so as to open in the axial direction on the proximal end surface of the thick-walled part A-70 which is provided at the proximal end part of the peripheral wall A-54.

The recess of the preceding practical embodiments is constituted by both the recess A-72 in which the injection gate trace A-98 is not provided and the concavity A-74 in which the injection gate trace A-98 is provided. However, the recess may be constituted by only one of them. Further, the number and arrangement of the recess A-72 and the concavity A-74 may be appropriately changed. Specifically, for example, there may be only one recess A-72, or there may be two or more concavities A-74. The recess may be dispensed with. Besides, the injection gate trace A-98 may be provided in the recess A-72.

The preceding practical embodiment illustrates an example in which the outer circumferential surface of the peripheral wall A-54 of the needle tip protector A-10 is molded by the split molds A-84, 86. However, it is also possible to mold the outer circumferential surface of the peripheral wall of the needle tip protector by using a die as shown in FIG. 11. A mold A-130 for molding the outer circumferential surface of the needle tip protector shown in FIG. 11 is a die having a tubular cavity wall inner surface and being released in the axial direction. Thus, the outer circumferential surface of the peripheral wall of the needle tip protector, which is the molded article, does not have a parting line extending in the axial direction. Therefore, it is possible to set the parting line to a portion that is less likely to touch the patient, the health care worker, or the like in the axial direction, thereby making the surface shape of the portion that easily touches the patient or the health care worker smoother. Accordingly, when the patient, the health care worker, or the like touches the needle tip protector, he/she is less likely to touch the burr-shaped parting line, thereby further reducing pain and sense of discomfort.

Further, an injection gate A-134 for injecting the resin material into a cavity A-132 of the mold A-130 of FIG. 11 is a tunnel gate for which the runner is cut in the shear direction by the mold A-130 when the mold A-130 is removed from the molded article (the needle tip protector). By adopting a tunnel gate, the length of the runner can be shortened, so that a molded article can be efficiently obtained with a small amount of resin material.

The injection gate trace A-98 is preferably formed on the inner surface of the recess, but may alternatively be formed on the outer surface of the thick-walled part A-70 at a position away from the recess without being housed in the recess.

The injection gate trace A-98 may be provided on the outer circumferential surface of the thick-walled part A-70, or may be provided on the axial end face of the thick-walled part A-70. That is, the outer surface of the thick-walled part A-70 does not mean only the outer circumferential surface, but also includes both the outer circumferential surface and the axial end face.

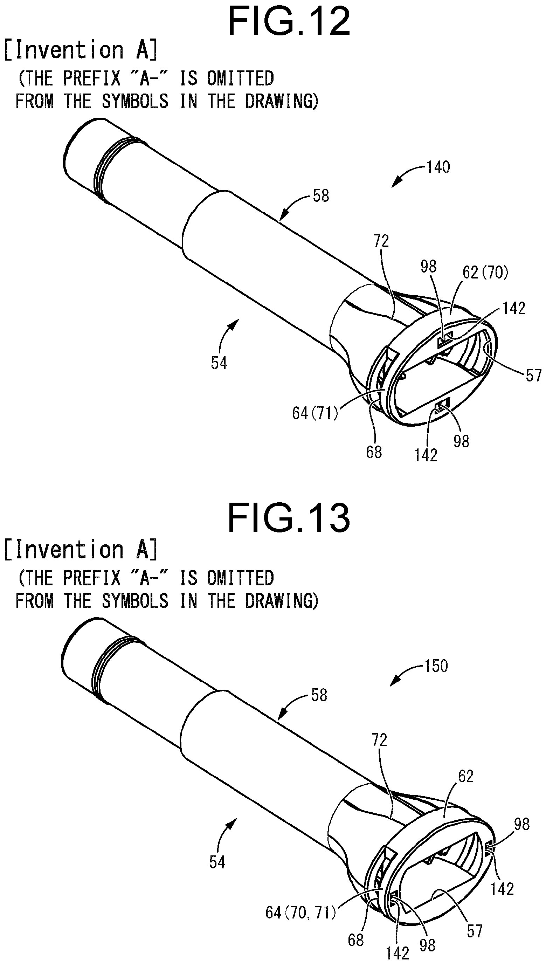

Specifically, for example, as in a needle tip protector A-140 shown in FIG. 12, it would also be acceptable to provide a recess A-142 that opens onto the proximal end surface in the axial direction of the expansion part A-60, and to provide the injection gate trace A-98 on the bottom surface of the recess A-142. In the needle tip protector A-140, the injection gate traces A-98 are provided on the respective axial end faces of the narrow parts A-62, 62 of the expansion part A-60. However, as in a needle tip protector A-150 shown in FIG. 13, the recesses A-142 and injection gate traces A-98 may be provided on the respective proximal end surfaces of the wide parts A-64, 64 of the expansion part A-60. In the needle tip protector A-150 shown in FIG. 13, the central portion of the wide parts A-64, 64 in the circumferential direction comprises the thick-walled part A-70 that is thicker than the opposite end portions.

As shown in FIG. 13, the injection gate A-96 (the injection gate trace A-98) is provided on the wide part A-64, 64 side, so that the weld line is less likely to be generated in the wide parts A-64, 64 serving as engaging parts having the through windows A-68, 68. By increasing the strength of the wide parts A-64, 64, the engaging arm A-50 of the needle hub A-18 can be more firmly fixed to the needle tip protector A-10. Therefore, unintended separation of the needle tip protector A-150 and the needle hub A-18 will be prevented, thereby preventing malfunction of the needle tip protection mechanism. Also, it is possible to prevent troubles such as the needle tip protection mechanism failing to normally function due to, for example, the wide parts A-64, 64 being destroyed by an external force. The injection gate A-96 (the injection gate trace A-98) is preferably provided on the proximal end side of the engaging part. The engaging part is often provided at the proximal end part of the needle tip protector A-150, and the strength of the proximal end part of the needle tip protector A-150 tends to decrease, so that higher strength is required. Therefore, with such a configuration, the weld line is less likely to be generated on the proximal end side of the engaging part. As a result, the strength of the engaging part is increased, and the safety is further improved.

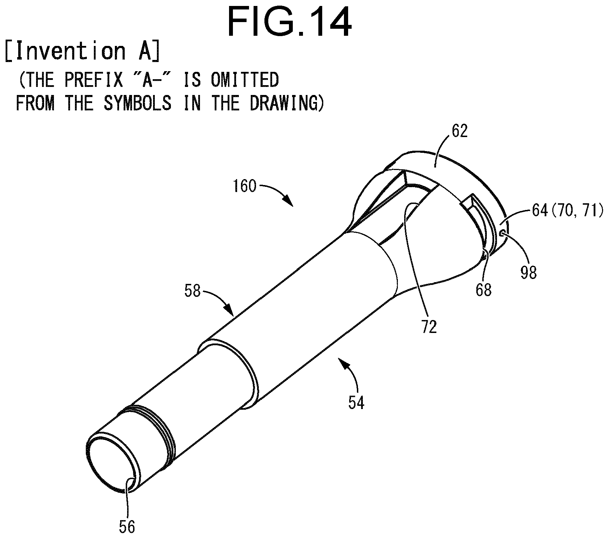

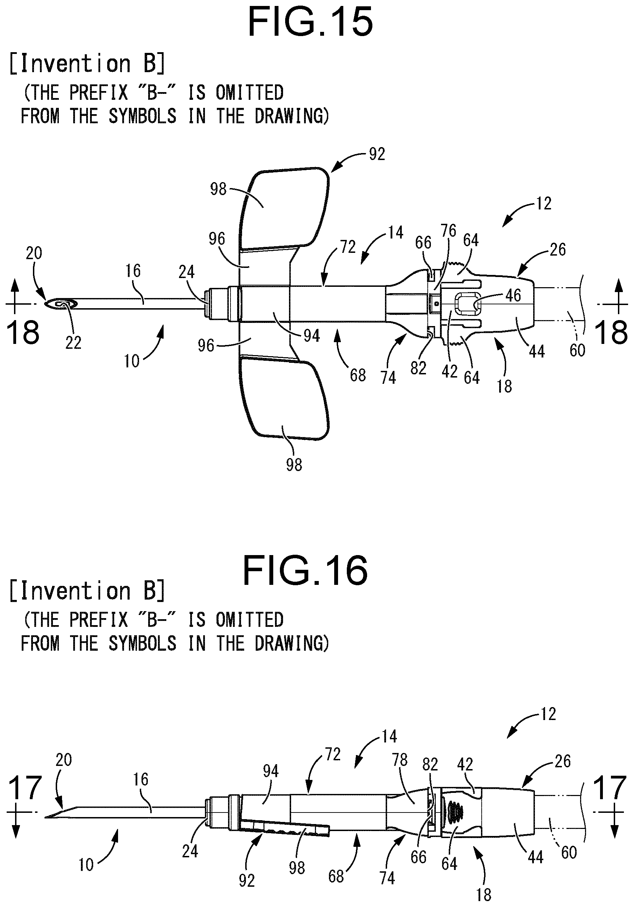

The needle tip protectors A-140, 150 shown in FIGS. 14 and 15 illustrate that the pair of upper and lower injection gate traces A-98, 98 or the pair of left and right injection gate traces A-98, 98 are provided. However, the number, the arrangement in the axial end face, and the like of the injection gate trace A-98 are not limited. For example, in addition to the pair of upper and lower injection gate traces A-98, 98 shown in FIG. 12, the pair of left and right injection gate traces A-98, 98 shown in FIG. 13 may be provided. In that case, the four injection gate traces A-98 are provided in the structure.

Further, since the proximal end surface of the expansion part A-60 is a non-contact portion that is less likely to touch the patient's skin, the injection gate trace A-98 may be provided so as to protrude from the proximal end surface of the needle tip protector without providing the recess A-142. However, in the state before use of the needle assembly in which the needle tip protectors A-140, 150 are coupled to the needle hub (not shown), the proximal end surface of the expansion part A-60 is overlapped with the distal end surface of the needle hub. Therefore, it is desirable that the injection gate trace A-98 is provided in a state of being housed in the recess A-142 without protruding from the proximal end surface of the extension portion A-60, thereby preventing rattling of the needle tip protector A-140, 150 and the needle hub or the like.

In the case where the position of the injection gate is set such that the injection gate trace A-98 is formed on the proximal end surface of the expansion part A-60, it is desirable that the injection gate trace A-98 is provided approximately parallel to the axial direction of the needle tip protectors A-140, 150 having a tubular shape. With this configuration, the resin injected from the injection gate into the cavity of the mold for molding the needle tip protectors A-140, 150 is likely to be uniformly distributed in the cavity, so that molding defects are less likely to occur.

In the preceding practical embodiment, the injection gate trace A-98 is provided on the outer circumferential surface of the narrow parts A-62, 62. However, in the case where the injection gate trace A-98 is provided on the outer circumferential surface of the expansion part A-60, the injection gate trace A-98 may be provided on the outer circumferential surface of the wide part A-64, as in a needle tip protector A-160 shown in FIG. 14. Since the wide part A-64 is located on the lateral side (in the left-right direction) which is less likely touch the patient, in the needle tip protector A-160, the injection gate trace A-98 is provided so as to protrude without being housed in the recess. Of course, even in the case where the injection gate trace A-98 is provided in the wide part A-64, by forming a recess opening onto the wide part A-64 so that the injection gate trace A-98 is provided in the recess, the protrusion of the injection gate trace A-98 can be suppressed. In the case where the injection gate trace A-98 is formed on the wide part A-64 constituting the side surface in the left-right direction as in the needle tip protector A-160, the injection gate traces A-98 may be provided to each of the pair of wide parts A-64, 64, or may be provided only to one of the wide parts A-64.

As shown in FIG. 14, the injection gate A-96 (the injection gate trace A-98) is provided on the wide part A-64, 64 side, so that the weld line is less likely to be generated in the wide parts A-64, 64 serving as engaging parts having the through windows A-68, 68. By increasing the strength of the wide parts A-64, 64, the engaging arm A-50 of the needle hub A-18 can be more firmly fixed to the needle tip protector A-160. Therefore, unintended separation of the needle tip protector A-160 and the needle hub A-18 will be prevented, thereby preventing malfunction of the needle tip protection mechanism. Also, it is possible to prevent a trouble that the needle tip protection mechanism does not normally function due to, for example, the wide parts A-64, 64 being destroyed by an external force. The injection gate A-96 (the injection gate trace A-98) is preferably provided on the proximal end side of the engaging part. The engaging part is often provided at the proximal end part of the needle tip protector A-160, and the strength of the proximal end part of the needle tip protector A-160 tends to decrease, so that higher strength is required. Therefore, with such a configuration, the weld line is less likely to be generated on the proximal end side of the engaging part. As a result, the strength of the engaging part is increased, and the safety is further improved.

It would also be possible to appropriately adopt in combination the injection gate trace A-98 provided on the outer circumferential surface of the thick-walled part A-70 of the narrow part A-62 as shown in the needle tip protector A-10, the injection gate trace A-98 provided on the proximal end surface in the axial direction of the expansion part A-60 as shown in the needle tip protectors A-140, 150, and the injection gate trace A-98 provided on the outer circumferential surface of the wide part A-64 as shown in the needle tip protector A-160.

The number and arrangement of the injection gate traces A-98 are not particularly limited. For example, when a plurality of injection gate traces A-98 are provided, they may be arranged at positions deviated from each other in the axial direction.

The expansion part A-60 is not essential. For example, it would also be acceptable that the outer diameter dimension of the peripheral wall A-54 is approximately constant in the axial direction, and the thick-walled part A-70 is formed by changing the inner diameter dimension thereof. Further, the proximal end portion of the peripheral wall A-54 provided with the thick-walled part A-70 may have a smaller diameter than the distal end portion thereof.

While an example is shown in which the locking piece A-78 is formed in the inside covered by the peripheral wall A-54, the present invention is not limited to such an embodiment. The expansion part A-60 may have a notch so that the locking piece A-78 is visible from the outside. Further, the locking piece A-78 may be provided in the recess A-72 or in the concavity A-74.

### [Invention B]

Meanwhile, FIGS. 15 to 20 show a needle assembly that can be recognized as an independent Invention B capable of solving a different problem from that of Invention A. Invention B relates to a puncture needle body provided with a needle hub on the proximal end side of a puncture needle and a puncture needle assembly using the puncture needle body.

Conventionally, a puncture needle such as an indwelling needle used for infusion, blood collection, hemodialysis, etc. has been known. The indwelling needle has a structure in which a needle hub is fixed to the proximal end side of a puncture needle having a sharp needle tip. The indwelling needle is stuck and indwelled in a somatic lumen such as a blood vessel of the patient, and an external conduit is connected to the needle hub, so that the infusion, blood collection, hemodialysis, etc. are performed through the lumen of the indwelling needle and the external conduit.

Meanwhile, in the indwelling needle, for example, as disclosed in Japanese Unexamined Patent Publication No. JP-A-2017-196060 (Patent Document 1), an external conduit is inserted into a connection hole provided in the needle hub, and the external conduit is connected by being fixed by means such as adhesion or welding to the inner surface of the connection hole.

However, as a result of detailed examination by the inventor, for example, when the external conduit undergoes a large deformation, there is a risk that the external conduit may be excessively bent and damaged at the opening end of the connection hole, or the external conduit may slip out from the connection hole.

It is an object of Invention B to provide a novel puncture needle body which is able to suppress deformation of the external conduit and prevent damage or slipping out of the external conduit.

It is another object of Invention B to provide a needle assembly including the above-mentioned puncture needle body.

Hereinafter, preferred embodiments for grasping Invention B will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in Invention B, various other preferred embodiments can be realized without being limited to those described below.

A first preferred embodiment of Invention B provides a puncture needle body in which a needle hub is provided on a proximal end side of the puncture needle, and a connection hole is formed on the proximal end side of the needle hub and a connection port to which an external conduit is externally mounted is formed projecting from a wall part of the connection hole toward the proximal end side of the needle hub.

According to the puncture needle body of the present preferred embodiment, since the external conduit is inserted into the connection hole and the external conduit is externally mounted about the connection port, the external conduit is less likely to be separated from the connection port.

A second preferred embodiment of Invention B provides the puncture needle body according to the first preferred embodiment of Invention B, wherein the connection port is located further inward than the opening end of the connection hole.

According to the puncture needle body of the present preferred embodiment, the amount of deformation of the external conduit, which is mounted onto the connection port in an externally placed state, is limited by coming into contact with the inner surface of the connection hole outside the protruding tip of the connection port. Therefore, it is possible to prevent the external conduit from being damaged due to excessive bending.

A third preferred embodiment of Invention B provides the puncture needle body according to the first or second preferred embodiment of Invention B, wherein the connection hole has an inner dimension made larger on the proximal end side than on the distal end side.

According to the puncture needle body of the present preferred embodiment, the work of inserting the external conduit into the connection hole is facilitated.

A fourth preferred embodiment of Invention B provides the puncture needle body according to the third preferred embodiment of Invention B, wherein the connection hole has a tapered shape that becomes larger in diameter toward the proximal end side of the needle hub.

According to the puncture needle body of the present preferred embodiment, it is possible to obtain a large area of the inner circumferential surface of the connection hole. Therefore, for example, when the external conduit is adhered to the inner circumferential surface of the connection hole, the adhesion area is made large, so that the adhesive strength can be greatly obtained. The present preferred embodiment includes not only the case where the entire connection hole has a tapered shape that becomes larger in diameter toward the opening end, but also the case where the connection hole has a tapered shape that becomes larger in diameter toward the opening end partially in the axial direction.

A fifth preferred embodiment of Invention B provides the puncture needle body according to any one of the first to fourth preferred embodiments of Invention B, wherein the cross section of an opening edge of the connection hole has a curved shape.

According to the puncture needle body of the present preferred embodiment, when the external conduit is inserted into the connection hole, the opening edge of the connection hole having a curved cross-sectional shape functions as a guide for guiding the external conduit to the connection hole. Therefore, it is easy to insert the external conduit into the connection hole. Further, for example, if the cross section of the opening edge of the connection hole has a curved shape that is convex toward the radial inside of the connection hole, by making the opening edge of the connection hole into a smooth shape, when the external conduit connected to the connection port deforms and comes into contact with the opening edge of the connection hole, damage to the external conduit due to touch on the opening edge of the connection hole will be prevented.

A sixth preferred embodiment of Invention B provides the puncture needle body according to any one of the first to fifth preferred embodiments of Invention B, wherein the connection port has a tapered outer circumferential surface.

According to the puncture needle body of the present preferred embodiment, the work of externally placing the external conduit onto the connection port is facilitated. In the case where the outer circumferential surface of the connection port is fastened to the external conduit, a large fastening area between the connection port and the external conduit can be secured, thereby improving the fastening strength. The present preferred embodiment includes not only the case where the entire outer circumferential surface of the connection port comprises a tapered outer circumferential surface but also the case where the outer circumferential surface of the connection port is provided with a tapered outer circumferential surface partially in the axial direction.

A seventh preferred embodiment of Invention B provides the puncture needle body according to any one of the first to sixth preferred embodiments of Invention B, wherein the cross section of the outer peripheral edge of the tip of the connection port has a round shape with no corner.

According to the puncture needle body of the present preferred embodiment, the cross section of the outer peripheral edge of the tip of the connection port has a round shape with no corners. Thus, even if the external conduit is bent, the connection port can be prevented from breaking through the external conduit.

The eighth preferred embodiment of Invention B provides a needle assembly including the puncture needle body according to any one of the first to seventh preferred embodiments of Invention B, and a needle tip protector configured to move in the needle axis direction with respect to the puncture needle body to cover a needle tip of the puncture needle, wherein a pair of arm parts that are engaged with the needle tip protector are provided on the proximal end side of the needle hub, and a minimum thickness dimension of the arm parts is set larger than that of a peripheral wall of the connection hole in a longitudinal cross section in an axial direction that is parallel to a direction of opposition of the arm parts.

According to the needle assembly of the present preferred embodiment, it is possible to secure the strength of the arm part that undergoes deformation or the like due to action of an external force when the needle hub and the needle tip protector are separated.

A ninth preferred embodiment of Invention B provides the needle assembly according to the eighth preferred embodiment of Invention B, wherein a reinforcing part that is thicker than the peripheral wall of the connection hole is provided on the distal end side with respect to the connection hole.

For example, in the case where the external conduit is adhered to the needle hub, there is a risk that the needle hub may be damaged due to adhesion of the plasticizer of the adhesive causing cracks on the distal end side with respect to the connection hole. According to the needle assembly of the present preferred embodiment, by providing the thick reinforcing part in a portion where damage may occur, it is possible to prevent poor adhesion of the external conduit due to damage to the needle hub.

According to Invention B, in the puncture needle body, it is possible to suppress the deformation of the external conduit and prevent the external conduit from being damaged or slipping out.

Hereinafter, practical embodiments of Invention B will be described with reference to the drawings.

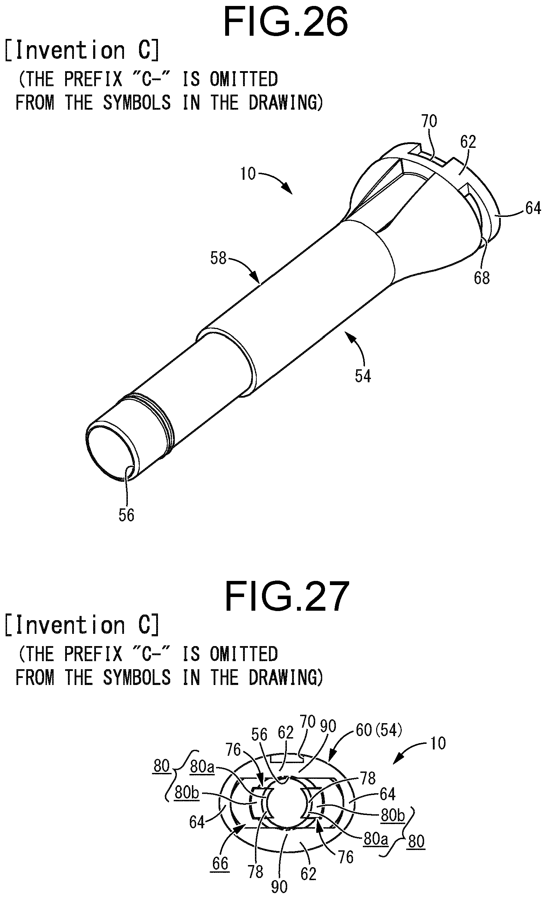

FIGS. 15 to 18 show a needle assembly B-12 including a puncture needle body B-10 according to a first practical embodiment of Invention B. The needle assembly B-12 has a structure in which the puncture needle body B-10 is movably inserted through the needle tip protector B-14 in the needle axis direction. In the following description, unless otherwise specified, the distal end side refers to the left side in FIG. 15 which is a needle tip B-20 side of the puncture needle body B-10, which will be described later, and the proximal end side refers to the right side in FIG. 15, which is the rear side of the puncture direction of the puncture needle body B-10. Further, as a general rule, the vertical direction refers to the vertical direction in FIG. 16, and the left-right direction refers to the vertical direction in FIG. 26, which is the horizontal direction. In FIGS. 15 to 20 according to Invention B, the description of "B-" is omitted in each reference numeral "B-XX" (where XX is a number corresponding to the part) for the sake of legibility. Besides, in order to make it easier to understand that FIGS. 15 to 20 are drawings according to Invention B, FIGS. 15 to 20 are labelled with [Invention B].

More specifically, the puncture needle body B-10 includes a puncture needle B-16 and a needle hub B-18 provided on the proximal end side of the puncture needle B-16. The puncture needle B-16 is made of a metal such as stainless steel, and has a sharp, tapered needle tip B-20 at one end. A through hole B-22 is formed at the distal end portion of the puncture needle B-16, so that blood can easily flow into the lumen of the puncture needle B-16.

The needle hub B-18 has an approximately cylindrical shape overall, and the proximal end portion of the puncture needle B-16 is fixed to the distal end portion of the needle hub B-18 in an inserted state. With this configuration, the lumen of the needle hub B-18 and the lumen of the puncture needle B-16 communicate with each other in the needle axis direction. The needle hub B-18 includes a needle support part B-24 forming its distal end portion and a protector coupling part B-26 forming its proximal end portion.

The needle support part B-24 has an approximately cylindrical shape, and is formed of, for example, a rigid synthetic resin. The inner diameter dimension at the distal end portion of the needle support part B-24 is approximately the same as the outer diameter dimension of the puncture needle B-16. The proximal end portion of the puncture needle B-16 is inserted from the opening on the distal end side of the needle support part B-24 and fixed thereto. The puncture needle B-16 and the needle support part B-24 may be adhered or the like to each other, if necessary.

The outer circumferential surface of the middle portion in the needle axis direction of the needle support part B-24 comprises a tapered surface B-30 that gradually increases in diameter toward the distal end side. With this configuration, in the needle support part B-24, the middle portion in the needle axis direction having the tapered surface B-30 comprises a small-diameter tube part B-32 having a diameter smaller than that of the distal end portion and the proximal end portion. The outer circumferential surface of the needle support part B-24 is not limited to the tapered surface B-30 whose outer dimension changes in the axial direction, but may have a generally constant outer dimension in the axial direction except for the concave part B-34 and the convex part B-36, which will be described later.

A concave part B-34 that opens onto the outer circumferential surface is formed at the further distal end portion of the needle support part B-24 than the tapered surface B-30. The concave part B-34 has an annular shape that is continuous with an approximately constant cross-sectional shape over the entire circumference. The minimum outer diameter dimension of the needle support part B-24 at the location where the concave part B-34 is formed is approximately the same as the outer diameter dimension of the proximal end of the small-diameter tube part B-32, which is the minimum outer diameter dimension thereof.

An annular convex part B-36 projects from the outer circumferential surface of the needle support part B-24 on the distal end side with respect to the concave part B-34. The convex part B-36 is provided at the distal end or a position close to the distal end of the needle support part B-24, and constitutes the distal end portion of the needle support part B-24 in the present practical embodiment. The outer diameter dimension of the needle support part B-24 in the portion forming the convex part B-36 is larger than the outer diameter dimension of the distal end of the small-diameter tube part B-32, which is the maximum outer diameter dimension thereof.

The proximal end side surface forming the inner surface of the concave part B-34 comprises an annular proximal-end-side restricting surface B-38 extending in the axis-perpendicular direction, and the bottom surface of the concave part B-34 and the tapered surface B-30 are continuous in a stepped manner via the proximal-end-side restricting surface B-38. The distal end side surface forming the inner surface of the concave part B-34 comprises an annular distal-end-side restricting surface B-40 extending in the axis-perpendicular direction, and the bottom surface of the concave part B-34 and the outer circumferential surface of the convex part B-36 are continuous in a stepped manner via the distal-end-side restricting surface B-40. The dimension in the axis-perpendicular direction of the proximal-end-side restricting surface B-38 formed by the proximal end side inner surface of the concave part B-34 is slightly larger than the dimension in the axis-perpendicular direction of the distal-end-side restricting surface B-40.

The protector coupling part B-26 is made of a rigid synthetic resin, like the needle support part B-24, for example. The protector coupling part B-26 has a stepped, approximately cylindrical shape overall, and has a coupling tube B-42 having a small diameter on the distal end side and a connecting tube B-44 having a large diameter on the proximal end side. The proximal end portion of the needle support part B-24 is inserted into the coupling tube B-42, and adhesion or welding is performed as necessary, so that the protector coupling part B-26 is fixed to the needle support part B-24. An approximately rectangular recess B-46 is formed on the surface of the protector coupling part B-26 in the vertical direction (the vertical direction in FIG. 16). As shown in FIG. 15, the recess B-46 of the present practical embodiment is provided on the surface of the coupling tube B-42, but may be provided on the surface of the connecting tube B-44, or may be provided so as to straddle the coupling tube B-42 and the connecting tube B-44. The needle support part B-24 and the protector coupling part B-26 may be integrally molded.

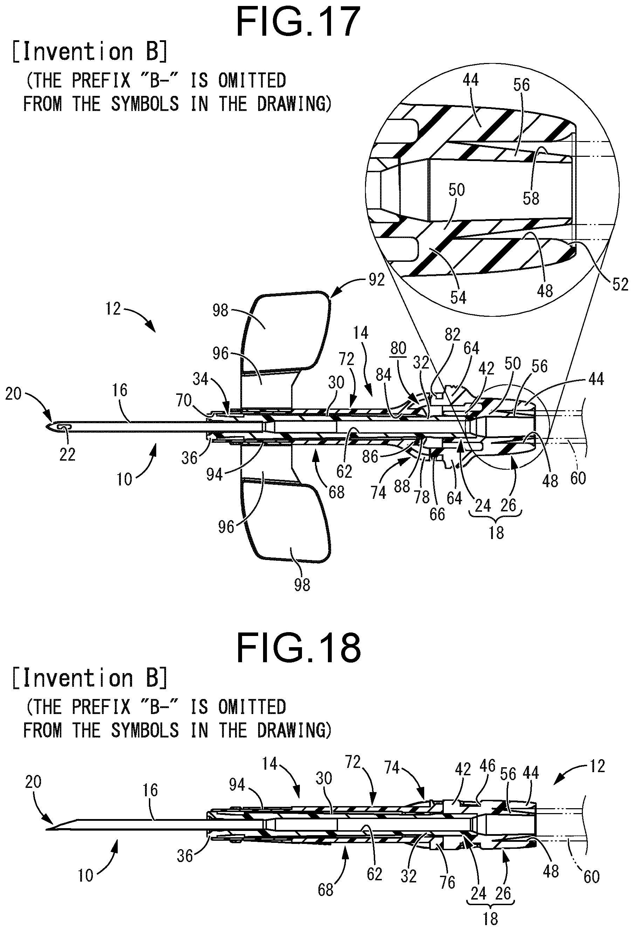

As shown in FIGS. 17 and 18, the connecting tube B-44 of the needle hub B-18 has a connection hole B-48. The connection hole B-48 has an approximately circular cross section. The connection hole B-48 has a bottom part B-50 on the distal end side opposite to the opening. The inner hole of the connecting tube B-44 penetrates the bottom part B-50 in the axial direction, and the bottom part B-50 is provided in an annular shape. The connection hole B-48 has a larger diameter at the proximal end side, which is the opening side, than at the distal end side, which is the bottom part B-50 side. The diameter of the connection hole B-48 gradually increases toward the opening end on the proximal end side, and the peripheral wall inner surface of the connection hole B-48 has a tapered tubular shape. The opening edge part of the connection hole B-48 includes an opening expansion part B-52 having a curved cross-sectional shape in which the inclination angle of the peripheral wall inner surface of the is partially increased.

On the distal end side with respect to the connection hole B-48, there is provided a reinforcing part B-54 made thicker than the peripheral wall of the connection hole B-48 in the axis-perpendicular direction. The reinforcing part B-54 of the present practical embodiment includes the bottom part B-50 of the connection hole B-48. The reinforcing part B-54 is thicker than the opening expansion part B-52. The inner circumferential surface and the outer circumferential surface of the reinforcing part B-54 are provided so as to be continuous with the outer circumferential surface of the peripheral wall of the connection hole B-48 and the inner circumferential surface of a connection port B-56 described later. However, the reinforcing part B-54 may be made thicker by being provided with a protrusion that projects to at least one of the radial outside and the radial inside.

The connection port B-56 is provided so as to project from the bottom part B-50 of the connection hole B-48. The connection port B-56 has a tubular shape. The connection port B-56 is provided on the radially inner side with respect to the peripheral wall of the connection hole B-48. The connection port B-56 protrudes from the bottom part B-50 constituting the wall part of the connection hole B-48 to the proximal end side toward the opening of the connection hole B-48, whereby the connecting portion of the external conduit B-60 can be relatively small in diameter in the needle hub B-18. The inner circumferential surface of the connection port B-56 comprises a cylindrical surface having an approximately constant diameter in the axial direction. The outer circumferential surface of the connection port B-56 has a diameter dimension that gradually decreases toward the proximal end side in the axial direction, and comprises a tapered outer circumferential surface B-58. As shown in the enlarged view of FIG. 17, the cross section of the outer peripheral edge of the tip of the connection port B-56 has a round shape with no corner. The connection port B-56 may be separate from the connecting tube B-44, but in the present practical embodiment, the connection port B-56 is integrally formed with the connecting tube B-44.

In the protector coupling part B-26, the distal end of the external conduit B-60 is inserted into the connection hole B-48 of the connecting tube B-44, and the distal end of the external conduit B-60 is attached to the connecting port B-56 in an externally fitted state. The external conduit B-60 is adhered or welded to the inner circumferential surface of the connection hole B-48 and the connection port B-56 as necessary. By the external conduit B-60 being connected to the connecting tube B-44, the needle support part B-24 and the external conduit B-60 are connected to the opposite sides of the protector coupling part B-26, and the lumen of the puncture needle B-16 and the lumen of the needle hub B-18 communicate with the external conduit B-60. With this configuration, a fluid flow path B-62 from the distal end opening of the puncture needle B-16 to the external conduit B-60 is provided to the puncture needle body B-10.

The distal end of the external conduit B-60 is not only inserted into the connection hole B-48, but also externally fitted onto the connection port B-56. Accordingly, the external conduit B-60 is more firmly connected to the needle hub B-18, thereby preventing the external conduit B-60 from slipping out from the needle hub B-18.

Since the inner circumferential surface of the connection hole B-48 has a tapered shape, a large fastening area is secured when the outer circumferential surface of the external conduit B-60 is fastened to the inner circumferential surface of the connection hole B-48. Similarly, since the outer circumferential surface of the connection port B-56 has a tapered shape, a large fastening area is secured when the inner circumferential surface of the external conduit B-60 is fastened to the outer circumferential surface of the connection port B-56. With these configurations, it is possible to obtain a large fastening strength of the external conduit B-60 to the needle hub B-18.

The connecting tube B-44 is provided with a pair of arm parts B-64, 64 extending toward the distal end side. The arm parts B-64, 64 are arranged in opposition to each other in the diametrical direction, and extend further to the distal end side than the coupling tube B-42 in the outer periphery of the coupling tube B-42. The arm parts B-64, 64 are arranged apart from the coupling tube B-42 to the radially outer side, and the connecting portion with the connecting tube B-44 is thin and narrow, so as to be elastically deformable in the direction of approaching each other by the action of an external force.

A hook B-66 is formed at the distal end portion of each of the arm parts B-64, 64. The hook B-66 projects outward in the direction of opposition of the arm parts B-64, 64 in each arm part B-64. The pair of hooks B-66, 66 are configured to move in the direction of approaching each other by the arm parts B-64, 64 elastically deforming in the direction of approach.

As shown in FIGS. 15 to 18, the needle tip protector B-14 has an approximately tubular shape extending in the needle axis direction overall, and is integrally molded by using a rigid synthetic resin such as polypropylene, polycarbonate, polyethylene terephthalate glycol, ABS resin, and MBS resin. The needle tip protector B-14 is preferably transparent or translucent to allow transmission of visible light. The needle tip protector B-14 is provided with a tubular peripheral wall B-68, and an inner hole B-70 penetrating in the needle axis direction is formed in the radial inside of the peripheral wall B-68.

The peripheral wall B-68 has a cylindrical part B-72 serving as a distal end tubular part having an approximately perfect circular ring-shaped cross section on the distal end side, and has an expansion part B-74 serving as a large-diameter tubular part having a diameter larger than that of the cylindrical part B-72 on the proximal end side. The shape of the expansion part B-74 is varied in the needle axis direction.

The expansion part B-74 has an approximately elliptical tubular shape, and the width dimension of the outer circumferential surface in the horizontal direction, which is the large-diameter direction, is larger than the width dimension of the outer circumferential surface in the vertical direction, which is the small-diameter direction. Of the wall parts that make up the expansion part B-74, the parts that make up the wall part in the vertical direction comprise narrow parts B-76, 76, while the parts that make up the wall part in the horizontal direction comprise wide parts B-78, 78 having a larger dimension than that of the narrow parts B-76, 76. The direction of opposition of the narrow parts B-76, 76 and the direction of opposition of the wide parts B-78, 78 are approximately orthogonal to each other.

The outer circumferential surfaces of the wide parts B-78, 78 gradually increases in outer diameter dimension from the distal end side to the proximal end side. The outer circumferential surface of the cylindrical part B-72 and the outer circumferential surface of the wide parts B-78, 78 are smoothly connected. The wall thickness dimension from the cylindrical part B-72 to the wide parts B-78, 78 is approximately constant in the needle axis direction. The wide parts B-78, 78 have an approximately elliptical arc cross section in which the outer circumferential surface and the inner circumferential surface correspond to each other, and the thickness dimension is approximately constant in the circumferential direction. In the radial inside of the expansion part B-74, an internal space B-80 having a flat cross section with its horizontal dimension larger than its vertical dimension is formed at the proximal end portion of the inner hole B-70 penetrating the needle tip protector B-14. The inside dimension in the horizontal direction of the internal space B-80 gradually increases toward the proximal end side.

The wide parts B-78, 78 are provided with through windows B-82, 82 serving as engaging parts that penetrate in the plate thickness direction. The circumferential dimension of the through windows B-82, 82 is larger than the circumferential dimension of the hooks B-66, 66. In the present practical embodiment, when the needle tip protector B-14 is molded, the mold for molding the outer circumferential surface is vertically split. Thus, the through windows B-82, 82 have a shape that will not cause any undercut at the time of mold release in the vertical direction.

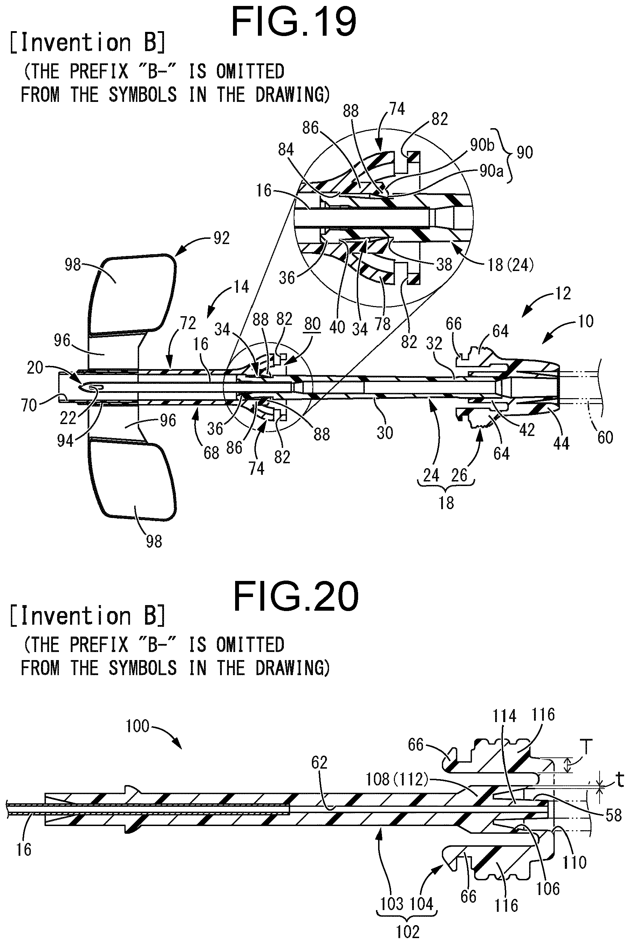

An annular stepped surface B-84 extending in the axis-perpendicular direction is formed on the inner circumferential surface of the peripheral wall B-68 at the front end portion of the expansion part B-74 (see FIG. 19). The inner circumferential surface of the peripheral wall B-68 has a diameter larger on the distal end side of the stepped surface B-84 than on the proximal end side thereof. The dimension in the axis-perpendicular direction of the stepped surface B-84 is smaller than the dimension in the axis-perpendicular direction of the distal-end-side restricting surface B-40 of the concave part B-34.

Further, the peripheral wall B-68 is provided with a pair of projecting pieces B-86, 86 protruding from the inner circumferential surface of the expansion part B-74 to the internal space B-80. As shown in FIG. 17, the projecting pieces B-86, 86 are integrally formed with the wide parts B-78, 78 of the expansion part B-74 of the peripheral wall B-68. The projecting pieces B-86, 86 are arranged at positions corresponding to the wide parts B-78, 78 on the radially inner side of the wide parts B-78, 78. The projecting pieces B-86, 86 are provided in opposition to each other in the horizontal direction and are remote from each other in the circumferential direction. The projecting pieces B-86, 86 project toward the proximal end side of the peripheral wall B-68 on the proximal end side of the stepped surface B-84. The projecting pieces B-86, 86 extend from the stepped surface B-84 toward the proximal end side approximately parallel to the needle axis direction, and at least the protruding tip portion is arranged away from the wide parts B-78, 78 of the peripheral wall B-68 to the radially inner side. The projecting pieces B-86, 86 are each curved in the circumferential direction. The projecting piece B-86 may be provided so as not to touch the outer circumferential surface of the needle support part B-24 except for the concave part B-34 and the convex part B-36. The expansion part B-74 may have a notch, and the projecting pieces B-86, 86 may be provided at a distance from the notch.

A pair of claw parts B-88, 88 that bend and project radially inward are formed at the respective protruding tips (the proximal ends in the needle axis direction) of the projecting pieces B-86, 86. The inner circumferential surfaces of the claw parts B-88, 88 are each curved in the circumferential direction, and the radius of curvature of the inner circumferential surface at the protruding tip of the claw parts B-88, 88 is approximately equal to the radius of curvature of the outer circumferential surface of the small-diameter tube part B-32 of the needle support part B-24. Further, the distance between the opposed faces in the diametrical direction at the protruding tips of the claw parts B-88, 88 is approximately equal to the outer diameter dimension of the small-diameter tube part B-32, while being smaller than the maximum outer diameter dimension of the distal end portion of the tapered surface B-30. However, the distance between the opposed faces in the diametrical direction at the protruding tips of the claw parts B-88, 88 may be slightly smaller or slightly larger than the outer diameter dimension of the small-diameter tube part B-32. In the case where the outer circumferential surface of the needle support part B-24 except for the convex part B-36 and the concave part B-34 has a constant diameter, the distance between the opposed faces in the diametrical direction at the protruding tips of the claw parts B-88, 88 may be slightly smaller, or slightly larger, or approximately equal with respect to the outer diameter dimension of the said outer circumferential surface.

Regarding proximal end side end faces B-90, 90 of the respective claw parts B-88, 88, the radially inner portions constitute vertical surfaces B-90a, 90a extending in the axis-perpendicular direction, while the radially outer portions constitute inclined surfaces B-90b, 90b inclining toward the distal end side in the needle axis direction toward the radially outer side (see FIG. 19). In the present practical embodiment, the dimension in the axis-perpendicular direction of the vertical surfaces B-90a, 90a is approximately the same as or slightly larger than the dimension in the axis-perpendicular direction of the proximal-end-side restricting surface B-38 of the concave part B-34. With this configuration, when the claw parts B-88, 88 come into contact with the proximal-end-side restricting surface B-38 as described later, the entire surface of the proximal-end-side restricting surface B-38 comes into contact with the vertical surfaces B-90a, 90a, thereby obtaining a sufficiently large contact area. Furthermore, since the inclined surfaces B-90b, 90b located on the radially outer side of the vertical surfaces B-90a, 90a incline toward the distal end side in the needle axis direction toward the radially outer side, the vertical surfaces B-90a, 90a and the proximal-end-side restricting surface B-38 come into contact with each other without any interference by the inclined surfaces B-90b, 90b.

Besides, the protruding tips (the proximal ends in the needle axis direction) of the claw parts B-88, 88 are located on the distal end side in the needle axis direction with respect to the proximal end of the expansion part B-74. Therefore, the entire projecting pieces B-86, 86 are housed in the internal space B-80 of the expansion part B-74.

As shown in FIG. 15, a wing-shaped part B-92 is provided at the distal end portion of the needle tip protector B-14. The wing-shaped part B-92 is formed of, for example, a soft synthetic resin. The wing-shaped part B-92 is integrally formed with a tubular fitting tube part B-94 with plate-shaped coupling parts B-96, 96 protruding in the tangential direction of the fitting tube part B-94. Besides, a wing main body B-98 is integrally formed on the protruding tip side of each of the coupling parts B-96, 96 from the fitting tube part B-94. The wing-shaped part B-92 is attached to the peripheral wall B-68 of the needle tip protector B-14 by the fitting tube part B-94 being fitted externally onto the distal end portion of the cylindrical part B-72.

The puncture needle body B-10 is inserted through the inner hole B-70 of the needle tip protector B-14 from the opening on the proximal end side, so that the needle tip protector B-14 is externally mounted onto the puncture needle body B-10, thereby constituting the needle assembly B-12. In the initial state before use of the needle assembly B-12 shown in FIG. 15 and the like, the needle tip B-20 of the puncture needle body B-10 is located on the distal end side with respect to the needle tip protector B-14, and the needle tip B-20 is exposed. In this initial state, as shown in FIG. 17, the hooks B-66, 66 provided to the needle hub B-18 of the puncture needle body B-10 are inserted in and engaged with the through windows B-82, 82 provided to the expansion part B-74 of the needle tip protector B-14. With this configuration, the needle tip protector B-14 and the needle hub B-18 are coupled to each other, and the protruding state of the needle tip B-20 is maintained. Besides, for example, when puncture is performed by holding the wing-shaped part B-92, the movement of the puncture needle B-16 toward the proximal end side in the needle axis direction due to resistance during the puncture or the like is restricted.

In the initial state of the needle assembly B-12, the claw parts B-88, 88 of the needle tip protector B-14 are in contact with the outer circumferential surface of the small-diameter tube part B-32 of the needle support part B-24. These claw parts B-88, 88 may be slightly pushed radially outward by being in contact with the outer circumferential surface of the small-diameter tube part B-32, or may be slightly remote from the outer circumferential surface of the small-diameter tube part B-32.

The needle assembly B-12 is used for infusion, blood collection, hemodialysis and the like through the fluid flow path B-62 by the puncture needle B-16 being stuck into the patient's blood vessel and the puncture needle body B-10 serving as the indwelling needle being indwelled therein. Since the needle assembly B-12 of the present practical embodiment is provided with the wing-shaped parts B-92, it is possible to stick the puncture needle B-16 of the puncture needle body B-14 while, for example, pinching the wing-shaped parts B-92. When indwelling the puncture needle body B-10 in the punctured state, by fixing with a tape fastener at the positions of the wing-shaped parts B-92, it is possible to fix to the skin with a wide touch area.

When the puncture needle body B-10 is removed, the arm parts B-64, 64 of the needle hub B-18 are pressed inward by fingers while the needle tip protector B-14 is kept fixed with a tape fastener at the wing-shaped parts B-92. By so doing, the hooks B-66, 66 and the through windows B-82, 82 are detached from each other, making it possible to move the puncture needle body B-10 toward the proximal end side with respect to the needle tip protector B-14. By moving the puncture needle body B-10 toward the proximal end side with respect to the needle tip protector B-14 and removing the puncture needle B-16 from the skin, the needle tip protector B-14 moves to the needle tip B-20 side with respect to the puncture needle body B-10.

With the hooks B-66, 66 and the through windows B-82, 82 detached from each other, unless an external force is applied to the puncture needle body B-10 to move toward the proximal end side with respect to the needle tip protector B-14, the puncture needle body B-10 is configured not to move toward the proximal end side with respect to the needle tip protector B-14. That is, since the claw parts B-88, 88 are in contact with the tapered surface B-30 that increases in diameter toward the distal end side, the puncture needle body B-10 is prevented from moving toward the proximal end side with respect to the needle tip protector B-14. With this configuration, it is possible to prevent a trouble that the needle tip B-20 of the puncture needle body B-10 is accidentally protected by the needle tip protector B-14.

By moving the puncture needle body B-10 toward the proximal end side with respect to the needle tip protector B-14, the claw parts B-88, 88 come into sliding contact with the tapered surface B-30, and elastic recovery force of the projecting pieces B-86, 86 acts as an urging force for pressing the claw parts B-88, 88 against the tapered surface B-30. Therefore, the user can move the puncture needle body B-10 while confirming the feeling that the puncture needle body B-10 is being pulled out from the needle tip protector B-14 by the resistance force due to the contact between the claw parts B-88, 88 and the tapered surface B-30.

As shown in FIG. 19, by the puncture needle body B-10 being moved backward to a predetermined position with respect to the needle tip protector B-14 (the needle tip protector is moved forward to the needle tip B-20 side of the puncture needle body B-10), the needle tip B-20 of the puncture needle B-16 is covered with the needle tip protector B-14. Moreover, the claw parts B-88, 88 provided on the free end side of the projecting pieces B-86, 86 move over the opening of the concave part B-34, so that the claw parts B-88, 88, which elastically recover their shapes, enter the concave part B-34. By so doing, the vertical surfaces B-90a, 90a of the claw parts B-88, 88 and the proximal-end-side restricting surface B-38 of the concave part B-34 are brought into contact and locked to each other. Then, the movement of the puncture needle body B-10 toward the distal end side with respect to the needle tip protector B-14 is restricted, thereby preventing re-exposure of the needle tip B-20 of the puncture needle body B-10.

The stepped surface B-84 provided at the front end portion of the projecting pieces B-86, 86 and the distal-end-side restricting surface B-40 of the concave part B-34 are brought into contact and locked to each other, so that movement of the puncture needle body B-10 to the proximal end side is restricted. By so doing, the puncture needle body B-10 is prevented from becoming dislodged to the proximal end side with respect to the needle tip protector B-14. Therefore, the puncture needle body B-10 is prevented from moving to the opposite sides in the axial direction with respect to the needle tip protector B-14, thereby maintaining the protected state of the needle tip B-20 by the needle tip protector B-14.

In this practical embodiment, the pair of projecting pieces B-86, 86 are provided, and in combination with the fact that the projecting pieces B-86, 86 are integrally molded with the peripheral wall B-68 of the needle tip protector B-14, when the claw parts B-88, 88 and the proximal-end-side restricting surface B-38 are locked, troubles such as the needle tip protector B-14 rattling with respect to the puncture needle body B-10 can be effectively prevented. By providing two, or three or more projecting pieces B-86 in this way, the needle tip protector B-14 is more reliably prevented from moving backward to the proximal end side with respect to the puncture needle body B-10, thereby more stably exhibiting the effect of preventing re-exposure of the needle tip B-20. In this case, it is preferable that the plurality of projecting pieces B-86 are arranged approximately symmetrically with respect to the central axis of the needle tip protector B-14.

With the needle tip B-20 of the puncture needle body B-10 protected by the needle tip protector B-14, the tape fixing of the wing-shaped parts B-92 is released, and the needle assembly B-12 is removed from the patient. However, the procedure for protecting the needle tip B-20 is not limited to the aforementioned one. Specifically, for example, it would also be acceptable to release the tape fixing at the wing-shaped part B-92, and move the entire needle assembly B-12 backward to remove the puncture needle B-16 from the blood vessel, and then move the puncture needle body B-10 backward with respect to the needle tip protector B-14, thereby protecting the needle tip B-20 of the puncture needle B-16.

When the claw parts B-88, 88 enter the concave part B-34, the tips of the claw parts B-88, 88 come into contact with the bottom surface of the concave part B-34, so that the user can confirm the impact and sound of the contact. Therefore, for example, the risk that the user may stop the pulling-out operation of the puncture needle body B-10 midway or the like is avoided, thereby more reliably protecting the needle tip B-20 with the needle tip protector B-14.

In the needle tip protector B-14 and the needle assembly B-12 having the above-mentioned structure, the projecting pieces B-86, 86 that prevent the re-exposure of the needle tip B-20 of the puncture needle body B-10 are provided so as to be entirely housed inside the tubular peripheral wall B-68. This makes it almost impossible to unintentionally touch the projecting pieces B-86, 86 from the outside. Therefore, in the protected state of the needle tip B-20 of the puncture needle body B-10 by the needle tip protector B-14, it is possible to effectively prevent the projecting pieces B-86, 86 and the concave part B-34 from being accidentally disengaged from each other to re-expose the needle tip B-20 from the needle tip protector B-14.

With the projecting pieces B-86, 86 locked to the needle hub B-18, for example, when an external force such as a force in the bending direction is applied to the needle tip protector B-14 and/or the needle hub B-18, the projecting pieces B-86, 86 may come into contact with the wide parts B-78, 78 located apart to the radially outer side. By so doing, the deformation of the projecting pieces B-86, 86 to the radially outer side is restricted, and the risk of the projecting pieces B-86, 86 being disengaged from the needle hub B-18 is reduced, thereby stably maintaining the protected state of the needle tip B-20 of the puncture needle body B-10.

The projecting pieces B-86, 86 are provided in the expansion part B-74 expanded on the proximal end side of the needle tip protector B-14. With this configuration, while obtaining a sufficient size of the projecting pieces B-86, 86, the outer diameter dimension of the needle support part B-24 of the needle hub B-18 inserted between the projecting pieces B-86, 86 can also be made large enough. Furthermore, in the present practical embodiment, the expansion part B-74 has an approximately elliptical shape, and the outer circumferential surface thereof is smoothly continuous from the outer circumferential surface of the cylindrical part B-72. This reduces the risk that the expansion part B-74 or the like may come into contact with the patient so that the patient feels pain.

The projecting pieces B-86, 86 are provided inside the wide parts B-78, 78 constituting the expansion part B-74. This prevents the wide parts B-78, 78 from being thick, thereby suppressing mixing of air bubbles in a component during molding, occurrence of dimensional errors and deterioration in quality due to generation of sink marks and voids, and the like. Further, it is preferable that the locking part (the projecting pieces B-86, 86) is provided so as to extend in the same direction as the wide parts B-78, 78 extend (to the proximal end side in the axial direction). With this configuration, the expansion part B-74 is unlikely to become larger than necessary, so that the space inside the wide parts B-78, 78 can be used skillfully, and the wide parts B-78, 78 are prevented from being thick.

Since the projecting pieces B-86, 86 extend toward the proximal end side, they do not get caught when the puncture needle body B-10 is pulled out with respect to the needle tip protector B-14, thereby realizing smooth pulling out.

The vertical surfaces B-90a, 90a of the claw parts B-88, 88 and the proximal-end-side restricting surface B-38 of the concave part B-34, which are mutually locked, all extend in the axis-perpendicular direction. Moreover, the stepped surface B-84 and the distal-end-side restricting surface B-40 of the concave part B-34, which are mutually locked, both extend in the axis-perpendicular direction. Therefore, the needle tip protector B-14 having the vertical surfaces B-90a, 90a and the stepped surface B-84, as well as the needle hub B-18 having the proximal-end-side restricting surface B-38 and the distal-end-side restricting surface B-40 do not have undercut shape and are easy to mold. Moreover, the contact force and the contact reaction force due to contact between the vertical surfaces B-90a, 90a and the proximal-end-side restricting surface B-38 act without being inclined with respect to the axial direction. Similarly, the contact force and the contact reaction force due to contact between the stepped surface B-84 and the distal-end-side restricting surface B-40 act without being inclined with respect to the axial direction. Besides, each contact area can be sufficiently obtained, and the movement of the needle tip protector B-14 with respect to the puncture needle body B-10 to the distal end side and to the proximal end side can be more reliably prevented.

The connection port B-56 is located on the bottom B-50 side with respect to the opening end surface of the connection hole B-48, and the connection port B-56 is arranged in a housed state in the connection hole B-48. Thus, when the distal end part of the external conduit B-60 connected to the needle hub B-18 bends with the tip of the connection port B-56 as a starting point, the amount of deformation of the external conduit B-60 is limited by contact with the peripheral wall inner surface of the connection hole B-48.

Since the cross section of the outer peripheral edge of the tip of the connection port B-56 is round shape with no corners, even if the inner circumferential surface of the external conduit B-60 is pressed against the outer peripheral edge of the tip of the connection port B-56, damage to the external conduit B-60 is unlikely to occur.

The connection port B-56 is provided in a housed state in the connection hole B-48. Thus, for example, it is possible to prevent the connection port B-56 before the external conduit B-60 is attached from interfering with a separate member or the like and being getting caught. Further, since the periphery of the connection port B-56 is protected by the peripheral wall of the connection hole B-48, damage to the connection port B-56 is also unlikely to occur.

Since the inner circumferential surface of the connection hole B-48 has a tapered shape and the connection hole B-48 increases in diameter toward the opening end, the external conduit B-60 is easy to insert into the connection hole B-48. In particular, the outer circumferential surface of the connection port B-56 comprises the tapered outer circumferential surface B-58. Thus, the spacing between the inner circumferential surface of the connection hole B-48 and the outer circumferential surface of the connection port B-56 is made large on the opening side of the connection hole B-48, thereby making it easy to insert the external conduit B-60 between the connection hole B-48 and the connection port B-56.

Moreover, the opening portion of the connection hole B-48 is provided with the large-diameter opening expansion part B-52 having a curved cross-sectional shape. Thus, when the external conduit B-60 is inserted into the connection hole B-48, the distal end of the external conduit B-60 is guided by coming into contact with the opening expansion part B-52, thereby facilitating the insertion of the external conduit B-60 into the connection hole B-48.

Since the opening portion of the connection hole B-48 is provided with the large-diameter opening expansion part B-52, the deformation of the external conduit B-60 is allowed to some extent without touching the opening portion of the connection hole B-48, thereby preventing excessive pressing of the external conduit B-60 against the peripheral wall the connection hole B-48. Besides, when the external conduit B-60 comes into contact with the opening expansion part B-52, since the opening expansion part B-52 has a curved cross-sectional shape, the external conduit B-60 is unlikely to be subjected to stress concentration due to contact with the opening expansion part B-52.

The thick reinforcing part B-54 is provided on the distal end side with respect to the connection hole B-48. Thus, in the case where, for example, the external conduit B-60 is adhered to the needle hub B-18, the portion of the needle hub B-18 that is easily damaged by the plasticizer of the adhesive comprises the reinforcing part B-54. With this configuration, damage to the needle hub B-18 is prevented, and poor adhesion between the external conduit B-60 and the needle hub B-18 and the like is avoided.

FIG. 20 depicts a puncture needle body B-100 according to a second practical embodiment of Invention B. The puncture needle body B-100 has a structure in which the proximal end part of the puncture needle B-16 is supported by a needle hub B-102. In the following description, components and parts that are substantially identical with those in the first practical embodiment of Invention B will be assigned like symbols and not described in any detail.

Regarding the needle hub B-102, the distal end portion constitutes a needle support part B-103 having a small-diameter tubular shape, while the proximal end portion constitutes a protector coupling part B-104 having a diameter larger than that of the needle support part B-103. The protector coupling part B-104 is integrally formed with the needle support part B-103, and has a connection hole B-106 that opens onto the proximal end surface.

The connection hole B-106 is a bottomed circular hole, and the inner hole of the needle hub B-102 penetrates the center of a bottom part B-108 in the axial direction. Regarding the peripheral wall inner surface of the connection hole B-106, the portion close to the bottom part B-108 has a tapered shape that gradually increases in diameter toward the opening side, while the portion close to the opening has a cylindrical surface that extends in the axial direction with an approximately constant diameter dimension. In this way, the connection hole does not necessarily have to have a tapered shape overall, and may have a partially tapered portion. The opening end edge of the connection hole B-106 comprises an opening expansion part B-110 whose cross section has a curved cross-sectional shape with no corners.

The protector coupling part B-104 includes a reinforcing part B-112 provided on the distal end side with respect to the connection hole B-106. The reinforcing part B-112 has a larger thickness dimension in the radial direction than that of the peripheral wall of the connection hole B-106. The reinforcing part B-112 includes the bottom part B-108 of the connection hole B-106. The end of the reinforcing part B-112 on the distal end side (the needle support part B-103 side) has a tapered shape that gradually decreases in diameter toward the distal end side.

The bottom part B-108 of the connection hole B-106 is provided with a connection port B-114 protruding toward the opening side of the connection hole B-106 in the axial direction. The connection port B-114 is located on the bottom part B-108 side with respect to the opening end surface of the connection hole B-106, and is provided in a housed state in the connection hole B-106.

A pair of arm parts B-116, 116 are integrally formed on the peripheral wall of the connection hole B-106. The arm parts B-116, 116 are arranged in opposition to each other in the axis-perpendicular direction, and are arranged outside the protector coupling part B-104, so as to be integrally connected with the proximal end part of the protector coupling part B-104 at the proximal end part. The arm parts B-116, 116 are arranged apart from the protector coupling part B-104, and the connecting portion with the protector coupling part B-104 is thin, so as to be elastically deformable in the direction of approaching each other by the action of an external force.

In FIG. 20, which shows a vertical cross section in the axial direction parallel to the direction of opposition of the pair of arm parts B-116, 116, the minimum thickness dimension T of the pair of arm parts B-116, 116 is set larger than the minimum thickness dimension t of the peripheral wall of the connection hole B-106. With this configuration, the strength of the arm parts B-116, 116 that are deformed by the external force when the needle hub B-102 and the needle tip protector (not shown) are separated is sufficiently secured. Moreover, when an external force acts on the arm parts B-116, 116, in the connection hole B-106, by dispersing the stress as necessary to the portion that is thinner than the minimum thickness of the arm parts B-116, 116, the stress concentration on the arm part B-116, 116 is moderated, thereby improving the durability of the arm part B-116, 116.

In the present practical embodiment, the arm parts B-116, 116 are provided so as to protrude toward the radially outer side and extend forward from the needle hub B-102. Then, in the mode in which the connection hole B-106 is provided so as to reach the distal end side with respect to the portion where the arm part B-116, 116 protrude, the minimum thickness dimension of the arm parts B-116, 116 is set larger than the minimum thickness dimension of the peripheral wall of the connection hole B-106 located on the distal end side with respect to the portion where the arm part B-116, 116 protrude in the needle hub B-102. Therefore, by setting a thickness dimension of the arm parts B-116, 116 to be even larger than that of the distal end side of the needle hub B-102, in which a required strength is higher than the rear end side with respect to the portion where the arm part B-116, 116 protrude, it is possible to secure the strength of the arm parts B-116, 116 more advantageously.

While Invention B has been described in detail hereinabove in terms of the practical embodiments, Invention B is not limited by the specific disclosures thereof. For example, the specific shape of the connection hole is not particularly limited, and the connection hole may have a diameter dimension that is approximately constant in the axial direction. Further, the inner circumferential surface of the connection hole B-48 may have, for example, a curved cross-sectional shape in which the entire surface curves in the vertical cross section.

In the connection port B-56 of the preceding practical embodiment, the entire outer circumferential surface is constituted by the tapered outer circumferential surface B-58. However, it would also be acceptable that, for example, the outer circumferential surface of the connection port B-56 on the proximal end side comprises the tapered outer circumferential surface B-58, while the outer circumferential surface of the connection port B-56 on the distal end side comprises a non-inclined tubular surface having an approximately constant outer diameter dimension. Besides, the tapered outer circumferential surface B-58 of the connection port B-56 may have a curved cross-sectional shape or a refracted cross-sectional shape in which the inclination angle changes in the axial direction. Further, the tapered outer circumferential surface B-58 is not essential, and for example, the outer diameter dimension of the connection port B-56 may be approximately constant in the axial direction. Additionally, for example, the inner diameter of the connection port B-56 may be larger or smaller toward the protruding tip in the axial direction.

For example, the connection port B-56 may be provided so as to project from the peripheral wall of the connection hole B-48 constituting the wall part of the connection hole B-48, and the bottom part B-50 is not essential in the connection hole B-48.

### [Invention C]

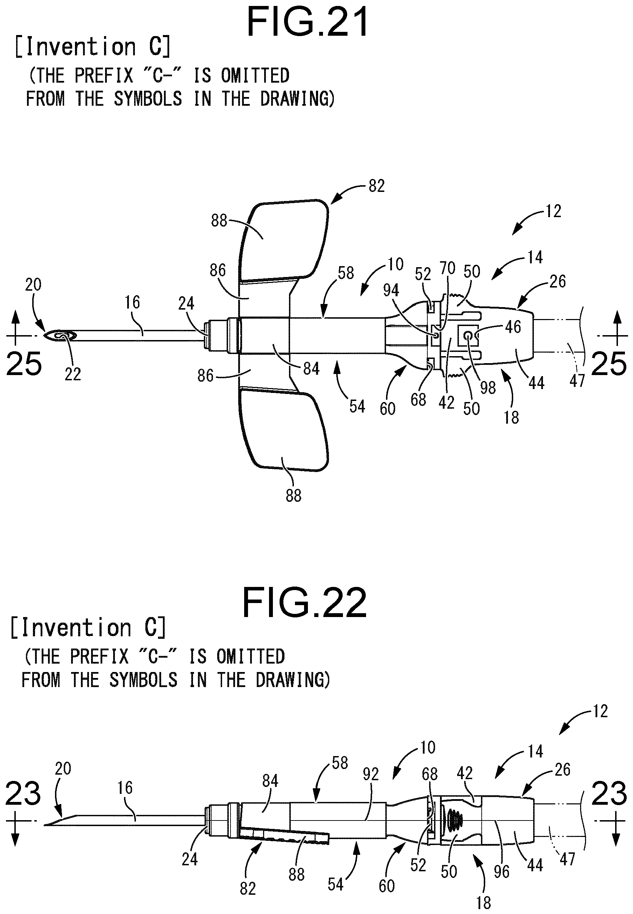

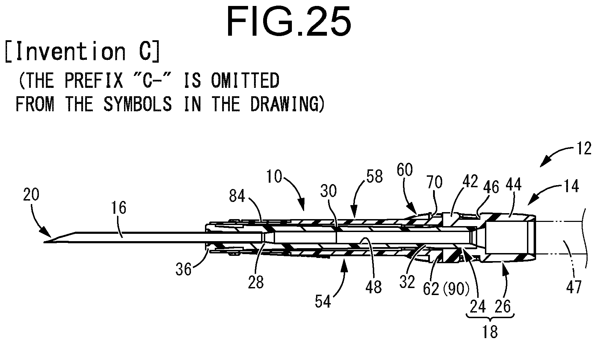

Further, FIGS. 21 to 32 show a needle assembly that can be recognized as an independent invention C capable of solving a different problem from those of Inventions A and B. Invention C relates to a needle assembly configured to protect a needle tip of a puncture needle that has been removed after the use of the puncture needle that is stuck and indwelled in a blood vessel or the like.

Conventionally, an indwelling needle used for infusion, blood collection, hemodialysis, etc. has been known. The indwelling needle has a structure in which a needle hub is fixed to the proximal end part of a puncture needle having a sharp needle tip. The indwelling needle is stuck and indwelled in a somatic lumen such as a blood vessel of the patient, and an external conduit is connected to the needle hub, so that the infusion, blood collection, hemodialysis, etc. are performed through the lumen of the indwelling needle and the external conduit.

Meanwhile, for the purpose of preventing inadvertent puncture and frequent use of a puncture needle body after use, or facilitating disposal, some puncture needle bodies include a needle tip protector that protects the needle tip of the puncture needle removed from the somatic lumen. A needle tip protector and a needle assembly including the needle tip protector are disclosed in, for example, Japanese Unexamined Patent Publication No. JP-A-2017-196060 (Patent Document 1). That is, according to the medical needle with a protector of Patent Document 1, when the needle tube is pulled out from a blood vessel or the like, the protector moves toward the needle tip side with respect to the needle tube, and the needle tube removed from the patient is accommodated in the radial inside of the protector. Therefore, the needle tip of the needle tube is prevented from being exposed to the outside.

However, a detailed examination of Patent Document 1 by the inventor found a new issue that when the needle tip protector is moved toward the needle tip side with respect to the puncture needle, the puncture needle may tilt largely relative to the needle tip protector. When the puncture needle tilts with respect to the needle tip protector, for example, the needle tip of the puncture needle removed from the patient may touch the patient's skin due to the tilt, causing pain to the patient.

It is an object of Invention C to provide a needle assembly having a novel structure which is able to suppress the tilt of the puncture needle with respect to the needle tip protector.

Hereinafter, preferred embodiments for grasping Invention C will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in Invention C, various other preferred embodiments can be realized without being limited to those described below.

A first preferred embodiment of Invention C provides a needle assembly comprising: a puncture needle provided with a needle hub on a proximal end side; and a needle tip protector attached to the needle hub and provided with a peripheral wall, the needle tip protector being configured to move relative to the puncture needle to cover a needle tip of the puncture needle, wherein a contact part is provided to an opening portion on the proximal end side of the peripheral wall, the contact part being configured to come into contact with the needle hub in the needle tip protector and limit a tilt of the needle tip protector with respect to the needle hub.

According to the needle assembly of the present preferred embodiment, the relative tilt of the puncture needle with respect to the needle tip protector can be limited by the needle hub coming into contact with the contact part provided to the opening portion on the proximal end side of the peripheral wall. By so doing, for example, when pulling out the puncture needle while holding the needle tip protector, the tilt of the needle tip of the puncture needle can be suppressed. This makes it possible to avoid problems such as the needle tip removed from the patient touching the patient's skin before being housed in the needle tip protector.

A second preferred embodiment of Invention C provides the needle assembly according to the first preferred embodiment of Invention C, wherein an opening part on the proximal end side of the peripheral wall has a flat shape in which a vertical direction is smaller than a horizontal direction, and the contact part is provided on each side in the vertical direction at the opening portion on the proximal end side of the peripheral wall.

According to the needle assembly of the present preferred embodiment, by providing the contact part on each side in the vertical direction in which the opening width is small in the opening portion on the proximal end side of the peripheral wall, the tilt of the puncture needle with respect to the needle tip protector can be effectively limited by contact of the needle hub with the contact part.

A third preferred embodiment of Invention C provides the needle assembly according to the second preferred embodiment of Invention C, wherein a locking part is provided along the horizontal direction in a radial inside of the peripheral wall, the locking part being configured to be locked to the needle hub at a movement position of the peripheral wall toward a needle tip side and prevent the peripheral wall from moving backward toward the proximal end side to prevent re-exposure of the needle tip.

According to the needle assembly of the present preferred embodiment, it is easy to secure a radially inner space of the peripheral wall relatively largely in the horizontal direction in which the opening width is large. Therefore, by utilizing the radially inner space in the horizontal direction of the peripheral wall to provide the locking part that prevents the needle tip protector from moving to the proximal end side of the puncture needle, it is possible to provide a mechanism for preventing re-exposure of the needle tip while suppressing increase in diameter of the needle tip protector particularly in the vertical direction.

A fourth preferred embodiment of Invention C provides the needle assembly according to any one of the first to third preferred embodiments of Invention C, wherein in a state where the needle tip of the puncture needle and a distal end of the peripheral wall on a needle tip side are at approximately the same position in a needle axis direction, tilt of the puncture needle with respect to the peripheral wall is limited by the contact part to such a size that the needle tip does not touch the peripheral wall.

In the state where the needle tip of the puncture needle is exposed from the peripheral wall, the tilt of the puncture needle is maximized immediately before the positions of the needle tip and the distal end of the peripheral wall coincide with each other. Therefore, according to the needle tip protector of the present preferred embodiment, the tilt of the puncture needle is limited so that the needle tip is located on the radially inner side than the peripheral wall inner surface in the state where positions of the needle tip and the distal end of the peripheral wall coincide with each other. By so doing, the tilt of the puncture needle relative to the needle tip protector is sufficiently suppressed, thereby, for example, preventing the needle tip from touching the patient or the like.

A fifth preferred embodiment of Invention C provides the needle assembly according to any one of the first to fourth preferred embodiments of Invention C, wherein a large-diameter fulcrum part is provided at the end of the needle hub on the needle tip side, and the large-diameter fulcrum part is brought into contact with the peripheral wall during tilt of the puncture needle with respect to the peripheral wall.

According to the needle assembly of the present preferred embodiment, the center of tilt of the puncture needle relative to the needle tip protector is set at the distal end part of the needle hub, and the distance from the tilt center of the puncture needle to the needle tip is made short, while the distance from the tilt center to the contact part is made long. With this configuration, when the puncture needle tilts, the movement of the needle tip is suppressed to a greater extent by the contact part.

A sixth preferred embodiment of Invention C provides the needle assembly according to any one of the first to fifth preferred embodiments of Invention C, wherein tilt of the puncture needle is limited by contact of the needle hub with the peripheral wall, and the contact part includes the opening portion on the proximal end side of the peripheral wall.

According to the needle assembly of the present preferred embodiment, direct or indirect contact of the needle hub with the peripheral wall limits the tilt of the needle hub relative to the peripheral wall. By so doing, it is not necessary to provide a special member or part for forming the contact part, and the contact part is realized by a simple structure utilizing the peripheral wall.

According to Invention C, it is possible to suppress the tilt of the puncture needle relative to the needle tip protector.

Hereinafter, practical embodiments of Invention C will be described with reference to the drawings.

FIGS. 21 to 25 show a needle assembly C-12 including a needle tip protector C-10 according to a first practical embodiment of Invention C. The needle assembly C-12 has a structure in which an indwelling needle C-14 is movably inserted through the needle tip protector C-10 in the needle axis direction. In the following description, unless otherwise specified, the distal end side refers to the left side in FIG. 21 which is a needle tip C-20 side of a puncture needle C-16, which will be described later, and the proximal end side refers to the right side in FIG. 21, which is the rear side of the puncture direction of the indwelling needle C-14. Further, as a general rule, the vertical direction refers to the vertical direction in FIG. 22, and the left-right direction refers to the vertical direction in FIG. 21, which is the horizontal direction. In FIGS. 21 to 32 according to Invention C, the description of "C-" is omitted in each reference numeral "C-XX" (where XX is a number corresponding to the part) for the sake of legibility. Besides, in order to make it easier to understand that FIGS. 21 to 32 are drawings according to Invention C, FIGS. 21 to 32 are labelled with [Invention C].

More specifically, the indwelling needle C-14 includes the puncture needle C-16 and a needle hub C-18. The puncture needle C-16 is made of a metal such as stainless steel, and has a sharp, tapered needle tip C-20 at one end. A through hole C-22 is formed at the distal end portion of the puncture needle C-16, so that blood can easily flow into the lumen of the puncture needle C-16.

The needle hub C-18 has an approximately cylindrical shape overall, and the proximal end portion of the puncture needle C-16 is fixed to the distal end portion of the needle hub C-18 in an inserted state. With this configuration, the lumen of the needle hub C-18 and the lumen of the puncture needle C-16 communicate with each other in the needle axis direction. The needle hub C-18 includes a needle support part C-24 forming its distal end portion and a protector coupling part C-26 forming its proximal end portion.

The needle support part C-24 has an approximately cylindrical shape, and is formed of, for example, a rigid synthetic resin. The inner diameter dimension at the distal end portion of the needle support part C-24 is approximately the same as the outer diameter dimension of the puncture needle C-16. An annular positioning protrusion C-28 that protrudes radially inward is provided on the inner circumferential surface of the distal end portion of the needle support part C-24. The proximal end portion of the puncture needle C-16 is inserted from the opening on the distal end side of the needle support part C-24, and the proximal end of the puncture needle C-16 and the positioning protrusion C-28 come into contact with each other, so that the puncture needle C-16 is positioned with respect to the needle hub C-18 in the needle axis direction (the left-right direction in FIG. 21). The puncture needle C-16 and the needle support part C-24 may be adhered or the like to each other, if necessary.

The outer circumferential surface of the middle portion in the needle axis direction of the needle support part C-24 comprises a tapered surface C-30 that gradually increases in diameter toward the distal end side. With this configuration, in the needle support part C-24, the middle portion in the needle axis direction having the tapered surface C-30 comprises a small-diameter tube part C-32 having a diameter smaller than that of the distal end portion and the proximal end portion.

A locking concave part C-34 that opens onto the outer circumferential surface is formed at the further distal end portion of the needle support part C-24 than the tapered surface C-30. The locking concave part C-34 has an annular shape that is continuous with an approximately constant cross-sectional shape over the entire circumference. The minimum outer diameter dimension of the needle support part C-24 at the location where the locking concave part C-34 is formed is approximately the same as the outer diameter dimension of the proximal end of the small-diameter tube part C-32, which is the minimum outer diameter dimension thereof.

An annular large-diameter fulcrum part C-36 projects from the outer circumferential surface of the needle support part C-24 on the distal end side with respect to the locking concave part C-34. The large-diameter fulcrum part C-36 is provided at the distal end or a position close to the distal end of the needle support part C-24, and constitutes the distal end portion of the needle support part C-24 in the present practical embodiment. The outer diameter dimension of the needle support part C-24 in the portion forming the large-diameter fulcrum part C-36 is larger than the outer diameter dimension of the distal end of the small-diameter tube part C-32, which is the maximum outer diameter dimension thereof.

The proximal end side surface forming the inner surface of the locking concave part C-34 comprises an annular proximal-end-side restricting surface C-38 extending in the axis-perpendicular direction, and the bottom surface of the locking concave part C-34 and the tapered surface C-30 are continuous in a stepped manner via the proximal-end-side restricting surface C-38. The distal end side surface forming the inner surface of the locking concave part C-34 comprises an annular distal-end-side restricting surface C-40 extending in the axis-perpendicular direction, and the bottom surface of the locking concave part C-34 and the outer circumferential surface of the large-diameter fulcrum part C-36 are continuous in a stepped manner via the distal-end-side restricting surface C-40. The dimension in the axis-perpendicular direction of the proximal-end-side restricting surface C-38 formed by the proximal end side inner surface of the locking concave part C-34 is slightly larger than the dimension in the axis-perpendicular direction of the distal-end-side restricting surface C-40.

The protector coupling part C-26 is made of a rigid synthetic resin, like the needle support part C-24, for example. The protector coupling part C-26 has a stepped, approximately cylindrical shape overall, and has a coupling tube C-42 having a small diameter on the distal end side and a connecting tube C-44 having a large diameter on the proximal end side. The proximal end portion of the needle support part C-24 is inserted into the coupling tube C-42, and adhesion or welding is performed as necessary, so that the protector coupling part C-26 is fixed to the needle support part C-24. An approximately rectangular concave part C-46 is formed on the surface of the protector coupling part C-26 in the vertical direction (the vertical direction in FIG. 22). As shown in FIG. 21, the concave part C-46 of the present practical embodiment is provided on the surface of the coupling tube C-42, but may be provided on the surface of the connecting tube C-44, or may be provided so as to straddle the coupling tube C-42 and the connecting tube C-44.

The protector coupling part C-26 is fixed to an external conduit C-47 by the distal end of the external conduit C-47 being inserted into the connecting tube C-44 and adhered or welded as necessary. By the needle support part C-24 and the external conduit C-47 being connected to the opposite sides of the protector coupling part C-26, the lumen of the puncture needle C-16 and the lumen of the needle hub C-18 communicate with the external conduit C-47, and a fluid flow path C-48 from the distal end opening of the puncture needle C-16 to the external conduit C-47 is provided to the indwelling needle C-14.

The connecting tube C-44 is provided with a pair of engaging arms C-50, 50 extending toward the distal end side. The engaging arms C-50, 50 are arranged in opposition to each other in the diametrical direction, and extend further to the distal end side than the coupling tube C-42 in the outer periphery of the coupling tube C-42. The engaging arms C-50, 50 are arranged apart from the coupling tube C-42 to the radially outer side, and the connecting portion with the connecting tube C-44 is thin and narrow, so as to be elastically deformable in the direction of approaching each other by the action of an external force.

A hook C-52 is formed at the distal end portion of each of the engaging arms C-50, 50. The hook C-52 projects outward in the direction of opposition of the engaging arms C-50, 50 in each engaging arm C-50. The pair of hooks C-52, 52 are configured to move in the direction of approaching each other by the engaging arms C-50, 50 elastically deforming in the direction of approach.

As shown in FIG. 26, the needle tip protector C-10 has an approximately tubular shape extending in the needle axis direction overall, and is integrally molded by using a rigid synthetic resin such as polypropylene, polycarbonate, polyethylene terephthalate glycol, and ABS resin. The needle tip protector C-10 includes a tubular peripheral wall C-54, and an inner hole C-56 penetrating in the needle axis direction is formed in the radial inside of the peripheral wall C-54.

The peripheral wall C-54 includes a cylindrical part C-58 having an approximately perfect circular ring-shaped cross section on the distal end side, and includes an expansion part C-60 having a diameter larger than that of the cylindrical part C-58 and extending radially outward on the proximal end side. Thus, the shape of the peripheral wall C-54 is varied in the needle axis direction.

As shown in FIG. 27, the expansion part C-60 has an approximately elliptical tubular shape, and the width dimension of the outer circumferential surface in the left-right direction (the horizontal direction) in FIG. 26, which is the large-diameter direction, is larger than the width dimension of the outer circumferential surface in the vertical direction in FIG. 26, which is the small-diameter direction. Of the wall parts that make up the expansion part C-60, the parts that make up the wall part in the vertical direction comprise narrow parts C-62, 62, while the parts that make up the wall part in the horizontal direction comprise wide parts C-64, 64. The direction of opposition of the narrow parts C-62, 62 and the direction of opposition of the wide parts C-64, 64 are orthogonal to each other. The expansion part C-60 may have approximately the same dimensions in the vertical direction and in the left-right direction. Further, the cross-sectional shape of the outer circumferential surface of the expansion part C-60 may be circular, rectangular, or the like.

The outer circumferential surfaces of the wide parts C-64, 64 gradually increases in outer diameter dimension from the distal end side to the proximal end side. The outer circumferential surface of the cylindrical part C-58 and the outer circumferential surface of the wide parts C-64, 64 are smoothly connected. The wall thickness dimension from the cylindrical part C-58 to the wide parts C-64, 64 is approximately constant in the needle axis direction, and is smaller than the wall thickness dimension of the narrow parts C-62, 62. In other words, the narrow parts C-62, 62 comprise thick-walled parts having a larger wall thickness dimension than the cylindrical part C-58 or the wide parts C-64, 64. In the radial inside of the expansion part C-60, an internal space C-66 having a flat cross section with its horizontal dimension larger than its vertical dimension is formed at the proximal end portion of the inner hole C-56 penetrating the needle tip protector C-10. The inside dimension in the horizontal direction of the internal space C-66 gradually increases toward the proximal end side.

The wide parts C-64, 64 are provided with through windows C-68, 68 serving as needle hub engaging parts that penetrate in the plate thickness direction. The circumferential dimension of the through windows C-68, 68 is larger than the circumferential dimension of the hooks C-52, 52. In the present practical embodiment, when the needle tip protector C-10 is molded, the mold for molding the outer circumferential surface is vertically split. Thus, the through windows C-68, 68 have a shape that will not cause any undercut at the time of mold release in the vertical direction.

A concave part C-70 is formed in the central portion in the circumferential direction on the outer circumferential surface of the narrow parts C-62, 62. The concave part C-70 has an opening on the outer surface in the vertical direction with an approximately rectangular opening shape, and is arranged apart from the through windows C-68, 68 in the circumferential direction.

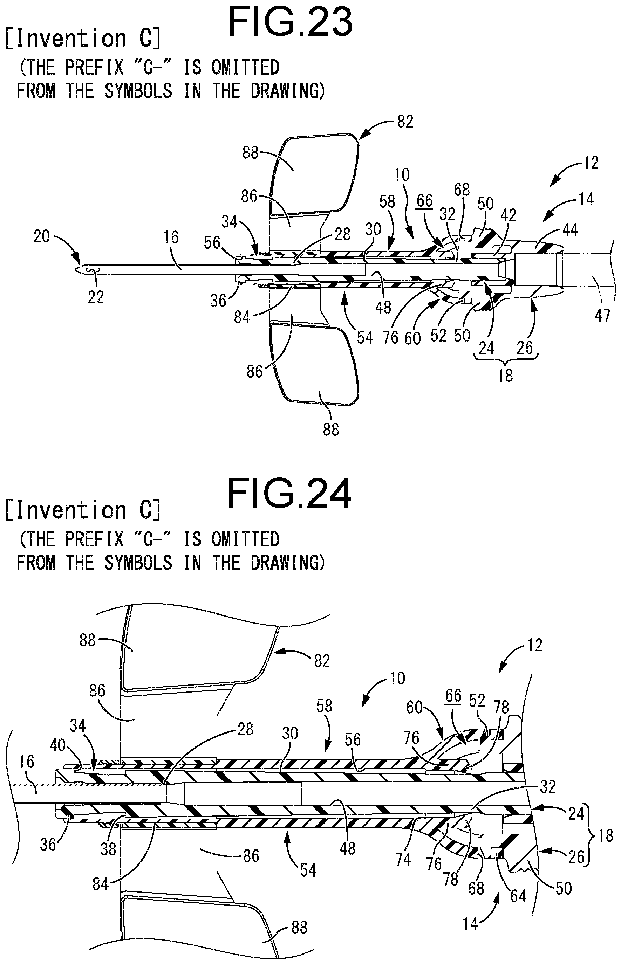

As shown in FIG. 24, an annular stepped surface C-74 extending in the axis-perpendicular direction is formed on the inner circumferential surface of the peripheral wall C-54 at the front end portion of the expansion part C-60. The inner circumferential surface of the peripheral wall C-54 has a diameter larger on the distal end side of the stepped surface C-74 than on the proximal end side thereof. The dimension in the axis-perpendicular direction of the stepped surface C-74 is smaller than the dimension in the axis-perpendicular direction of the distal-end-side restricting surface C-40 of the locking concave part C-34.

Further, in the internal space C-66, a pair of locking pieces C-76, 76 serving as locking parts is provided so as to protrude from the inner circumferential surface of the expansion part C-60 of the peripheral wall C-54 to the internal space C-66. As shown in FIGS. 24 and 27, the locking pieces C-76, 76 are integrally formed with the wide parts C-64, 64 of the expansion part C-60 of the peripheral wall C-54. The locking pieces C-76, 76 are arranged at positions corresponding to the wide parts C-64, 64 on the radially inner side of the wide parts C-64, 64. The locking pieces C-76, 76 are provided in opposition to each other in the horizontal direction and are remote from each other in the circumferential direction. The locking pieces C-76, 76 project toward the proximal end side of the peripheral wall C-54 on the proximal end side of the stepped surface C-74. The locking pieces C-76, 76 extend from the stepped surface C-74 toward the proximal end side approximately parallel to the needle axis direction, and are each curved in the circumferential direction.

A pair of locking claws C-78, 78 that bend and project radially inward are formed at the respective protruding tips (the proximal ends in the needle axis direction) of the locking pieces C-76, 76. As shown in FIG. 27, the inner circumferential surfaces of the locking claws C-78, 78 are each curved in the circumferential direction, and the radius of curvature of the inner circumferential surface at the protruding tip of the locking claws C-78, 78 is approximately equal to the radius of curvature of the outer circumferential surface of the small-diameter tube part C-32 of the needle support part C-24. Further, the distance between the opposed faces in the diametrical direction at the protruding tips of the locking claws C-78, 78 is approximately equal to the outer diameter dimension of the small-diameter tube part C-32, while being smaller than the maximum outer diameter dimension of the distal end portion of the tapered surface C-30. However, the distance between the opposed faces in the diametrical direction at the protruding tips of the locking claws C-78, 78 may be slightly smaller or slightly larger than the outer diameter dimension of the small-diameter tube part C-32.

Regarding proximal end side end faces C-80, 80 of the respective locking claws C-78, 78, the radially inner portions constitute vertical surfaces C-80a, 80a extending in the axis-perpendicular direction, while the radially outer portions constitute inclined surfaces C-80b, 80b inclining toward the distal end side in the needle axis direction toward the radially outer side. In the present practical embodiment, the dimension in the axis-perpendicular direction of the vertical surfaces C-80a, 80a is approximately the same as or slightly larger than the dimension in the axis-perpendicular direction of the proximal-end-side restricting surface C-38 of the locking concave part C-34. With this configuration, when the locking claws C-78, 78 come into contact with the proximal-end-side restricting surface C-38 as described later, the entire surface of the proximal-end-side restricting surface C-38 comes into contact with the vertical surfaces C-80a, 80a, thereby obtaining a sufficiently large contact area. Furthermore, since the inclined surfaces C-80b, 80b located on the radially outer side of the vertical surfaces C-80a, 80a incline toward the distal end side in the needle axis direction toward the radially outer side, the vertical surfaces C-80a, 80a and the proximal-end-side restricting surface C-38 come into contact with each other without any interference by the inclined surfaces C-80b, 80b.

Besides, the protruding tips (the proximal ends in the needle axis direction) of the locking claws C-78, 78 are located on the distal end side in the needle axis direction with respect to the proximal end of the expansion part C-60. Therefore, the entire locking pieces C-76, 76 are housed in the internal space C-66 of the expansion part C-60.

A wing-shaped part C-82 is provided at the distal end portion of the needle tip protector C-10. The wing-shaped part C-82 is formed of, for example, a soft synthetic resin. The wing-shaped part C-82 is integrally formed with a tubular fitting tube part C-84 with plate-shaped coupling parts C-86, 86 protruding in the tangential direction of the fitting tube part C-84. Besides, a wing main body C-88 is integrally formed on the protruding tip side of each of the coupling parts C-86, 86 from the fitting tube part C-84. The wing-shaped part C-82 is attached to the peripheral wall C-54 of the needle tip protector C-10 by the fitting tube part C-84 being fitted externally onto the distal end portion of the cylindrical part C-58.

The indwelling needle C-14 is inserted through the inner hole C-56 of the needle tip protector C-10, so that the needle tip protector C-10 is externally mounted onto the indwelling needle C-14, thereby constituting the needle assembly C-12. In the initial state before use of the needle assembly C-12 shown in FIG. 21 and the like, the needle tip C-20 of the indwelling needle C-14 is located on the distal end side with respect to the needle tip protector C-10, and the needle tip C-20 is exposed. In this initial state, the hooks C-52, 52 provided to the needle hub C-18 of the indwelling needle C-14 are inserted and locked in the through windows C-68, 68 provided to the expansion part C-60 of the needle tip protector C-10. With this configuration, the needle tip protector C-10 and the needle hub C-18 are coupled to each other, and the protruding state of the needle tip C-20 is maintained. Besides, for example, when puncture is performed by holding the wing-shaped part C-82, the movement of the puncture needle C-16 toward the proximal end side in the needle axis direction due to resistance during the puncture or the like is restricted.

In the initial state of the needle assembly C-12, the locking claws C-78, 78 of the needle tip protector C-10 are in contact with the outer circumferential surface of the small-diameter tube part C-32 of the needle support part C-24. These locking claws C-78, 78 may be slightly pushed radially outward by being in contact with the outer circumferential surface of the small-diameter tube part C-32, or may be slightly remote from the outer circumferential surface of the small-diameter tube part C-32.

The needle assembly C-12 is used for infusion, blood collection, and hemodialysis through the fluid flow path C-48 by the puncture needle C-16 being stuck into the patient's blood vessel and the indwelling needle C-14 being indwelled therein. Since the needle assembly C-12 of the present practical embodiment is provided with the wing-shaped parts C-82, it is possible to stick the puncture needle C-16 of the indwelling needle C-14 while, for example, pinching the wing-shaped parts C-82. When indwelling the indwelling needle C-14 in the punctured state, by fixing with a tape fastener at the positions of the wing-shaped parts C-82, it is possible to fix to the skin with a wide touch area.

When the indwelling needle C-14 is removed, the engaging arms C-50, 50 of the needle hub C-18 are pressed inward by fingers while the needle tip protector C-10 is kept fixed with a tape fastener at the wing-shaped parts C-82. By so doing, the hooks C-52, 52 and the through windows C-68, 68 are detached from each other, making it possible to move the indwelling needle C-14 toward the proximal end side with respect to the needle tip protector C-10. By moving the indwelling needle C-14 toward the proximal end side with respect to the needle tip protector C-10 and removing the puncture needle C-16 from the skin, the needle tip protector C-10 moves to the needle tip C-20 side with respect to the indwelling needle C-14.

With the hooks C-52, 52 and the through windows C-68, 68 disengaged from each other, unless an external force is applied to the indwelling needle C-14 to move toward the proximal end side with respect to the needle tip protector C-10, the indwelling needle C-14 is configured not to move toward the proximal end side with respect to the needle tip protector C-10. That is, since the locking claws C-78, 78 are in contact with the tapered surface C-30 that increases in diameter toward the distal end side, the indwelling needle C-14 is prevented from moving toward the proximal end side with respect to the needle tip protector C-10. With this configuration, it is possible to prevent a trouble that the needle tip C-20 of the indwelling needle C-14 is accidentally protected by the needle tip protector C-10.

By moving the indwelling needle C-14 toward the proximal end side with respect to the needle tip protector C-10, the locking claws C-78, 78 come into sliding contact with the tapered surface C-30, and elastic recovery force of the locking pieces C-76, 76 acts as an urging force for pressing the locking claws C-78, 78 against the tapered surface C-30. Therefore, the user can move the indwelling needle C-14 while confirming the feeling that the indwelling needle C-14 is being pulled out from the needle tip protector C-10 by the resistance force due to the contact between the locking claws C-78, 78 and the tapered surface C-30.

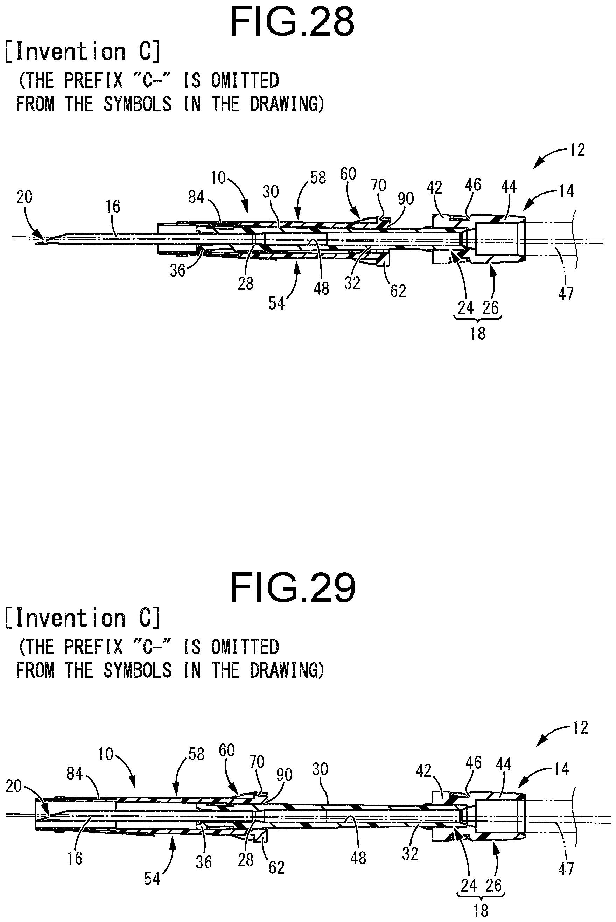

When the indwelling needle C-14 is moved toward the proximal end side with respect to the needle tip protector C-10, the indwelling needle C-14 may tilt with respect to the needle tip protector C-10. That is, except that the locking claws C-78, 78 are in sliding contact with the tapered surface C-30, there is a gap between the needle support part C-24 of the indwelling needle C-14 and the peripheral wall C-54 of the needle tip protector C-10. Therefore, when the hooks C-52, 52 and the through windows C-68, 68 are disengaged from each other and the indwelling needle C-14 moves toward the proximal end side with respect to the needle tip protector C-10, the central axis of the needle tip protector C-10 may tilt relative to the needle axis, which is the central axis of the indwelling needle C-14. When the indwelling needle C-14 tilts in the vertical direction with respect to the needle tip protector C-10, the indwelling needle C-14 comes into contact with the peripheral wall C-54 of the needle tip protector C-10 at the large-diameter fulcrum part C-36 provided at the distal end portion of the needle hub C-18. Therefore, the vertical tilt of the indwelling needle C-14 with respect to the needle tip protector C-10 occurs around the contact portion between the large-diameter fulcrum part C-36 and the peripheral wall C-54, as shown in FIG. 28.

Then, the needle hub C-18 comes into direct contact with the narrow part C-62 of the expansion part C-60 at the position sufficiently distant from the contact portion between the large-diameter fulcrum part C-36, which is the tilt center, and the peripheral wall C-54 toward the proximal end side, whereby the vertical tilt of the indwelling needle C-14 with respect to the needle tip protector C-10 is limited. In this way, a contact part C-90 for the vertical direction, which functions as a tilt limiting part that limits the tilt of the indwelling needle C-14 by coming into contact with the needle hub C-18, is provided in the opening portion on the proximal end side of the peripheral wall C-54 of the needle tip protector C-10. The contact part C-90 of the present practical embodiment is constituted by the radially inner portions of the narrow parts C-62, 62 that form the opening portion on the proximal end side of the peripheral wall C-54, and the contact part C-90 is provided on each side in the vertical direction.

Due to such tilt limitation of the indwelling needle C-14 by the contact part C-90, when the indwelling needle C-14 is pulled out toward the proximal end side with respect to the needle tip protector C-10, the tilt of the puncture needle C-16 is limited and the movement of the needle tip C-20 in the axis-perpendicular direction is suppressed. This avoids problems such as the needle tip C-20 of the puncture needle C-16 removed from the blood vessel or the like touching the patient's skin due to the vertical tilt of the indwelling needle C-14 and injuring the patient.

Preferably, in a state where the needle tip C-20 shown in FIG. 29 is at the same position in the needle axis direction with respect to the distal end of the peripheral wall C-54 of the needle tip protector C-10, the tilt of the indwelling needle C-14 is limited by the contact part C-90 so that the needle tip C-20 does not come into contact with the peripheral wall C-54 due to the tilt of the indwelling needle C-14. By so doing, in the state where the needle tip C-20 is exposed further to the distal end than the peripheral wall C-54 of the needle tip protector C-10, the needle tip C-20 will not be located on the radially outer side of the peripheral wall C-54, thereby effectively preventing the needle tip C-20 from touching the patient's skin or the like.

With the needle tip C-20 being at the same position in the needle axis direction with respect to the distal end of the peripheral wall C-54 of the needle tip protector C-10, it is desirable that the large-diameter fulcrum part C-36, which is the center of tilt of the indwelling needle C-14 with respect to the needle tip protector C-10, is located on the proximal end side with respect to the axial center of the needle tip protector C-10. In other words, in the aforementioned state, it is desirable that the distance from the needle tip C-20 to the large-diameter fulcrum part C-36 is longer than 1/2 of the axial length of the needle tip protector C-10. More preferably, the distance from the needle tip C-20 to the large-diameter fulcrum part C-36 longer than 2/3 of the axial length of the needle tip protector C-10.

The expansion part C-60 constituting the proximal end portion of the peripheral wall C-54 has a flat shape in which the vertical dimension is smaller than the horizontal dimension, and the inside dimension in the vertical direction is smaller than the inside dimension in the horizontal direction. With this configuration, when the indwelling needle C-14 tilts in the vertical direction with respect to the needle tip protector C-10, the needle hub C-18 quickly comes into contact with the contact part C-90 at a stage where the tilt is relatively small, thereby limiting the tilt of the indwelling needle C-14 to a small extent. Besides, the proximal end portion of the needle tip protector C-10 secure a sufficient inside dimension in the horizontal direction while the inside dimension in the vertical direction is made small. Thus, it is possible to secure a space for arranging the locking pieces C-76, 76 and the like in the horizontal direction while effectively limiting the tilt of the indwelling needle C-14 in the vertical direction.

Meanwhile, the tilt of the indwelling needle C-14 in the horizontal direction with respect to the needle tip protector C-10 is limited to some extent by the large-diameter fulcrum part C-36 of the needle hub C-18 coming into contact with the inner circumferential surface of the peripheral wall C-54 and the locking pieces C-76, 76 coming into contact with the outer circumferential surface of the needle hub C-18. With this configuration, in the needle tip protector C-10 of the present practical embodiment, a contact part for the horizontal direction is provided to the locking pieces C-76, 76, and the locking pieces C-76, 76 constitute a tilt limiting part for the horizontal direction.

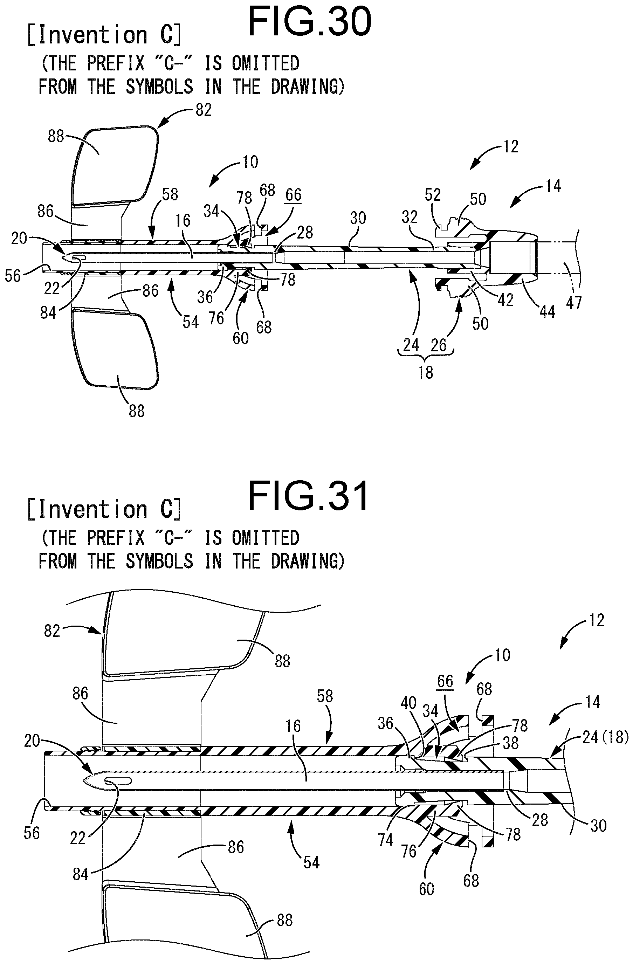

As shown in FIGS. 30 and 31, by the indwelling needle C-14 being moved backward to a predetermined position with respect to the needle tip protector C-10 (the needle tip protector is moved forward to the needle tip C-20 side of indwelling needle C-14), the needle tip C-20 of the puncture needle C-16 is covered with the needle tip protector C-10. Moreover, the locking claws C-78, 78 provided on the free end side of the locking pieces C-76, 76 move over the opening of the locking concave part C-34, so that the locking claws C-78, 78, which elastically recover their shapes, enter the locking concave part C-34. Then, the vertical surfaces C-80a, 80a of the locking claws C-78, 78 and the proximal-end-side restricting surface C-38 of the locking concave part C-34 are brought into contact and locked to each other, so that the movement of the indwelling needle C-14 toward the distal end side with respect to the needle tip protector C-10 is restricted, thereby preventing re-exposure of the needle tip C-20 of the indwelling needle C-14.

The stepped surface C-76 provided at the front end portion of the locking pieces C-76, 76 and the distal-end-side restricting surface C-40 of the locking concave part C-34 are brought into contact and locked to each other, so that movement of the indwelling needle C-14 to the proximal end side is restricted. By so doing, the indwelling needle C-14 is prevented from becoming dislodged to the proximal end side with respect to the needle tip protector C-10. Therefore, movement of the indwelling needle C-14 to the opposite sides in the axial direction with respect to the needle tip protector C-10 is restricted, thereby maintaining the protected state of the needle tip C-20 by the needle tip protector C-10.

In this practical embodiment, the pair of locking pieces C-76, 76 are provided, and in combination with the fact that the locking pieces C-76, 76 are integrally molded with the peripheral wall C-54 of the needle tip protector C-10, when the locking claws C-78, 78 and the proximal-end-side restricting surface C-38 are locked, troubles such as the needle tip protector C-10 rattling with respect to the indwelling needle C-14 can be effectively prevented. By providing two, or three or more locking pieces C-76 in this way, the needle tip protector C-10 is more reliably prevented from moving backward to the proximal end side with respect to the indwelling needle C-14, thereby more stably exhibiting the effect of preventing re-exposure of the needle tip C-20. In this case, it is preferable that the plurality of locking pieces C-76 are arranged approximately symmetrically with respect to the central axis of the needle tip protector C-10.

Further, in a state where the needle tip C-20 is housed in the peripheral wall C-54 of the needle tip protector C-10, it is desirable that the tilt of the indwelling needle C-14 with respect to the peripheral wall C-54 is limited to such a size that the needle tip C-20 does not touch the inner circumferential surface of the peripheral wall C-54.

With the needle tip C-20 of the indwelling needle C-14 protected by the needle tip protector C-10, the tape fixing of the wing-shaped parts C-82 is released, and the needle assembly C-12 is removed from the patient. However, the procedure for protecting the needle tip C-20 is not limited to the aforementioned one. Specifically, for example, it would also be acceptable to release the tape fixing at the wing-shaped part C-82, and move the entire needle assembly C-12 backward to remove the puncture needle C-16 from the blood vessel, and then move the indwelling needle C-14 backward with respect to the needle tip protector C-10, thereby protecting the needle tip C-20 of the puncture needle C-16.

When the locking claws C-78, 78 enter the locking concave part C-34, the tips of the locking claws C-78, 78 come into contact with the bottom surface of the locking concave part C-34, so that the user can confirm the impact and sound of the contact. Therefore, for example, the risk that the user may stop the pulling-out operation of the indwelling needle C-14 midway or the like is avoided, thereby more reliably protecting the needle tip C-20 with the needle tip protector C-10.

In the needle tip protector C-10 and the needle assembly C-12 having the above-mentioned structure, the locking pieces C-76, 76 that prevent the re-exposure of the needle tip C-20 of the indwelling needle C-14 are provided so as to be entirely housed inside the tubular peripheral wall C-54. This makes it almost impossible to unintentionally touch the locking pieces C-76, 76 from the outside. Therefore, in the protected state of the needle tip C-20 of the indwelling needle C-14 by the needle tip protector C-10, it is possible to effectively prevent the locking pieces C-76, 76 and the locking concave part C-34 from being accidentally disengaged from each other to re-expose the needle tip C-20 from the needle tip protector C-10.

With the locking pieces C-76, 76 locked to the needle hub C-18, for example, when an external force such as a force in the bending direction is applied to the needle tip protector C-10 for an indwelling needle and/or the needle hub C-18, the locking pieces C-76, 76 may come into contact with the wide parts C-64, 64 located apart to the radially outer side. By so doing, the deformation of the locking pieces C-76, 76 to the radially outer side is restricted, and the risk of the locking pieces C-76, 76 being disengaged from the needle hub C-18 is reduced, thereby stably maintaining the protected state of the needle tip C-20 of the indwelling needle C-14.

The locking pieces C-76, 76 are provided in the expansion part C-60 expanded on the proximal end side of the needle tip protector C-10. With this configuration, while obtaining a sufficient size of the locking pieces C-76, 76, the outer diameter dimension of the needle support part C-24 of the needle hub C-18 inserted between the locking pieces C-76, 76 can also be made large enough. Furthermore, in the present practical embodiment, the expansion part C-60 has an approximately elliptical shape, namely a smooth curved surface shape in which a portion configured to touch the patient has no break points or corners, and the outer circumferential surface thereof is smoothly continuous from the outer circumferential surface of the cylindrical part C-58. This reduces the risk that the expansion part C-60 or the like may come into contact with the patient so that the patient feels pain.

The locking pieces C-76, 76 are provided inside the wide parts C-64, 64 constituting the expansion part C-60. This prevents the wide parts C-64, 64 from being thick, thereby suppressing mixing of air bubbles in a component during molding, occurrence of dimensional errors and deterioration in quality due to generation of sink marks and voids, and the like. Further, it is preferable that the locking part (the locking pieces C-76, 76) is provided so as to extend in the same direction as the wide parts C-64, 64 extend (to the proximal end side in the axial direction). With this configuration, the expansion part C-60 is unlikely to become larger than necessary, so that the space inside the wide parts C-64, 64 can be used skillfully, and the wide parts C-64, 64 are prevented from being thick.

Since the locking pieces C-76, 76 extend toward the proximal end side, they do not get caught when the indwelling needle C-14 is pulled out with respect to the needle tip protector C-10, thereby realizing smooth pulling out.

The locking pieces C-76, 76 are provided in pairs in opposition to each other in the diametrical direction. Thus, in the protected state of the needle tip C-20 by the needle tip protector C-10, that is, in the locked state where the locking pieces C-76, 76 are inserted in the locking concave part C-34, the fitting tube part C-84 is clasped between the locking pieces C-76, 76 in the diametrical direction. Therefore, the needle tip protector C-10 and the indwelling needle C-14 (the needle support part C-24) do not wobble in the diametrical direction, and the protected state of the needle tip C-20 of the indwelling needle C-14 by the needle tip protector C-10 can be stably maintained.

The vertical surfaces C-80a, 80a of the locking claws C-78, 78 and the proximal-end-side restricting surface C-38 of the locking concave part C-34, which are mutually locked, all extend in the axis-perpendicular direction. Moreover, the stepped surface C-74 and the distal-end-side restricting surface C-40 of the locking concave part C-34, which are mutually locked, both extend in the axis-perpendicular direction. Therefore, the needle tip protector C-10 having the vertical surfaces C-80a, 80a and the stepped surface C-74, as well as the needle hub C-18 having the proximal-end-side restricting surface C-38 and the distal-end-side restricting surface C-40 do not have undercut shape and are easy to mold. Moreover, the contact force and the contact reaction force due to contact between the vertical surfaces C-80a, 80a and the proximal-end-side restricting surface C-38 act without being inclined with respect to the axial direction. Similarly, the contact force and the contact reaction force due to contact between the stepped surface C-74 and the distal-end-side restricting surface C-40 act without being inclined with respect to the axial direction. Besides, each contact area can be sufficiently obtained, and the movement of the needle tip protector C-10 with respect to the indwelling needle C-14 to the distal end side and to the proximal end side can be more reliably prevented.

The locking pieces C-76, 76 are provided so as to project into the radially inner space of the peripheral wall C-54. Thus, for example, when the needle tip protector C-10 is manufactured by molding, the mold for forming the radially inner side of the needle tip protector C-10 can be removed in the needle axis direction of the needle tip protector C-10. Therefore, the number of types of molds can be minimized, and the production efficiency of the needle tip protector C-10 can be improved.

Regarding the needle tip protector C-10, for example, the radially inner shape is molded by a mold combined in the axial direction, and the outer peripheral shape is molded by a mold combined in the vertical direction. Since the outer circumferential surface of the needle tip protector C-10 is molded by a mold combined in the vertical direction, as shown in FIG. 22, a parting line C-92 protruding from the parting surface of the mold is formed so as to extend in the axial direction on the lateral outer surface of the needle tip protector C-10. With this configuration, the parting line C-92 is less likely to touch the patient, and the pain or the like caused by the contact of the parting line C-92 is avoided.

A gate trace C-94, which is a trace of a gate that fills the cavity of the mold with a resin material when molding the needle tip protector C-10, is provided in the concave part C-70 of the needle tip protector C-10 (see FIG. 21). Therefore, even if the gate trace C-94 is formed so as to project from the vertical surface of the needle tip protector C-10, the gate trace C-94 is housed in the concave part C-70, so that the gate trace C-94 is less likely to touch the patients or the like. Moreover, the opening area of the concave part C-70 is smaller than the size of the fingertip. This makes it difficult for the fingertip to enter the concave part C-70, and a health care worker who operates the needle tip protector C-10 by holding it with his/her fingertip is less likely to touch the gate trace C-94 in the concave part C-70. Therefore, for example, even in the case where the gate is a pin gate that is pulled and cut off when the mold is demolded and the gate trace C-94 remains protruding, touch on the gate trace C-94 is unlikely to become a problem.

By charging the resin material from the portion where the cavity of the mold is wide, poor charge of the resin material or the like is unlikely to occur. However, when the resin material is charged from the expansion part C-60 having a large diameter, the gate trace C-94 is formed on the radially outer side of the needle support part C-24 having a small diameter, so as to be likely to touch the patient or the like. Nevertheless, since the gate trace C-94 is housed in the concave part C-70, it is possible to set the position of the gate to the proximal end portion of the needle tip protector C-10 having a large diameter while preventing the patient or the like from touching the gate trace C-94, thereby efficiently manufacturing the needle tip protector C-10.

Similarly, the protector coupling part C-26 of the needle hub C-18 is also formed by a mold divided into upper and lower parts, and since a parting line C-96 projects from the lateral surface, the parting line C-96 of the protector coupling part of C-26 is less likely to touch the patient. Further, a gate trace C-98 of the protector coupling part C-26 protrudes from the bottom surface of the concave part C-46 formed on the vertical surface of the protector coupling part C-26, and the gate trace C-98 is housed in the concave part C-46. Therefore, even if the gate trace C-98 is formed on the vertical surface of the protector coupling part C-26, the gate trace C-98 is less likely to touch the patient. The opening area of the concave part C-46 is smaller than the size of the fingertip, and a health care worker who operates the needle hub C-18 by holding it with his/her fingertip is also less likely to the gate trace C-98 in the concave part C-46. The gate trace C-98 may be provided on the wide part C-64, 64 side. With this configuration, a weld line is less likely to occur in the wide parts C-64, 64 serving as the engaging part including the through windows C-68, 68, which are likely to be thin in the needle tip protector C-10. As a result, the strength of the wide parts C-64, 64 is increased, and the engaging arms C-50, 50 of the needle hub C-18 can be more firmly engaged with the needle tip protector C-10. Therefore, unintentional separation of the needle hub C-18 and the needle tip protector C-10 or the like can be avoided, thereby preventing malfunction of the needle tip protection mechanism. Besides, it is possible to prevent troubles such as the needle tip protection mechanism failing to normally function due to, for example, the wide parts C-64, 64 being destroyed by an external force.

A practical embodiment of Invention C has been described in detail above, but Invention C is not limited to those specific descriptions. In the preceding practical embodiment, the contact part C-90 that limits the vertical tilt of the indwelling needle C-14 with respect to the needle tip protector C-10, and the contact part (the locking pieces C-76, 76) that limits the lateral tilt are both provided. However, it would also be possible to provide only the contact part for the vertical direction.

The preceding practical embodiment illustrated the structure in which the contact part for limiting the horizontal tilt of the indwelling needle C-14 with respect to the needle tip protector C-10 is constituted by the locking pieces C-76, 76. However, it would also be possible to constitute the contact part for the horizontal direction by contact between the needle hub C-18 of the indwelling needle C-14 and the peripheral wall C-54 (the wide parts C-64, 64) of the needle tip protector C-10.

Regarding the contact part C-90, it would also be possible to, for example, make the narrow parts C-62, 62 thickened toward the radially inner side, or to provide a convex part protruding from the narrow parts C-62, 62 toward the radial inside, thereby adjusting the vertical clearance of the indwelling needle C-14 with respect to the needle hub C-18. With this configuration, the allowable range of tilt of the indwelling needle C-14 with respect to the needle tip protector C-10 can be adjusted. By providing a recess on the inner circumferential surface of the narrow parts C-62, 62, or by making the narrow parts C-62, 62 thin on the radially inner side, the tilt of the indwelling needle C-14 with respect to the needle tip protector C-10 will be more greatly allowed.

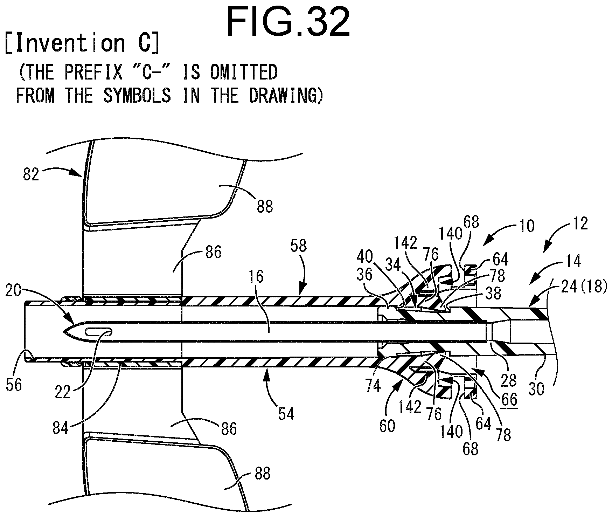

For example, as shown in FIG. 32, in the internal space C-66 of the expansion part C-60, it would also be possible to provide deformation amount limiting parts C-140, 140 that limit the deformation amount of the locking pieces C-76, 76 to the radially outer side. The deformation amount limiting part C-140 is constituted by a protrusion C-142 protruding from the inner circumferential surface of the expansion part C-60 toward the proximal end side. It would also be acceptable to provide a plurality of protrusions C-142 side by side so as to be remote from each other in the circumferential direction between the narrow parts C-62, 62, for example. The protrusions C-142 are provided at a predetermined distance in the horizontal direction (the vertical direction in FIG. 32) with respect to the locking piece C-76, and elastic deformation of the locking piece C-76 outward in the horizontal direction is allowed.

By providing such deformation amount limiting parts C-140, 140, movement of the locking piece C-76, 76 outward in the horizontal direction is limited by the deformation amount limiting parts C-140, 140, so that the locking pieces C-76, 76 and the proximal-end-side restricting surface C-38, 38 are less likely to be disengaged. Moreover, by limiting the amount of deformation of the locking pieces C-76, 76 outward in the horizontal direction, the horizontal tilt of the indwelling needle C-14 and the needle tip protector C-10 is effectively limited by the locking piece C-76, 76. In the structure of FIG. 32, the horizontal tilt of the indwelling needle C-14 is limited by the needle hub C-18 of the indwelling needle C-14 coming into contact with the deformation amount limiting parts C-140, 140 via the locking pieces C-76, 76. Therefore, in the structure of FIG. 32, the contact part that comes into contact with the needle hub C-18 is constituted by the locking pieces C-76, 76, and the tilt limiting part that limits the horizontal tilt of the needle hub C-18 is constituted by the locking pieces C-76, 76 and the wide parts C-64, 64 of the peripheral wall C-54 including the deformation amount limiting parts C-140, 140.

The opening portion on the proximal end side of the peripheral wall C-54 in the preceding practical embodiment comprise the expansion part C-60 having the narrow parts C-62, 62 and the wide parts C-64, 64, and has a flat shape in which vertical dimension is smaller than the horizontal dimension. However, the opening portion on the proximal end side of the peripheral wall C-54 may have a perfect circular cross section, for example. Further, the opening portion on the proximal end side of the peripheral wall C-54 has a diameter larger than that of the cylindrical part C-58 which is the distal end portion. However, the peripheral wall C-54 may have an approximately constant diameter entirely in the axial direction, or may have a smaller diameter dimension at the proximal end portion than at the distal end portion. The cross-sectional shape of the peripheral wall C-54 is preferably circular or elliptical shape with no corners, but may be polygonal shape or irregular or deformed shape, for example.

The large-diameter fulcrum part C-36, which is the center of tilt of the indwelling needle C-14 and the needle tip protector C-10, is preferably provided at the distal end portion of the needle hub C-18. However, the large-diameter fulcrum part C-36 may be provided at, for example, the axially middle portion of the needle support part C-24 of the needle hub C-18. It would be acceptable as long as the large-diameter fulcrum part is located on the distal end side with respect to the contact part, which is provided in the opening portion on the proximal end side of the needle tip protector C-10, in a state where the needle tip C-20 is housed in the needle tip protector C-10.

### [Invention D]

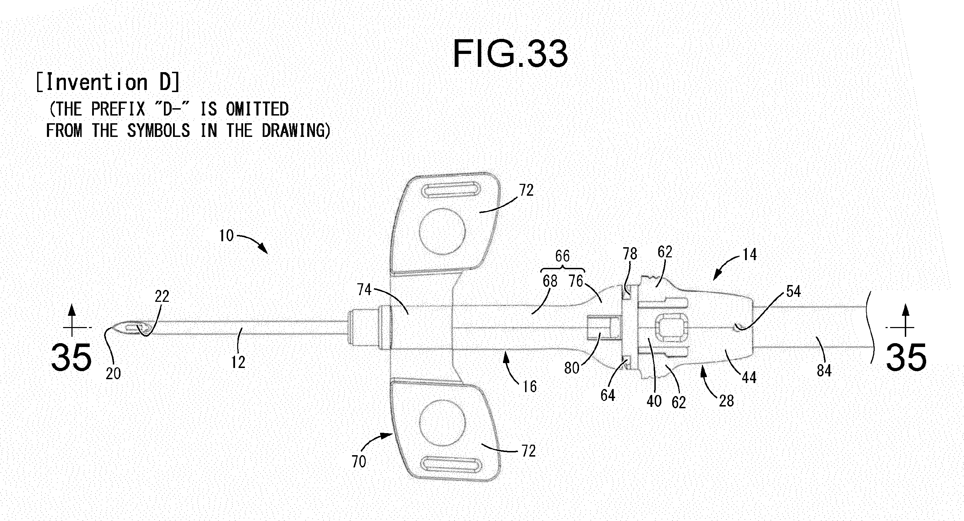

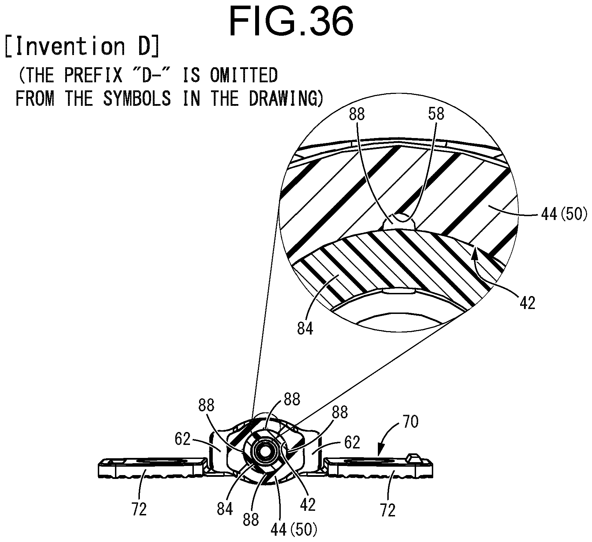

Further, FIGS. 33 to 37 show a needle assembly that can be recognized as an independent invention D capable of solving a different problem from those of Inventions A to C. Invention D relates to a needle assembly used as, for example, an indwelling needle configured to be stuck and indwelled in a blood vessel.

Conventionally, a needle assembly used for infusion, medication, artificial dialysis, etc. has been known. The needle assembly has a structure in which a needle hub is provided on the proximal end side of a hollow needle, as shown in, for example, Japanese Unexamined Patent Publication No. JP-A-2017-196060 (Patent Document 1).

Meanwhile, as shown in Patent Document 1, a flexible tube may be connected to the needle hub. In Patent Document 1, a connecting tube part is provided on the proximal end side of the needle hub, and the flexible tube is connected to the needle hub by being inserted into the radial inside of the connecting tube part. Besides, the connecting tube part of the needle hub and the flexible tube may be fixed to each other by, for example, adhesion with an adhesive.

However, as a result of examination by the inventor, a new issue was found. Namely, in the structure described in Patent Document 1, when the needle hub and the flexible tube are to be adhered to each other, it is difficult to bond an appropriate area between the needle hub and the flexible tube with an adhesive. Specifically, for example, there is a possibility of improper adhesion mode that the adhesive will not reach the entire area to be adhered, or the adhesive will reach so far as the distal end side of the flexible tube and obstructs the lumen of the flexible tube, or the like, and a structure capable of achieving more appropriate adhesion between the needle hub and the flexible tube has been required.

It is an object of Invention D to provide a needle assembly having a novel structure in which the needle hub and the flexible tube can be appropriately adhered to each other.

Hereinafter, preferred embodiments for grasping Invention D will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in Invention D, various other preferred embodiments can be realized without being limited to those described below.

A first preferred embodiment of Invention D provides a needle assembly comprising a puncture needle and a needle hub provided on a proximal end side of the puncture needle, wherein a connection hole opening toward the proximal end side of the needle hub is provided, an end part of a tube having flexibility is inserted into the connection hole, and the tube is fitted into a fitting part provided to the connection hole with an inner diameter dimension that is not greater than an outer diameter dimension of the tube, while an adhesive part having an enlarged diameter is provided on the proximal end side of the connection hole with respect to the fitting part, and an outer circumferential surface of the tube is adhered to the adhesive part.

According to the needle assembly structured following the present preferred embodiment, for example, in a state where the tube is fitted into the fitting part on the distal end side with respect to the adhesive part, when the adhesive is injected between the inner circumferential surface of the adhesive part and the outer circumferential surface of the tube, the injected adhesive is held between the adhesive and the tube. By so doing, the adhesive is less likely to wrap around to the distal end side of the tube, thereby preventing the lumen of the needle hub and the lumen of the tube from being narrowed or obstructed by the adhesive.

Further, in the case where the end of the tube is inserted into the connection hole after the adhesive is applied to at least one of the outer circumferential surface of the tube and the inner circumferential surface of the adhesive part, the adhesive is less likely to reach the distal end side with respect to the fitting part. That is, for example, even if the adhesive adheres to the outer circumferential surface of the end of the tube when the end of the tube passes through the radial inside of the adhesive part, the adhered adhesive is scraped off by the tube being fitted into the fitting part, and the adhesive is less likely to enter between the tube and the fitting part. Thus, the adhesive is held between the tube and the adhesive part. As a result, it is possible to prevent the adhesive from entering the lumen of the needle hub on the distal end side of the tube, and prevent the lumen of the needle hub and the lumen of the tube from being narrowed or obstructed by the adhesive.

Further, since the tube is fitted into the fitting part, the end of the tube is positioned with respect to the needle hub before being adhered to the adhesive part. Therefore, the tube can be easily adhered to the needle hub, thereby facilitating manufacture and achieving stabilization of the quality of the adhered portion.

A second preferred embodiment of Invention D provides the needle assembly according to the first preferred embodiment of Invention D, wherein at least one communication passage is provided, the communication passage placing a radially inner region of the adhesive part in communication with an external space at a position apart from a proximal end of the adhesive part toward the distal end side.

When injecting the adhesive, the air existing in the radially inner region of the adhesive part needs to be discharged to the outside. Here, for example, when the adhesive is injected between the adhesive part and the tube from the proximal end side, there is a possibility that the air may be sealed on the distal end side with respect to the adhesive, and the infiltration of the adhesive into the distal end side may be hindered by the air. According to the needle assembly structured following the present preferred embodiment, since the radially inner region of the adhesive part communicates with the external space through the communication passage on the distal end side, the air is discharged to the external space through the communication passage when the adhesive is injected from the proximal end side. Therefore, the adhesive is introduced to the distal end side without being blocked by the air, so that adhesion mode is stabilized. Besides, the adhesive part is adhered to the tube over a wide range, thereby obtaining a large adhesive strength.

Additionally, for example, it is also possible to inject the adhesive between the adhesive part and the tube through the communication passage. By so doing, since the adhesive is injected from the distal end side, the air between the adhesive part and the tube is discharged to the outside from the proximal end opening of the connection hole, and the adhesive is introduced to the proximal end side, thereby securing a large adhesion area between the adhesive part and the tube.

A third preferred embodiment of Invention D provides the needle assembly according to the second preferred embodiment of Invention D, wherein the at least one communication passage comprises a plurality of communication passages that are arranged at a plurality of locations in a circumferential direction of the needle hub.

According to the needle assembly structured following the present preferred embodiment, even if the flexible tube deforms to obstruct the opening of the communication passage, since the plurality of communication passages are arranged at the plurality of locations in the circumferential direction, the air can be discharged or the adhesive can be injected through other passages that are not obstructed by the tube.

A fourth preferred embodiment of Invention D provides a needle assembly comprising a puncture needle and a needle hub provided on a proximal end side of the puncture needle, wherein a connection hole opening toward the proximal end side of the needle hub is provided, an end part of a tube having flexibility is inserted into the connection hole, and an outer circumferential surface of the tube is adhered to an adhesive part provided to the connection hole with an inner diameter dimension that is not smaller than an outer diameter dimension of the tube, while a plurality of communication passages are provided at a plurality of locations in a circumferential direction of the needle hub, the communication passages placing a radially inner region of the adhesive part in communication with an external space at a position apart from a proximal end of the adhesive part toward the distal end side.

According to the needle assembly structured following the present preferred embodiment, the radially inner region of the adhesive part communicates with the external space through the communication passages on the distal end side. Thus, when the adhesive is injected from the proximal end side, the air is discharged to the external space through the communication passages. Therefore, the adhesive is introduced to the distal end side without being blocked by the air, so that adhesion mode is stabilized. Besides, the adhesive part is adhered to the tube over a wide range, thereby obtaining a large adhesive strength.

Additionally, for example, it would also be also acceptable to inject the adhesive between the adhesive part and the tube through the communication passage. By so doing, the air between the adhesive part and the tube is discharged to the outside from the proximal end opening of the connection hole, and the adhesive is introduced to the proximal end side, thereby securing a large adhesion area between the adhesive part and the tube.

Even if the flexible tube deforms to obstruct the opening of the communication passage, since the plurality of communication passages are arranged at the plurality of locations in the circumferential direction, the air can be discharged or the adhesive can be injected through other passages that are not obstructed by the tube.

A fifth preferred embodiment of Invention D provides the needle assembly according to any one of the second to fourth preferred embodiments of Invention D, wherein a concave groove is formed on a wall inner surface of a distal end portion of the connection hole, and the communication passage is formed by an opening of the concave groove being covered with the tube.

According to the needle assembly structured following the present preferred embodiment, the communication passage can be obtained by a simple structure in which the concave groove is formed on the wall inner surface of the connection hole, thereby facilitating manufacture of the needle hub. Further, the structure in which the opening of the concave groove is covered with the tube makes it easy to form the communication passage having a smaller passage cross sectional area. For example, it is possible to realize the communication passage that allows passage of the air while preventing passage of the adhesive by reducing the passage cross sectional area so as to set the flow resistance sufficiently high.

A sixth preferred embodiment of Invention D provides the needle assembly according to any one of the second to fifth preferred embodiments of Invention D, wherein the communication passage penetrates a peripheral wall part of the connection hole at the adhesive part.

According to the needle assembly structured following the present preferred embodiment, the communication passage can be easily provided by the hole penetrating the adhesive part.
In addition, the cross-sectional shape of the communication passage is less likely to change, thereby preventing unintended narrowing or obstruction in the communication passage.

A seventh preferred embodiment of Invention D provides the needle assembly according to any one of the second to sixth preferred embodiments of Invention D, wherein the communication passage allows passage of air and prevents passage of an adhesive.

According to the needle assembly structured following the present preferred embodiment, it is possible to prevent the adhesive from leaking to the outside through the communication passage, and to prevent the lumen of the needle hub or the lumen of the tube from being narrowed, obstructed or the like by the adhesive entering the lumen of the needle hub through the communication passage.

An eighth preferred embodiment of Invention D provides the needle assembly according to any one of the first to seventh preferred embodiments of Invention D, wherein a stepped surface overlapped on a distal end surface of the tube is provided in a radial inside of the needle hub.

According to the needle assembly structured following the present preferred embodiment, the tube is positioned in the axial direction with respect to the needle hub by the distal end surface of the tube being overlapped on the stepped surface. Therefore, the tube is inserted to the proper position with respect to the connection hole of the needle hub, thereby facilitating proper adhesion between the tube and the needle hub.

According to Invention D, the needle hub and the flexible tube can be appropriately adhered to each other in the needle assembly.

Hereinafter, practical embodiments of Invention D will be described with reference to the drawings.

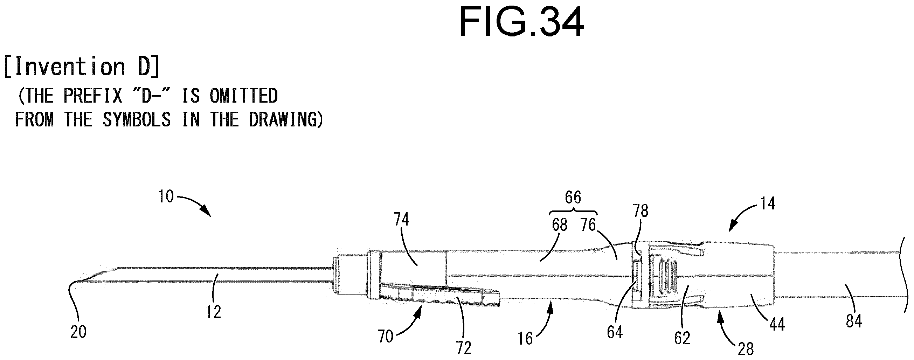

FIGS. 33 to 36 show a needle tip protector-attached needle assembly D-10 according to a first practical embodiment of Invention D. The needle tip protector-attached needle assembly D-10 has a structure in which a needle tip protector D-16 is attached to a needle hub D-14 provided on the proximal end side of a puncture needle D-12. In the following description, unless otherwise specified, the distal end side refers to the left side in FIG. 33 which is a needle tip D-20 side of the puncture needle D-12, which will be described later, and the proximal end side refers to the right side in FIG. 33, which is the rear side of the puncture direction of the needle tip protector-attached needle assembly D-10. Further, as a general rule, the vertical direction refers to the vertical direction in FIG. 34, and the left-right direction refers to the vertical direction in FIG. 33, which is the horizontal direction. In FIGS. 33 to 37 according to Invention D, the description of "D-" is omitted in each reference numeral "D-XX" (where XX is a number corresponding to the part) for the sake of legibility. Besides, in order to make it easier to understand that FIGS. 33 to 37 are drawings according to Invention D, FIGS. 33 to 37 are labelled with [invention D].

More specifically, the puncture needle D-12 is made of a metal such as stainless steel, and has a sharp, tapered needle tip A-20 at one end. A through hole D-22 is formed at the distal end portion of the puncture needle D-12, so that blood can easily flow into the lumen of the puncture needle D-12.

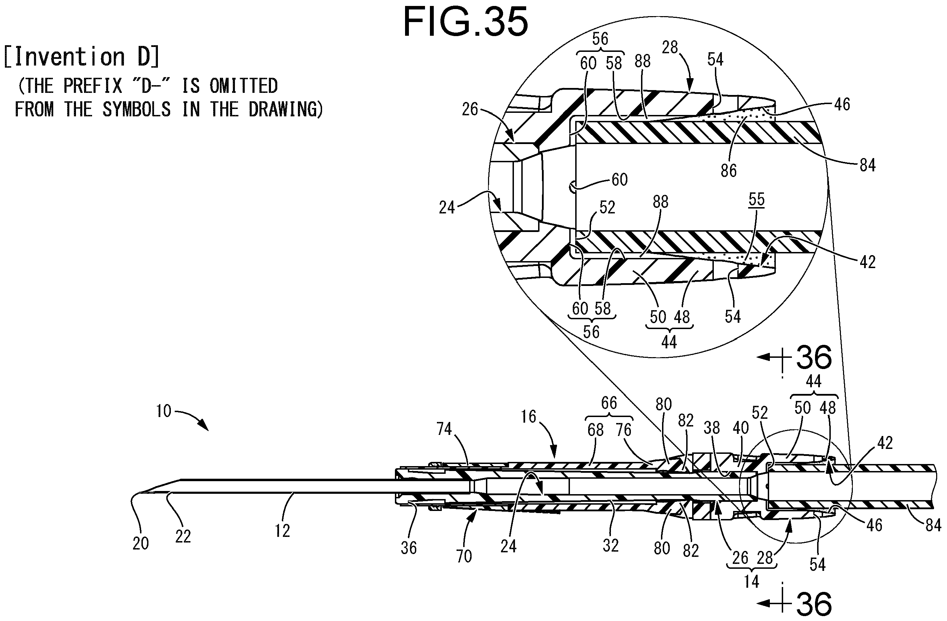

The needle hub D-14 has a tubular shape overall, and includes a lumen D-24 penetrating in the axial direction as shown in FIG. 35. The proximal end portion of the puncture needle D-12 is fixed to the distal end portion of the lumen D-24 of the needle hub D-14 in an inserted state. With this configuration, the lumen of the puncture needle D-12 communicates with the lumen D-24 of the needle hub D-14. The needle hub D-14 includes a first component D-26 that constitutes its distal end portion and a second component D-28 that constitutes its proximal end portion. Although the first component D-26 and the second component D-28 of the present practical embodiment are separate components, the first component D-26 and the second component D-28 may be integrally molded with each other.

The first component D-26 has an approximately cylindrical shape, and is formed of, for example, a rigid synthetic resin. The inner diameter dimension at the distal end portion of the first component D-26 is approximately the same as the outer diameter dimension of the puncture needle D-12. The proximal end portion of the puncture needle D-12 is inserted from the opening on the distal end side of the first component D-26, and the puncture needle D-12 is fixed to the needle hub D-14. The puncture needle D-12 may be fixed in a non-adhesive way to the first component D-26 of the needle hub D-14 by means such as press-fitting, or may be adhered to the first component D-26 with an adhesive.

The outer circumferential surface of the middle portion in the needle axis direction of the first component D-26 comprises a tapered surface D-32 that gradually increases in diameter toward the distal end side. With this configuration, in the first component D-26, the middle portion in the needle axis direction having the tapered surface D-32 has a diameter smaller than that of the distal end portion and the proximal end portion.

A locking concave part D-36 that opens onto the outer circumferential surface is formed further at the distal end portion of the first component D-26 than the tapered surface D-32. The locking concave part D-36 has an annular shape that is continuous with an approximately constant cross-sectional shape over the entire circumference. The minimum outer diameter dimension of the first component D-26 at the location where the locking concave part D-36 is formed is approximately the same as the minimum outer diameter dimension of the tapered surface D-32.

The second component D-28 is made of a rigid synthetic resin, like the first component D-26, for example. The second component D-28 has a stepped, approximately cylindrical shape overall. A coupling tube D-40 provided with a coupling hole D-38 that opens toward the distal end is provided at the distal end portion of the second component D-28. The proximal end portion of the first component D-26 is inserted into the coupling hole D-38 of the coupling tube D-40 and fixed by adhesion or welding as necessary, whereby the second component D-28 is fixed to the first component D-26.

The proximal end portion of the second component D-28 is provided with a connecting tube D-44 including a connection hole D-42 that opens toward the proximal end. As shown in FIGS. 35 to 37, the connecting tube D-44 has an approximately cylindrical shape. The connecting tube D-44 comprises the peripheral wall of the connection hole D-42, and the lumen of the connecting tube D-44 comprises the connection hole D-42. The proximal end portion of the lumen D-24 of the needle hub D-14 is constituted by the connection hole D-42.

As shown in FIG. 35, regarding the connecting tube D-44, the inner circumferential surface of the proximal end portion comprises a tapered inner surface D-46 that inclines radially outward toward the proximal end, and the connecting tube D-44 is gradually thin-walled toward the proximal end. Therefore, the connection hole D-42, which is the lumen of the connecting tube D-44, is made larger in diameter at its proximal end part, which is the end part on the opening side, than at its distal end part. In the connecting tube D-44, the proximal end portion having the tapered inner surface D-46 as the peripheral wall inner surface comprises an adhesive part D-48, and the distal end side with respect to the tapered inner surface D-46 comprises a fitting part D-50 that has a small diameter than that of the adhesive part D-48. The adhesive part D-48 is provided on the proximal end side with respect to the fitting part D-50 in the connecting tube D-44. The maximum inner diameter dimension of the bonded portion D-48 is not smaller than the outer diameter dimension of a tube D-84 described later, and is preferably larger than the outer diameter dimension of the tube D-84. Further, the minimum inner diameter dimension of the fitting part D-50 is not greater than the outer diameter dimension of the tube D-84, and is preferably smaller than the outer diameter dimension of the tube D-84.

A stepped surface D-52 is provided on the distal end side of the connection hole D-42. That is, the lumen D-24 of the needle hub D-14 is enlarged in diameter at the connection hole D-42, and the stepped surface D-52 is formed at the boundary between the distal end side of the inner circumferential surface of the needle hub D-14 with respect to the connection hole D-42 and the connection hole D-42. The stepped surface D-52 is an approximately annular plane extending in the circumferential direction, and spreads in the axis-perpendicular direction.

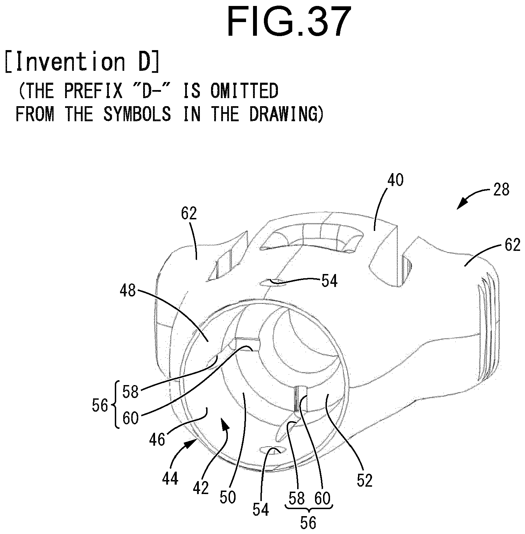

As shown in FIGS. 35 and 37, the connecting tube D-44 includes a first communication passage D-54 serving as a communication passage penetrating in the radial direction. The first communication passage D-54 penetrates the adhesive part D-48 and opens onto the tapered inner surface D-46, and communicates with the connection hole D-42 on the distal end side with respect to the proximal end of the adhesive part D-48. Through the first communication passage D-54, a radially inner region D-55 of the adhesive part D-48 communicates with and opens to the external space on the distal end side with respect to the proximal end of the adhesive part D-48. In the present practical embodiment, a pair of first communication passages D-54, 54 are provided on the upper and lower opposite sides of the connecting tube D-44 extending in the vertical direction. Further, the first communication passage D-54 of the present practical embodiment is a hole having a circular cross section, but the cross-sectional shape of the first communication passage D-54 is not limited. Besides, the cross-sectional area of the first communication passage D-54 is preferably larger than the cross-sectional area of a second communication passage D-88, which will be described later.

As shown in FIGS. 35 to 37, concave grooves D-56 are formed in the connecting tube D-44. Each concave groove D-56 has an approximately semicircular cross-sectional shape. The concave grooves D-56 are formed at the distal end portion of the connecting tube D-44, and their proximal end parts are located on the tapered inner surface D-46. The concave grooves D-56 are formed at a plurality of locations in the circumferential direction, and in the present practical embodiment, four concave grooves D-56, 56, 56, 56 are provided and are arranged at approximately equal intervals in the circumferential direction. The concave groove D-56 integrally and continuously includes a first groove D-58 that opens onto the inner circumferential surface of the connecting tube D-44 and extends in the axial direction, and a second groove D-60 that opens onto the stepped surface D-52 and extends from the tip of the first groove D-58 toward the radial inside in the radial direction.

The second component D-28 is provided with a pair of engaging arms D-62, 62 located on the left and right opposite sides of the coupling tube D-40. The engaging arms D-62 are provided so as to project from the connecting tube D-44 toward the distal end, and extend further to the distal end side than the coupling tube D-40. The engaging arms D-62, 62 are arranged apart from the coupling tube D-40 to the outer side in the left-right direction, and the connecting portion with the connecting tube D-44 is thin and narrow, so as to be elastically deformable in the direction of approaching each other by the action of an external force.

A hook D-64 is formed at the distal end portion of each engaging arm D-62. The hook D-64 projects toward the outer side in the left-right direction in each engaging arm D-62. The hooks D-64, 64 provided on the pair of engaging arms D-62, 62 are configured to move in the direction of approaching each other by the engaging arms D-62, 62 elastically deforming in the direction of approach.

The needle tip protector D-16 is attached to the needle hub D-14. The needle tip protector D-16 is a member that covers the needle tip D-20 of the used puncture needle D-12 to prevent frequent use of the puncture needle D-12 and inadvertent puncture of the puncture needle D-12, and various conventionally known structures can be adopted. As shown in FIGS. 33 to 35, the needle tip protector D-16 of the present practical embodiment has an approximately tubular shape extending in the needle axis direction overall, and is integrally molded by using a rigid synthetic resin such as polypropylene, polycarbonate, polyethylene terephthalate glycol, and ABS resin.

The needle tip protector D-16 includes a tubular peripheral wall D-66. The distal end side of the peripheral wall D-66 comprises a cylinder part D-68 having an approximately perfect circular ring-shaped cross section. A wing-shaped part D-70 is attached to the distal end portion of the cylinder part D-68. The wing-shaped part D-70 is formed of, for example, a soft synthetic resin. The wing-shaped part D-70 has a structure in which a plate-shaped wing main body D-72 projects from a tubular fitting tube part D-74 in the tangential direction. The wing-shaped part D-70 is attached to the peripheral wall D-66 of the needle tip protector D-16 by the fitting tube part D-74 being fitted externally onto the distal end portion of the cylinder part D-68.

The proximal end side of the peripheral wall D-66 comprises an expansion part D-76 having a diameter larger than that of the cylinder part D-68 and extending radially outward, so that the shape of the peripheral wall D-66 is varied in the needle axis direction. The expansion part D-76 has an approximately elliptical tubular shape, and the width dimension of the outer circumferential surface in the left-right direction (the vertical direction in FIG. 33), which is the large-diameter direction, is larger than the width dimension of the outer circumferential surface in the vertical direction (the vertical direction in FIG. 34), which is the small-diameter direction. The outer dimension of the expansion part D-76 in the left-right direction gradually increases from the distal end side toward the proximal end side, and the distal end of the outer circumferential surface of the expansion part D-76 is smoothly connected to the outer circumferential surface of the cylinder part D-68 without forming steps or irregularities. Regarding the expansion part D-76, the outer dimension in the vertical direction and the outer dimension in the left-right direction may be approximately the same. Further, the cross-sectional shape of the outer circumferential surface of the expansion part D-76 may be circular, rectangular, or the like.

On the left-right opposite sides of the expansion part D-76, respective through windows D-78 are formed so as to penetrate in the left-right direction. As shown in FIGS. 33 and 34, each through window D-78 has a slit shape having a predetermined length in the circumferential direction. The circumferential length dimension of the through window D-78 is larger than the circumferential width dimension of the hook D-64.

Locking parts D-80 are provided at the central portion of the expansion part D-76 in the left-right direction. As shown in FIGS. 33 and 35, the locking parts D-80 are integrally formed at the respective proximal end portions on the upper and lower opposite sides of the expansion part D-76. By slit-shaped notches being formed on the left and right opposite sides, the locking parts D-80 are independent of the wall part of the expansion part D-76 in the circumferential direction. With this configuration, the locking part D-80 has a plate shape that protrudes from the distal end portion of the expansion part D-76 toward the proximal end, and is allowed to undergo bending deformation in an elastic manner in the vertical direction, which is the plate thickness direction.

Further, the locking parts D-80 are provided with respective locking claws D-82 projecting inward in the vertical direction. Each locking claw D-82 has an inclined shape in which the inner circumferential surface inclines inward toward the proximal end side in the isolated state of the needle tip protector D-16. The distance between the pair of upper and lower locking claws D-82, 82 that are opposed in the vertical direction decreases toward the proximal end. Besides, the minimum dimension of the distance between the opposed pair of upper and lower locking claws D-82, 82 is smaller than the outer diameter dimension of the first component D-26 of the needle hub D-14.

As shown in FIG. 35, the needle tip protector D-16 is externally attached to the needle hub D-14. Then, the hooks D-64, 64 of the needle hub D-14 are inserted into the through windows D-78, 78 of the needle tip protector D-16, and the hooks D-64, 64 are locked to the proximal end edges of the through windows D-78, 78, so that the needle tip protector D-16 is coupled to the needle hub D-14 in a state where the movement toward the distal end side is restricted. The locking claws D-82 of the needle tip protector D-16 are elastically pressed against the outer circumferential surface of the first component D-26 of the needle hub D-14.

The needle tip protector D-16 covers the needle tip D-20 of the used puncture needle D-12. That is, when the puncture needle D-12 is stuck into a blood vessel or the like and then removed from the blood vessel or the like, the engaging arms D-62, 62 are pushed in the direction of approaching each other in the left-right direction by being pinched by the user's fingertips or the like. By so doing, the hook D-64, 64 moves to the radially inner side than the through window D-78, 78, and the hook D-64, 64 and the proximal end edges of the through window D-78, 78 are disengaged from each other. As a result, the needle tip protector D-16 is allowed to move toward the distal end side with respect to the needle hub D-14. Then, by the user pushing and moving the needle tip protector D-16 toward the distal end side with respect to the needle hub D-14, in other words, by pulling and moving the needle hub D-14 toward the proximal end side with respect to the needle tip protector D-16, the needle tip D-20 of the puncture needle D-12 is housed in the radial inside of the needle tip protector D-16.

Regarding the needle tip protector D-16 that has moved to a position where the needle tip protector D-16 covers the needle tip D-20, the locking claws D-82, 82 enter the locking concave part D-36 of the needle hub D-14, and the locking claws D-82 , 82 are locked to the distal end inner surface and the proximal end inner surface of the locking concave part D-36, so that the movement of the needle tip protector D-16 with respect to the needle hub D-14 to the distal end side and to the proximal end side is restricted. By so doing, the needle tip D-20 of the puncture needle D-12 is housed in the radial inside of the needle tip protector D-16 without being re-exposed from the needle tip protector D-16.

Meanwhile, a medical tube D-84 is connected to the needle hub D-14. The tube D-84 is made of a synthetic resin material or the like, has flexibility, and is flexibly deformable. One end of the tube D-84 is fixed to the needle hub D-14. That is, the one end of the tube D-84 is inserted in the connection hole D-42 that opens toward the proximal end side of the needle hub D-14. Then, the one end of the tube D-84 is fitted in the fitting part D-50, and the outer circumferential surface of the tube D-84 and the inner circumferential surface of the adhesive part D-48 are adhered to each other by an adhesive D-86. Further, the distal end surface of the tube D-84 is axially overlapped with the stepped surface D-52 provided on the inner circumferential surface of the needle hub D-14. By so doing, the position of the insertion end of the tube D-84 into the connection hole D-42 is determined in the axial direction by contact with the stepped surface D-52. The distal end portion of the tube D-84 fitted in the fitting part D-50 is in a deformed state by being pushed inward in the radial direction by the contact reaction force with the fitting part D-50, and is fitted to the fitting part D-50 based on the elasticity of the tube D-84.

By the tube D-84 being fitted in the fitting part D-50 of the connection hole D-42, the inside openings of the concave grooves D-56 are covered by the tube D-84. With this configuration, the second communication passage D-88 serving as a communication passage extending between the overlapped surfaces of the connecting tube D-44 of the needle hub D-14 and the tube D-84 is constituted by each concave groove D-56. In the present practical embodiment, the concave grooves D-56 are arranged at four locations in the circumferential direction. Thus, the inside openings of the respective concave grooves D-56 are covered by the tube D-84, so that the second communication passages D-88 are arranged at four locations in the circumferential direction.

Regarding the second communication passage D-88, the proximal end opening opens to the tapered inner surface D-46 and communicates with the radially inner region D-55 of the adhesive part D-48, while the distal end opening opens to the radial inside on the distal end side with respect to the tube D-84, and communicates with the lumen D-24 of the needle hub D-14. Then, the radially inner region D-55 of the adhesive part D-48 and the external space are placed in communication with each other through the second communication passage D-88 and the lumen D-24 of the needle hub D-14, and the radially inner region D-55 of the adhesive part D-48 opens to the external space through the second communication passage D-88.

The adhesive D-86 is a curable adhesive that can be used for adhesion between the needle hub D-14 and the tube D-84, which are medical instruments. For example, in addition to an ultraviolet curable adhesive that is cured by irradiation with ultraviolet rays, a cyanoacrylate-based adhesive, an epoxy resin-based adhesive, a hot-melt adhesive, an acrylic adhesive and the like are preferably used.

The adhesive D-86 is injected into the connection hole D-42 of the needle hub D-14 in which the end of the tube D-84 is inserted. The adhesive D-86 may be injected from the proximal end opening of the connection hole D-42 between the connecting tube D-44 of the needle hub D-14 and the tube D-84, but in the present practical embodiment, the adhesive D-86 is injected through the first communication passage D-54 between the connecting tube D-44 of the needle hub D-14 and the tube D-84. The adhesive part D-48, which is the opening portion on the proximal end side of the connection hole D-42, has an inner diameter larger than that of the fitting part D-50, which is the distal end portion. Thus, the gap between the inner circumferential surface of the D-44 and the outer circumferential surface of the tube D-84 is made larger at the adhesive part D-48. With this configuration, a predetermined amount of the adhesive D-86 is accumulated and held between the connecting tube D-44 and the tube D-84. When injecting the adhesive D-86, it is desirable to hold the needle hub D-14 so that the distal end side, which is the needle tip D-20 side, faces vertically downward, or to cover the proximal end opening of the connection hole D-42 by an appropriate lid member or the like. By so doing, the adhesive D-86 injected into the connection hole D-42 from the first communication passage D-54 is less likely to spill from the proximal end opening of the connection hole D-42.

When the adhesive D-86 is injected through the first communication passage D-54 toward the distal end side with respect to the first communication passage D-54, the air in the connection hole D-42 is discharged to the lumen D-24 of the needle hub D-14 through the second communication passage D-88 arranged on the distal end side with respect to the first communication passage D-54. By so doing, progress of the adhesive D-86 toward the distal end side is not hindered by the air in the connection hole D-42, and the needle hub D-14 and the tube D-84 are adhered to each other over a wider range.

The second communication passage D-88 is partially provided in the circumferential direction so that the passage cross sectional area is sufficiently small to allow passage of air and prevent passage of the adhesive D-86. Therefore, the adhesive D-86 injected into the connection hole D-42 does not enter the lumen D-24 of the needle hub D-14 through the second communication passage D-88, and the distal end openings of the lumen D-24 of the needle hub D-14 and the tube D-84 are not completely or partially obstructed by the adhesive D-86. There is no problem even if the adhesive D-86 infiltrates halfway through the second communication passage D-88, but it is desirable that the adhesive D-86 does not pass through the second communication passage D-88 to reach the lumen D-24 of the needle hub D-14.

The second communication passage D-88 is formed by the opening of the concave groove D-56 formed on the inner surface of the needle hub D-14 being covered with the tube D-84. Thus, the passage cross sectional area of the second communication passage D-88 is easily made smaller than that of the first communication passage D-54, which is constituted by the hole penetrating the rigid needle hub D-14. Therefore, by sufficiently reducing the area of the opening of the second communication passage D-88 to the proximal end side, it is also possible to set a high flow resistance to the second communication passage D-86 to such an extent that can prevent the adhesive D-86 from passing through the second communication passage D-88.

In this way, the adhesion region between the inner surface of the connection hole D-42 of the needle hub D-14 and the tube D-84 by the adhesive D-86 is appropriately controlled and provided. Therefore, a sufficiently large fixing strength can be obtained by adhering the needle hub D-14 and the tube D-84 to each other, and the lumen D-24 of the needle hub D-14 and the tube D-84 are prevented from being narrowed or obstructed due to wraparound of the adhesive D-86.

In some cases, the flexible tube D-84 is not inserted straight into the needle hub D-14 as shown in FIG. 35. For example, if the tube D-84 is curved upward toward the proximal end side, the upper second communication passage D-88 may be covered and blocked by the tube D-84. However, in the needle hub D-14, the plurality of second communication passages D-88 are arranged at different positions in the circumferential direction. Thus, even if one second communication passage D-88 is blocked, the other second communication passages D-88 are placed in a communicating state, and ventilation of air through the second communication passages D-88 is realized. Similarly, the first communication passages D-54 are arranged in plurality at different positions in the circumferential direction. This prevents the situation in which all the first communication passages D-54 are blocked at the same time, whereby the adhesive D-86 can be injected smoothly.

The adhesive D-86 is injected into the connection hole D-42 with the end of the tube D-84 inserted into the connection hole D-42 of the needle hub D-14, for example. However, the tube D-84 may be inserted into the connection hole D-42 after the adhesive D-86 is applied to at least one of the outer circumferential surface of the tube D-84 and the inner circumferential surface of the connection hole D-42 (the adhesive part D-48). In this case, when the distal end portion of the tube D-84 is fitted into the fitting part D-50 of the connection hole D-42, fitting is performed with the outer circumferential surface of the tube D-84 pressed against the inner circumferential surface of the fitting part D-50, so that the adhesive D-86 adhering to the outer circumferential surface of the tube D-84 is scraped off by the fitting part D-50. Therefore, for example, even if the adhesive D-86 adheres to the outer circumferential surface of the distal end portion of the tube D-84 when the distal end portion of the tube D-84 passes through the radial inside of the adhesive part D-48, the adhesive D-86 is less likely to enter between the tube D-84 and the fitting part D-50. This makes it possible to prevent the adhesive D-86 from entering the lumen D-24 of the needle hub D-14 on the distal end side of the tube D-84, thereby preventing the lumen D-24 of the needle hub D-14 and the tube D-84 from being narrowed or obstructed by the adhesive D-86.

A practical embodiment of Invention D has been described in detail above, but Invention D is not limited to those specific descriptions. For example, the preceding practical embodiment illustrates that the hole-shaped first communication passage D-54 is provided on the proximal end side of the connecting tube D-44, while the second communication passage D-88 formed by the concave groove D-56 is provided on the distal end side of the connecting tube D-44. However, the communication passage may be constituted by only one of the first communication passage D-54 and the second communication passage D-88. Further, a hole-shaped communication passage may be provided at the distal end portion of the adhesive part D-48.

The preceding practical embodiment exemplifies the structure in which the passage cross sectional area of the second communication passage D-88 is reduced to set a high flow resistance, as a structure for allowing the passage of the air and preventing the passage of the adhesive D-86 in the second communication passage D-88. However, the structure for allowing the passage of the air and preventing the passage of the adhesive D-86 can also be realized by, for example, providing a filter that allows the passage of the air and prevents the passage of the adhesive D-86 to the second communication passage D-88.

In the second communication passage D-88 of the preceding practical embodiment, the opening on the distal end side communicates with the external space through the lumen D-24 of the needle hub D-14, but the said opening may directly communicates with the external space, bypassing the lumen D-24 of the needle hub D-14. As a specific example, instead of the second groove D-60 extending radially inward from the distal end part of the first groove D-58, a hole extending radially outward from the distal end part of the first groove D-58 may be provided. This configuration enables the second communication passage to directly communicate with the external space.

The preceding practical embodiment illustrates that the adhesive D-86 is introduced into the connection hole D-42 through the first communication passage D-54, and the air in the connection hole D-42 is discharged through the second communication passage D-88. However, for example, it would also be acceptable that the adhesive D-86 is introduced into the connection hole D-42 through the second communication passage D-88, and the first communication passage D-54 and the proximal end opening of the connection hole D-42 serve as a vent for discharging the air. In the case where the first communication passage D-54 is used as a vent for discharging the air in the connection hole D-42, it is desirable that the first communication passage D-54 allows the passage of the air and prevents the passage of the adhesive D-86, similarly to the second communication passage D-88 of the preceding practical embodiment.

Besides, it would also be acceptable that the adhesive D-86 is introduced into the connection hole D-42 from the proximal end opening of the connection hole D-42, and both the first communication passage D-54 and the second communication passage D-88 serve as a vent for discharging the air in the connection hole D-42. In this case, when the adhesive D-86 is injected from the proximal end opening of the connection hole D-42, initially, the air in the connection hole D-42 is discharged to the outside through both the first communication passage D-54 and the second communication passage D-88. By so doing, the adhesive D-86 enters the inside of the connection hole D-42 without staying in the opening thereof, and the inner circumferential surface of the connecting tube D-44 and the outer circumferential surface of the tube D-84 are adhered to each other over a wide range. Next, when the adhesive D-86 is charged to a position where the inside opening of the first communication passage D-54 is obstructed, the air in the connection hole D-42 will not be discharged to the outside through the first communication passage D-54. Here, since the second communication passage D-88 is arranged on the distal end side with respect to the first communication passage D-54, the discharge of the air to the lumen D-24 of the needle hub D-14 is continued through the second communication passage D-88. Therefore, even if the first communication passage D-54 is obstructed by the adhesive D-86, the air in the connection hole D-42 does not hinder the progress of the adhesive D-86 toward the distal end side, so that the needle hub D-14 and tube D-84 are adhered to each other over a wider range.

Any of the number, arrangement, shape, size, etc. of the communication passages is not particularly limited. For example, when the communication passage is used for introducing the adhesive D-86, it is desirable that the passage cross sectional area is made relatively large as in the first communication passage D-54 of the preceding practical embodiment, so that the adhesive D-86 is smoothly introduced. Further, when the communication passage is used for discharging air, it is desirable that the cross sectional area is made relatively small as in the second communication passage D-88 of the preceding practical embodiment, so that the adhesive D-86 is less likely to infiltrate.

In the preceding practical embodiment, the inner circumferential surface of the adhesive part D-48 comprises the tapered inner surface D-46, so as to form a space for holding the adhesive D-86 between the adhesive part D-48 and the tube D-84. However, the structure that forms a space between the adhesive part D-48 and the tube D-84 is not limited to the tapered inner surface D-46. As a specific example, it would also be possible to provide a step on the inner circumferential surface of the connecting tube D-44 and make the inner diameter dimension of the adhesive part D-48 larger than the outer diameter dimension of the tube D-84, thereby forming a space for holding the adhesive D-86 between the adhesive part D-48 and the tube D-84.

The needle tip protector D-16 is not essential and may be omitted. Besides, a needle tip protector whose structure and material are different from those of the needle tip protector D-16 of the preceding practical embodiment may be adopted.

### KEYS TO SYMBOLS

A-10 needle tip protector
A-12 needle assembly
A-14 puncture needle body
A-16 puncture needle
A-18 needle hub
A-20 needle tip
A-22 through hole
A-24 needle support part
A-26 protector coupling part
A-28 positioning protrusion
A-30 tapered surface
A-32 small-diameter tube part
A-34 locking concave part
A-36 large-diameter fulcrum part
A-38 proximal-end-side restricting surface
A-40 distal-end-side restricting surface
A-42 coupling tube
A-44 connecting tube
A-46 concave part
A-47 external conduit
A-48 fluid flow path
A-50 engaging arm
A-52 hook
A-54 peripheral wall
A-56 inner hole
A-57 proximal end side opening
A-58 cylindrical part
A-60 expansion part
A-62 narrow part
A-64 wide part
A-66 internal space
A-68 through window
A-70 thick-walled part
A-71 bridge part
A-72 recess
A-74 concavity
A-76 stepped surface
A-78 locking piece (flexible piece)
A-80 locking claw
A-82 proximal end side end face
A-82a small wall part
A-82b inclined surface
A-84 split mold
A-86 split mold
A-88 mold
A-90 parting surface
A-92 parting line
A-94 cavity
A-96 injection gate
A-98 injection gate trace
A-100 protruding pin
A-102 protruding pin trace
A-104 wing-shaped part
A-106 fitting tube part
A-108 coupling part
A-110 wing main body
A-112 touch part
A-120 needle tip protector
A-122 needle assembly
A-130 mold
A-132 cavity
A-134 injection gate
A-140 needle tip protector
A-142 recess
A-150 needle tip protector
A-160 needle tip protector
B-10 puncture needle body
B-12 needle assembly
B-14 needle tip protector
B-16 puncture needle
B-18 needle hub
B-20 needle tip
B-22 through hole
B-24 needle support part
B-26 protector coupling part
B-30 tapered surface
B-32 small-diameter tube part
B-34 concave part
B-36 convex part
B-38 proximal-end-side restricting surface
B-40 distal-end-side restricting surface
B-42 coupling tube
B-44 connecting tube
B-46 recess
B-48 connection hole
B-50 bottom part
B-52 opening expansion part
B-54 reinforcing part
B-56 connection port
B-58 tapered outer circumferential surface
B-60 external conduit
B-62 fluid flow path
B-64 arm part
B-66 hook
B-68 peripheral wall
B-70 inner hole
B-72 cylindrical part
B-74 expansion part
B-76 narrow part
B-78 wide part
B-80 internal space
B-82 through window
B-84 stepped surface
B-86 projecting piece
B-88 claw part
B-90 proximal end side end face
B-90a vertical surface
B-90b inclined surface
B-92 wing-shaped part
B-94 fitting tube part
B-96 coupling part
B-98 wing main body
B-100 puncture needle body
B-102 needle hub
B-103 needle support part
B-104 protector coupling part
B-106 connection hole
B-108 bottom part
B-110 opening expansion part
B-112 reinforcing part
B-114 connection port
B-116 arm part
C-10 needle tip protector
C-12 needle assembly
C-14 indwelling needle
C-16 puncture needle
C-18 needle hub
C-20 needle tip
C-22 through hole
C-24 needle support part
C-26 protector coupling part
C-28 positioning protrusion
C-30 tapered surface
C-32 small-diameter tube part
C-34 locking concave part
C-36 large-diameter fulcrum part
C-38 proximal-end-side restricting surface
C-40 distal-end-side restricting surface
C-42 coupling tube
C-44 connecting tube
C-46 concave part
C-47 external conduit
C-48 fluid flow path
C-50 engaging arm
C-52 hook
C-54 peripheral wall
C-56 inner hole
C-58 cylindrical part
C-60 expansion part
C-62 narrow part
C-64 wide part
C-66 internal space
C-68 through window
C-70 concave part
C-74 stepped surface
C-76 locking piece
C-78 locking claw
C-80 proximal end side end face
C-80a vertical surface
C-80b inclined surface
C-82 wing-shaped part
C-84 fitting tube part
C-86 coupling part
C-88 wing main body
C-90 contact part
C-92 parting line
C-94 gate trace
C-96 parting line
C-98 gate trace
C-140 deformation amount limiting part
C-142 protrusion
D-10 needle tip protector-attached needle assembly (needle assembly)
D-12 puncture needle
D-14 needle hub
D-16 needle tip protector
D-20 needle tip
D-22 through hole
D-24 lumen
D-26 first component
D-28 second component
D-32 tapered surface
D-36 locking concave part
D-38 coupling hole
D-40 coupling tube
D-42 connection hole
D-44 connecting tube
D-46 tapered inner surface
D-48 adhesive part
D-50 fitting part
D-52 stepped surface
D-54 first communication passage (communication passage)
D-55 radially inner region
D-56 concave groove
D-58 first groove
D-60 second groove
D-62 engaging arm
D-64 hook
D-66 peripheral wall
D-68 cylinder part
D-70 wing-shaped part
D-72 wing main body
D-74 fitting tube part
D-76 expansion part
D-78 through window
D-80 locking part
D-82 locking claw
D-84 tube
D-86 adhesive
D-88 second communication passage (communication passage)

## Claims

1. A needle tip protector that is made of resin and includes a peripheral wall configured to move in a needle axis direction with respect to a puncture needle to cover a needle tip of the puncture needle, wherein
the peripheral wall includes a tubular part on a distal end side of the peripheral wall,
the peripheral wall includes a thick-walled part on a proximal end side of the tubular part, the thick-walled part including a portion having a wall thickness dimension larger than that of the tubular part, and
at least one injection gate trace of a resin material during molding is provided to the thick-walled part.

2. The needle tip protector according to claim 1, wherein a recess is provided on an outer surface of the thick-walled part.

3. The needle tip protector according to claim 2, wherein the injection gate trace is provided on an inner surface of the recess.

4. The needle tip protector according to claim 2 or 3, wherein a depth dimension of the recess gradually decreases toward the distal end side of the peripheral wall.

5. The needle tip protector according to any one of claims 2-4, wherein an opening end edge of the recess has a curved cross-sectional shape with no corners.

6. The needle tip protector according to any one of claims 1-5, wherein
the peripheral wall includes an expansion part on the proximal end side of the tubular part, the expansion part including a portion whose wall thickness is larger than that of the tubular part and having an outer dimension larger than that of the tubular part, and
the injection gate trace is provided on an outer surface of the expansion part.

7. A needle tip protector that is made of resin and includes a peripheral wall configured to move in a needle axis direction with respect to a puncture needle to cover a needle tip of the puncture needle, wherein
the peripheral wall includes a tubular part and an expansion part having an outer dimension larger than that of the tubular part, and
at least one injection gate trace of a resin material during molding is provided to the expansion part.

8. The needle tip protector according to any one of claims 1-7, wherein the needle tip protector further includes an engaging part engaged with a needle hub provided on a proximal end side of the puncture needle, and the injection gate trace is provided at a position away from the engaging part.

9. The needle tip protector according to any one of claims 1-8, wherein the needle tip protector further includes a locking piece configured to, with the needle tip of the puncture needle covered with the peripheral wall, be locked to a needle hub provided on a proximal end side of the puncture needle to prevent re-exposure of the needle tip, and the injection gate trace is provided at a position away from the locking piece.

10. The needle tip protector according to any one of claims 1-9, wherein an outer surface of the peripheral wall is molded by split molds combined in an axis-perpendicular direction and has a parting line extending in an axial direction.

11. The needle tip protector according to any one of claims 1-9, wherein an outer surface of the peripheral wall is molded by a die to be released in an axial direction and has no parting line extending in the axial direction.

12. The needle tip protector according to any one of claims 1-11, wherein the at least one injection gate trace comprises a plurality of injection gate traces provided in a circumferential direction of the peripheral wall.

13. The needle tip protector according to any one of claims 1-12, wherein
at least a proximal end part of the peripheral wall has outer dimensions in an axis-perpendicular direction that are different in a circumferential direction,
in a wide part in which the outer dimension is larger, at least one of an engaging part engaged with a needle hub fixed to a proximal end part of the puncture needle and a locking piece configured to be locked to the needle hub to prevent re-exposure of the needle tip, and
in a narrow part in which the outer dimension is smaller, the injection gate trace is provided.

14. The needle tip protector according to any one of claims 1-13, wherein the peripheral wall is transparent or translucent to allow transmission of light, and light transparency of the peripheral wall at the injection gate trace is lower than that of a rest of the peripheral wall.

15. A needle assembly including: a puncture needle body having a puncture needle; and a needle tip protector configured to move in a needle axis direction with respect to the puncture needle body to cover a needle tip of the puncture needle, wherein
the needle tip protector comprises the needle tip protector according to any one of claims 1-14, and
at least a part of an outer surface of the thick-walled part of the needle tip protector comprises a touch part configured to touch a skin of a patient, and the recess according to claim 2 opens onto the touch part.

16. The needle assembly according to claim 15, wherein the touch part is configured to touch the skin of the patient when the puncture needle body is indwelled.

17. The needle assembly according to claim 15 or 16, wherein the injection gate trace is provided at a position away from the touch part.

18. The needle assembly according to any one of claims 15-17, wherein on the outer surface of the thick-walled part of the needle tip protector, a protruding pin trace is provided at one of the touch part and a portion located on an opposite side of the touch part in a diametrical direction.

19. A needle assembly including: a puncture needle body having a puncture needle; and a needle tip protector configured to move in a needle axis direction with respect to the puncture needle body to cover a needle tip of the puncture needle, wherein
the needle tip protector comprises the needle tip protector according to claim 6 or 7,
the expansion part includes a narrow part and a wide part in which an outer dimension is larger than that of the narrow part, and the injection gate trace is provided to a wide part side, and
the wide part of the needle tip protector includes an engaging part detachably engaged with an engaging arm provided to a needle hub fixed to a proximal end part of the puncture needle.

20. The needle assembly according to claim 19, wherein the injection gate trace is provided on a proximal end side of the engaging part.
